(19) Europäisches Patentamt
European Patent Office
Office européen des brevets

(11) Publication number: **0 029 998**
**B1**

(12) # EUROPEAN PATENT SPECIFICATION

(45) Date of publication of patent specification: **06.03.85**

(51) Int. Cl.[4]: **C 07 D 501/36,** A 61 K 31/545 // C07D257/04, C07D277/20, C07D209/48

(21) Application number: **80107350.3**

(22) Date of filing: **25.11.80**

(54) **Cephem compounds, processes for their preparation and pharmaceutical compositions containing them.**

(30) Priority: **30.11.79 GB 7941417**
**17.12.79 GB 7943363**

(43) Date of publication of application:
**10.06.81 Bulletin 81/23**

(45) Publication of the grant of the patent:
**06.03.85 Bulletin 85/10**

(84) Designated Contracting States:
**AT BE CH DE FR GB IT LI NL SE**

(56) References cited:
**EP-A-0 000 272**
**FR-A-2 255 077**
**FR-A-2 283 686**

(73) Proprietor: **Fujisawa Pharmaceutical Co., Ltd.**
**3, Doshomachi 4-chome Higashi-ku**
**Osaka-shi, Osaka 541 (JP)**

(72) Inventor: **Takaya, Takao**
**No. 1-5-87, Suimeidai**
**Kawanishi (JP)**
Inventor: **Inoue, Yoshikazu**
**No. 233-3, Aza-Minamidai**
**Shimokema Amagasaki (JP)**
Inventor: **Yasuda, Nobuyoshi**
**No. 10-12, Kitashowa-cho**
**Nishinomiya (JP)**
Inventor: **Murata, Masayoshi**
**No. 2-5-5, Segawa**
**Mino (JP)**
Inventor: **Ueda, Ikuo**
**No. 4-13-36, Kumano-cho**
**Toyanaka (JP)**
Inventor: **Matsuo, Masaaki**
**No. 5-4-12, Nakasakurazuka**
**Toyonaka (JP)**
Inventor: **Tsuji, Kiyoshi**
**No. 170, Hata-cho**
**Kishiwada (JP)**
Inventor: **Kato, Masayuki**
**No. 3-11-30, Mino**
**Mino (JP)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 029 998 B1

Courier Press, Leamington Spa, England.

0 029 998

(74) Representative: **Türk, Dietmar, Dr. rer. nat. et al**
**Redies, Redies, Türk & Gille Patentanwälte**
**Brucknerstrasse 20**
**D-4000 Düsseldorf 13 (DE)**

**Description**

The present invention relates to new cephem compounds and pharmaceutically acceptable salts thereof. More particularly, it relates to new cephem compounds and pharmaceutically acceptable salts thereof, which have antimicrobial activities and to processes for preparation thereof, and to pharmaceutical composition comprising the same.

Accordingly, it is one object of the present invention to provide new cephem compounds and pharmaceutically acceptable salts thereof, which are active against a number of pathogenic microorganisms.

Another object of the present invention is to provide processes for the preparation of new cephem compounds and pharmaceutically acceptable salts thereof.

A further object of the present invention is to provide pharmaceutical composition comprising, as active ingredients, said new cephem compounds and pharmaceutically acceptable salts thereof.

FR—A—2283686 discloses cephem compounds having a tetrazolylthiomethyl substituent in the 3-position of the ring system, the substituent on the tetrazolyl group being a carboxyalkylene. Furtheron, EP—00 00 272 claims tetrazolylthiomethyl substituted cephem compounds, wherein a carboxyalkylene substituted amino-alkylene group is attached to the tetrazolyl moiety. All these compounds are claimed to have antibacterial properties.

The object new cephem compounds are novel and can be represented by the following general formula (I).

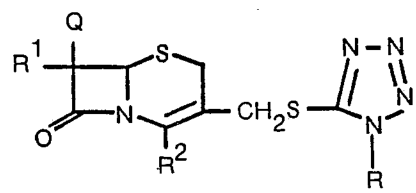

(I)

wherein

$R^1$ is amino or an acylamino group;

$R^2$ is carboxy or an esterified carboxy group;

R is a group of the formula: $—A_1—R^{3a}$

[in which $A_1$ is hydroxy($C_1$ to $C_6$)alkylene, amino($C_1$ to $C_6$)alkylene, protected amino($C_1$ to $C_6$)alkylene, alkenylene with up to 6C-atoms or hydroxyimino($C_1$ to $C_6$)alkylene wherein the hydrogen atom of the hydroxyimino group may be replaced with a ($C_1$ to $C_6$)aliphatic hydrocarbon group; and $R^{3a}$ is carboxy or an esterified carboxy group] or a group of the formula:

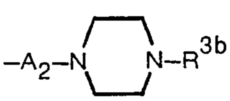

[in which $A_2$ is ($C_1$ to $C_6$)alkylene which may have an oxo group; and $R^{3b}$ is carboxy, esterified carboxy, an aliphatic or aromatic group]; and Q is hydrogen or ($C_1$ to $C_6$) alkoxy;

and pharmaceutically acceptable salts thereof.

As to the object compounds (I) and the starting compounds of the present invention, it is to be understood that there may be one or more stereoisomeric pair(s) such as optical and/or geometrical isomers due to asymmetric carbon atom(s) and/or double bond(s), in the molecule, and these isomers are also included within the scope of the present invention.

The particulars of such isomers will be made more clear in the following explanation.

According to the present invention, the object compounds (I) can be prepared by the following processes which are illustrated in the following scheme.

PROCESS 1

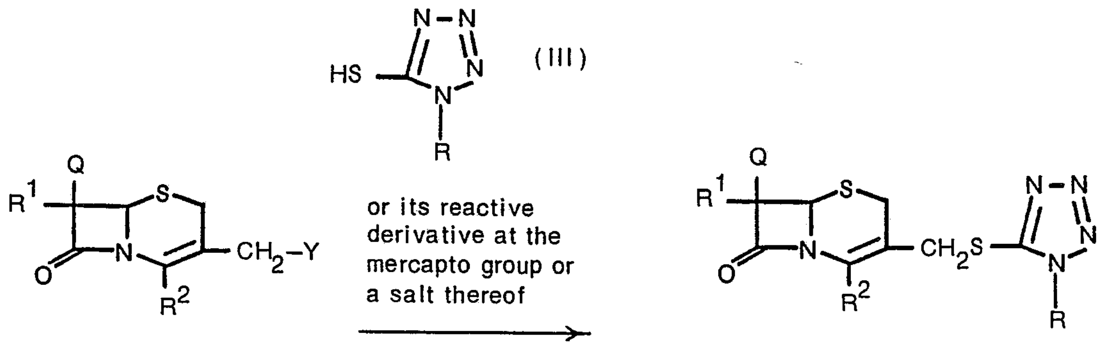


(III)

(II) (I)

or a salt thereof
or a salt thereof

PROCESS 2

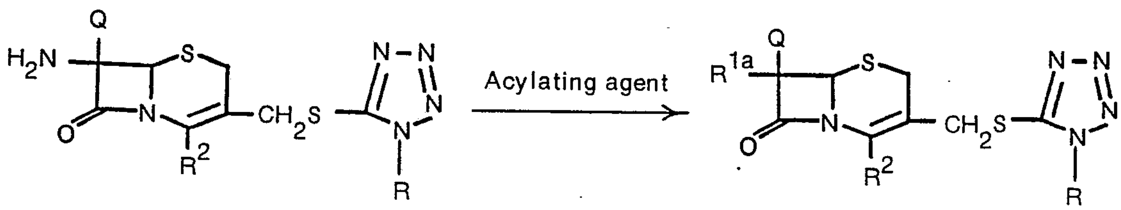


(Ib) (Ia)

or its reactive
derivatives at
the amino group

or a salt thereof

or a salt thereof

PROCESS 3

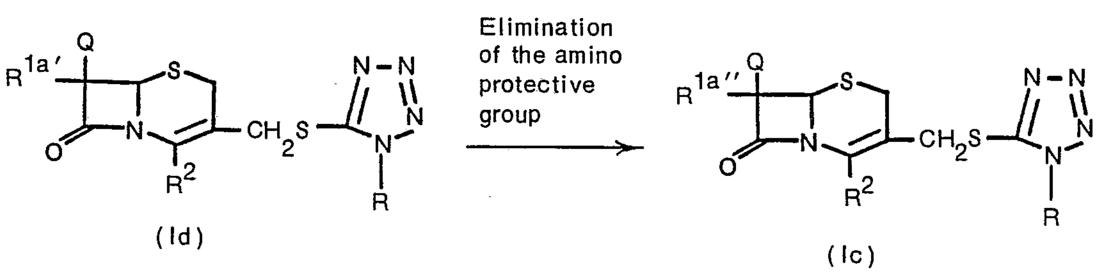


(Id) (Ic)

or a salt thereof
or a salt thereof

PROCESS 4

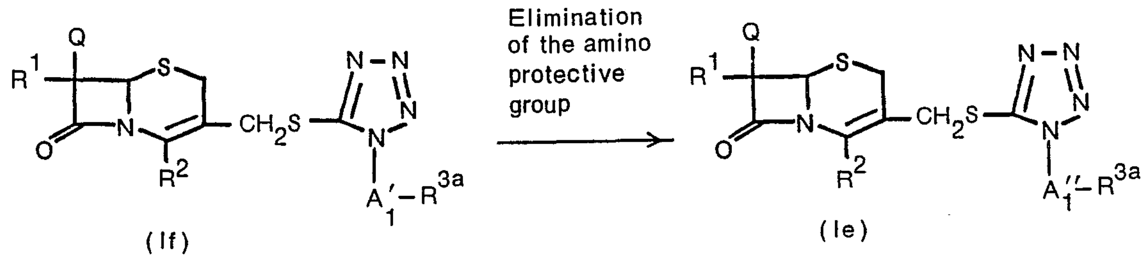


(If) (Ie)

or a salt thereof or a salt thereof

wherein

$R^1$, $R^2$, R and Q are each as defined above,

Y is a group which can be substituted by a group of the formula:

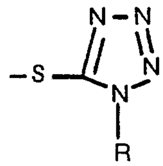

(wherein R is as defined above).

$R^{1a}$ is acylamino;

$R^{1a'}$ is acylamino having a protected amino group;

$R^{1a''}$ is acylamino having an amino group;

$A_1'$ is a protected amino($C_1$ to $C_6$)alkylene; and

$A_1''$ is amino($C_1$ to $C_6$)alkylene.

Among the starting compounds in the present invention, the compound (III) and a part of the acylating agent and the compounds (II) are novel and can be prepared by the processes which are illustrated in the following scheme.

PROCESS A

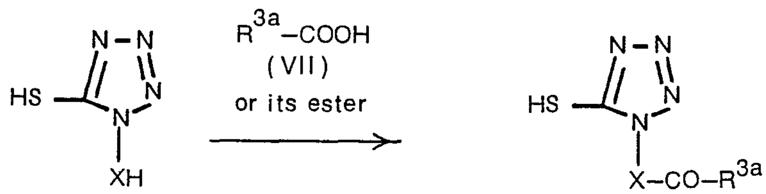


(V) (IV)

or its reactive derivative at the mercapto group

or its reactive derivative at the mercapto group or a salt thereof

PROCESS B

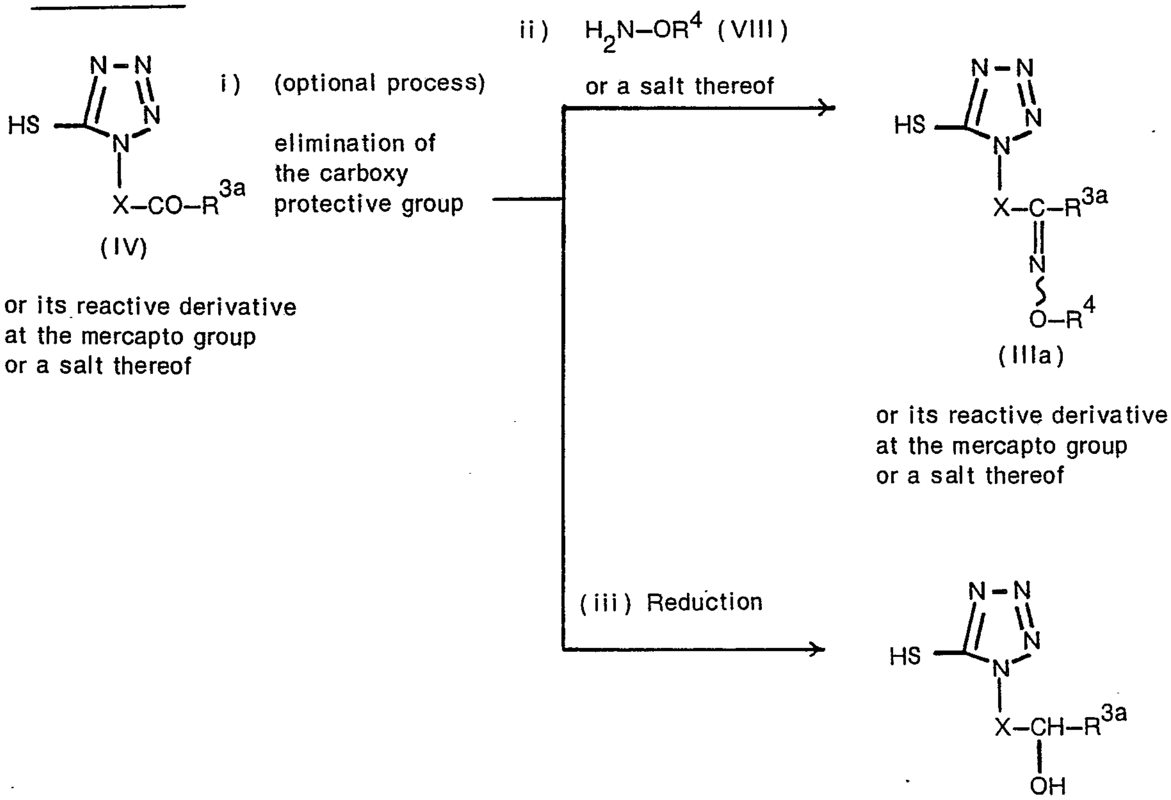


(IV)

or its reactive derivative
at the mercapto group
or a salt thereof

(IIIa)

or its reactive derivative
at the mercapto group
or a salt thereof

(IIIb)

or its reactive derivative
at the mercapto group
or a salt thereof

PROCESS C

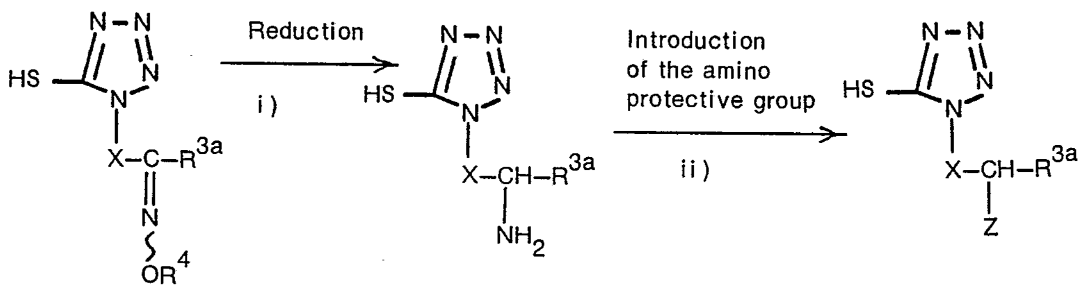


(IIIa)

or its reactive derivative
at the mercapto group
or a salt thereof

(IIId)

or its reactive derivative
at the mercapto group
or a salt thereof

(IIIc)

or its reactive derivative
at the mercapto group
or a salt thereof

PROCESS D

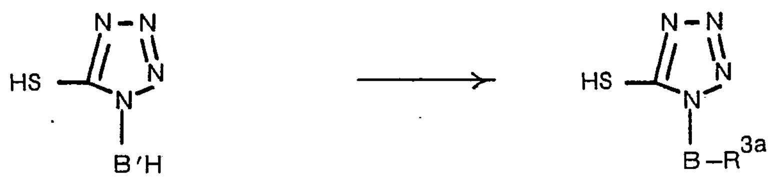

(IIIf)

or its reactive derivative at the mercapto group

(IIIe)

or its reactive derivative at the mercapto group or a salt thereof

PROCESS E

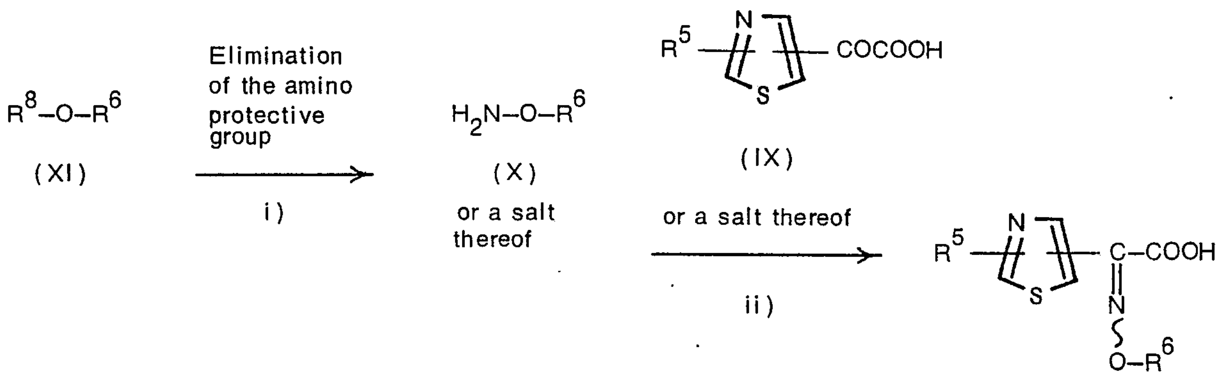


(XI)

(X) or a salt thereof

(IX) or a salt thereof

(VIa)

or a salt thereof

PROCESS F

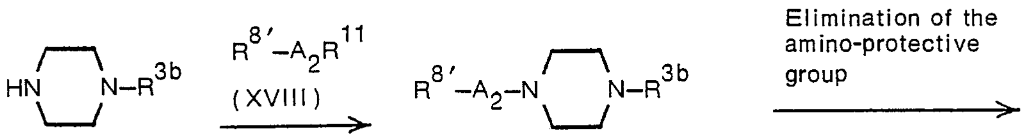


(XIX) or a salt thereof

(XVII) or a salt thereof

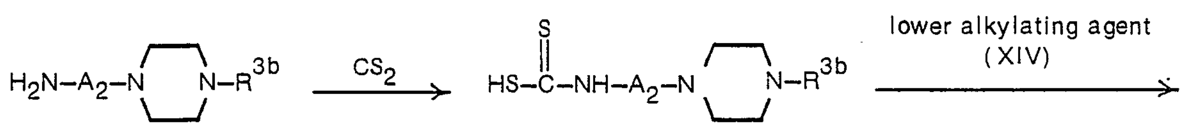


(XVI) or a salt thereof

(XV) or a salt thereof

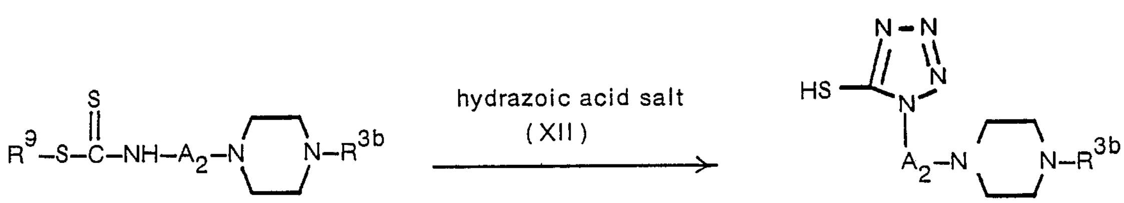


(XIII) or a salt thereof

(IIIg) or a salt thereof

PROCESS G

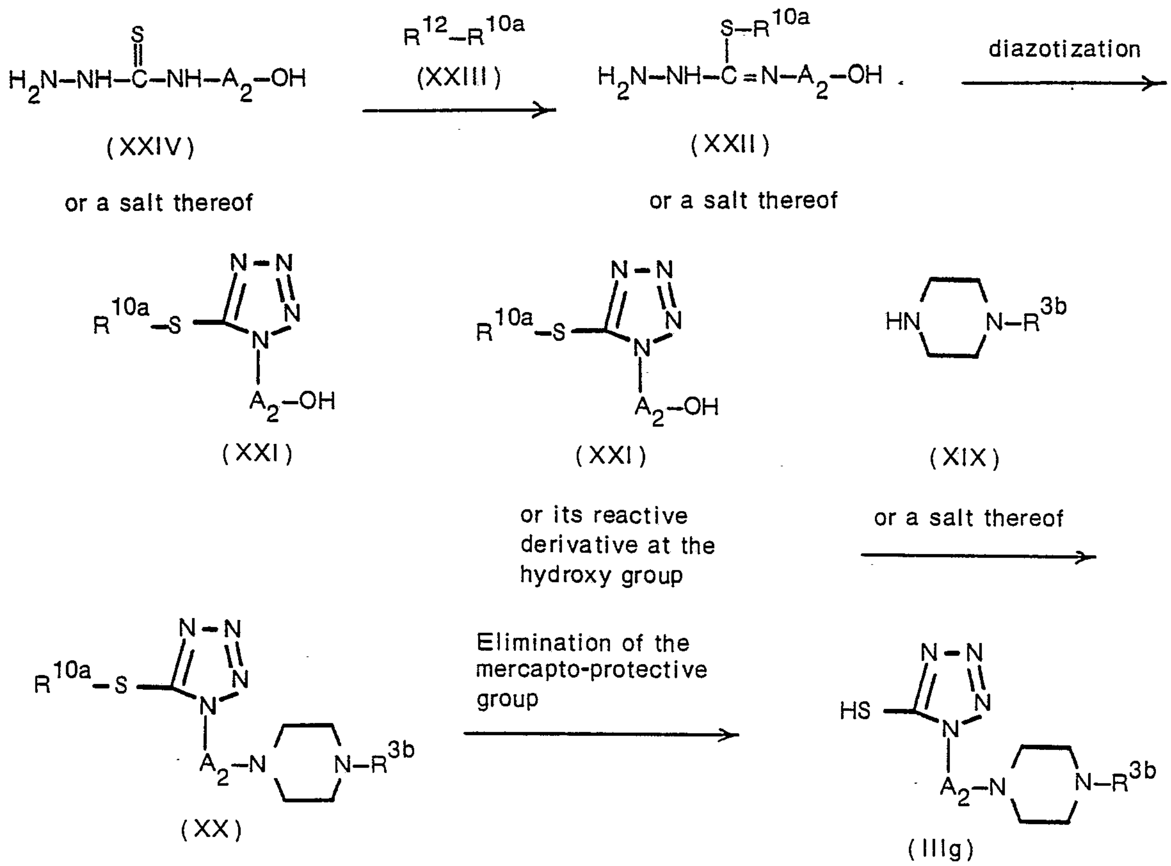


(XXIV) or a salt thereof

(XXIII)

(XXII) or a salt thereof

(XXI)

(XXI) or its reactive derivative at the hydroxy group

(XIX) or a salt thereof

(XX) or a salt thereof

(IIIg) or a salt thereof

PROCESS H

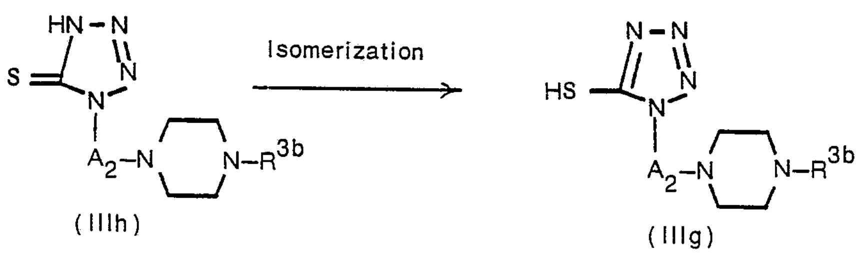


(IIIh) or a salt thereof

(IIIg) or a salt thereof

PROCESS I

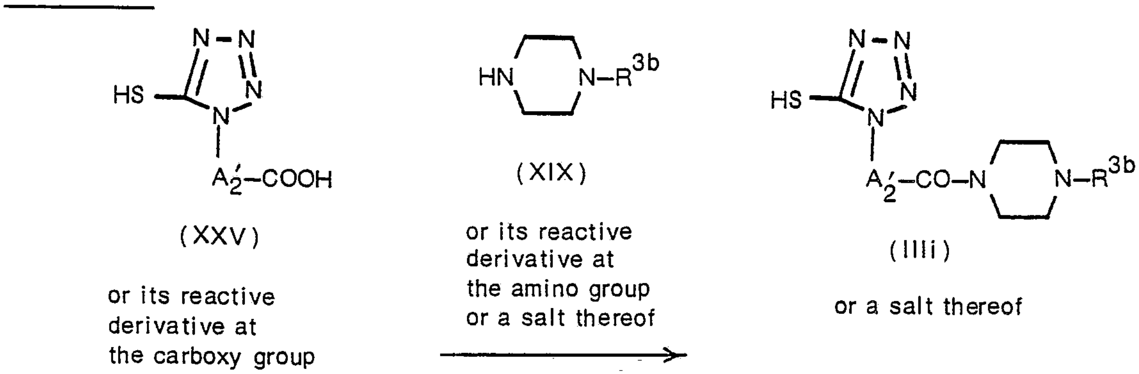

(XXV) or its reactive derivative at the carboxy group or a salt thereof

(XIX) or its reactive derivative at the amino group or a salt thereof

(IIIi) or a salt thereof

PROCESS J

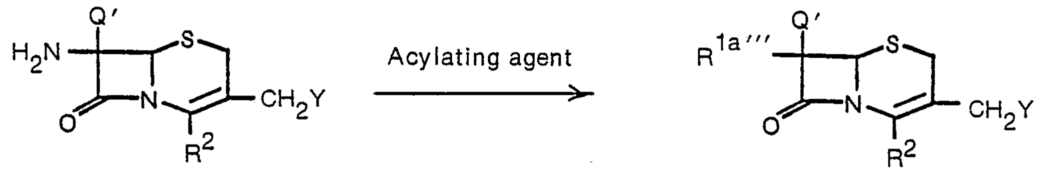


(XXVI)

or its reactive derivative at the amino group or a salt thereof

(IIa)

or a salt thereof

PROCESS K

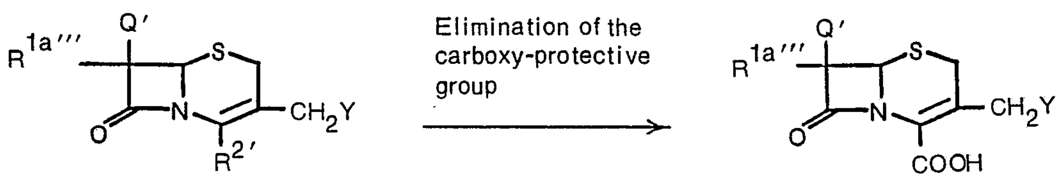


(IIc)

or a salt thereof

(IIb)

or a salt thereof

wherein

$R^1$, $R^{1a}$, $R^2$, $R^{3a}$, $R^{3b}$, $A_2$ and Y are each as defined above;
X is ($C_1$ to $C_6$)alkylene;
$R^4$ is hydrogen or a ($C_1$ to $C_6$) aliphatic hydrocarbon group;
Z is a protected amino group;
B is alkenylene with up to 6C-atoms;
B' is alkenylene with up to 6C-atoms;
$R^5$ is amino or a protected amino group;
$R^6$ is an aliphatic or aromatic group;
$R^8$ and $R^{8'}$ are each amino having a protective group;
$R^{2'}$ is an esterified carboxy group;
Q' is ($C_1$ to $C_6$) alkoxy;
$R^9$ is ($C_1$ to $C_6$) alkyl;
$R^{10a}$ is a mercapto-protective group;
$A_2'$ is ($C_2$ to $C_6$) alkylene;
$R^{11}$ and $R^{12}$ are each an acid residue; and
$R^{1a'''}$ is phenylacetamido having hydroxy and/or carboxy.

Suitable pharmaceutically acceptable salts of the object compounds (I) are conventional non-toxic salt and include a metal salt such as an alkali metal salt (e.g. sodium salt, potassium salt, etc.) and an alkaline earth metal salt (e.g. calcium salt, magnesium salt, etc.), an ammonium salt, an organic base salt (e.g. trimethylamine salt, triethylamine salt, pyridine salt, picoline salt, dicyclohexylamine salt, N,N'-dibenzylethylenediamine salt, etc.), an organic acid salt (e.g. acetate, maleate, tartrate, methanesulfonate, benzenesulfonate, formate, toluenesulfonate, etc.), an inorganic acid salt (e.g. hydrochloride, hydrobromide, sulfate, phosphate, etc.), or a salt with an amino acid (e.g. arginine, aspartic acid, glutamic acid, etc.), and the like.

In the above and subsequent descriptions of the present specification, suitable examples and illustrations of the various definitions which the present invention include within the scope thereof are explained in details as follows.

The term "lower" is intended to mean 1 to 6 carbon atoms, unless otherwise indicated.

The term "halogen" includes fluorine, chlorine, bromine and iodine.

Suitable "protected amino" in the terms "protected amino" for $R^5$ and Z, "acylamino having a protected amino group" for $R^{1a'}$ and "protected amino(lower)alkylene" for $A_1$ and $A_1'$ may include an acylamino or an amino group substituted by a conventional protecting group such as ar(lower)alkyl which may have at least one suitable substituent(s), (e.g. benzyl, trityl, etc.) or the like.

Suitable "acyl" and "acyl moiety" in the term "acylamino" may include carbamoyl, an aliphatic acyl group, an acyl group containing an aromatic ring (hereinafter referred to as aromatic acyl) and an acyl group containing a heterocyclic ring (hereinafter referred to as heterocyclic acyl).

Suitable example of said acyl may be illustrated as follows:—

Aliphatic acyl such as lower or higher alkanoyl (e.g. formyl, acetyl, succinyl, hexanoyl, heptanoyl, stearoyl, etc.);
lower or higher alkoxycarbonyl (e.g. methoxycarbonyl, ethoxycarbonyl, tert-butoxycarbonyl, tert-pentyloxycarbonyl, heptyloxycarbonyl, etc.);
lower or higher alkanesulfonyl (e.g. methanesulfonyl, ethanesulfonyl, etc.); or the like.

Aromatic acyl such as aroyl (e.g. benzoyl, toluoyl, naphthoyl, etc.);
ar(lower)alkanoyl (e.g. phenylacetyl, phenylpropionyl, etc.);
aryloxycarbonyl (e.g. phenoxycarbonyl, naphthyloxycarbonyl, etc.);
ar(lower)alkoxycarbonyl (e.g. benzyloxycarbonyl, phenethyloxycarbonyl, etc.);
aryloxy(lower)alkanoyl (e.g. phenoxyacetyl, phenoxypropionyl, etc.);
arylglyoxyloyl (e.g. phenylglyoxyloyl, naphthylglyoxyloyl, etc.);
arenesulfonyl (e.g. benzenesulfonyl, p-toluenesulfonyl, etc.); or the like;

Heterocyclic acyl such as heterocycliccarbonyl (e.g thenoyl, furoyl, nicotinoyl, etc.);
heterocyclic(lower)alkanoyl (e.g. thienylacetyl, thiazolylacetyl, thiadiazolylacetyl, dithiinylacetyl, pyridylacetyl, pyrimidinylacetyl, triazolylacetyl, tetrazolylacetyl, furylacetyl, oxazolylacetyl, thiazolylpropionyl, etc.);
heterocyclicglyoxyloyl (e.g. thiazolylglyoxyloyl, thienylglyoxyloyl, etc.); or the like.

The acyl and acyl moiety as stated above may have one or more, same or different, suitable substituent(s) such as lower alkyl as mentioned below:
lower alkoxy (e.g. methoxy, ethoxy, propoxy, etc.); lower alkylthio (e.g. methylthio, ethylthio, etc.); lower alkylamino (e.g. methylamino, etc.); halogen (e.g. chlorine, bromine, fluorine or iodine); amino; a protected amino group as mentioned above; hydroxy; a protected hydroxy such as tetrahydropyranyloxy or acyloxy wherein the acyl moiety is as stated above; imino; oxo; carboxy, protected carboxy as mentioned below; a group of the formula: $=N—OR^6$ wherein $R^6$ is as defined above; or the like.

Preferable examples of the "acylamino" in the terms "acylamino" as the suitable substituted amino group for $R^1$, "acylamino" for $R^{1a}$, "acylamino having a protected amino group" for $R^{1a'}$ and "acylamino having an amino group" for $R^{1a''}$ may include phenylacetamido which may have hydroxy and/or carboxy; and the group of the formula:

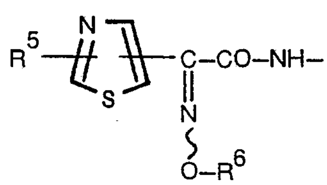

wherein $R^5$ and $R^6$ are each as defined above.

In the connection, as we stated above, it is to be understood that the object compounds (I), (Ia), (Ic), (Id), (Ie) and (If) and the starting compounds (II), (IIa), (IIb), (IIc), (VIa), and (IX) include tautomeric isomers. That is, in case that the group of the formula:

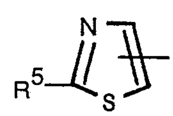

($R^5$ is as defined above) is contained in the molecules of said object and starting compounds, said group of the formula can also be alternatively represented by its tautomeric formula:

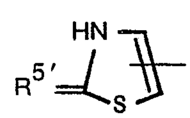

($R^{5'}$ is imino or a protected imino group). That is, the both of said groups are in the state of equilibrium each other and such tautomerism can be represented by the following equilibrium.

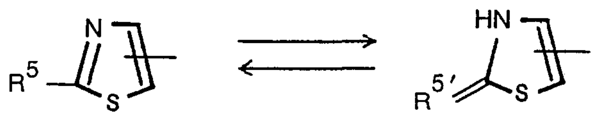

wherein $R^5$ and $R^{5'}$ are each as defined above.

These types of tautomerism between the amino-compound and the corresponding imino-compound as stated above have been well known in the literature, and it is obvious to a person skilled in the arts that both of the tautomeric isomers are easily convertible reciprocally and are included within the same category of the compound *per se*. Accordingly, the both of the tautomeric forms of the object compounds (I), (Ia), (Ic), (Id), (Ie) and (If) are clearly included within the scope of the present invention. In the present specification and claims, the object and starting compounds including the group of such tautomeric isomers are represented by using one of the expressions therefor, that is the formula:

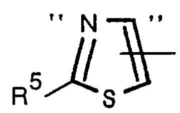

wherein $R^5$ is as defined above.

Furthermore, with regard to the object compounds (I), (Ia), (Ib), (Ic), (Id), (Ie) and (If) and the starting compounds (II), (IIa), (IIb), (IIc), (III), (IIIa), and (VIa), it is also to be noted that where the oximino group(s) is contained in the chemical structure thereof, and these compounds exist as geometrical isomers, i.e. syn isomer, anti isomer and a mixture thereof. For example, with regard to the object compound (I), the syn isomer in the 7-position means on geometrical isomer having the partial structure represented by the following formula:

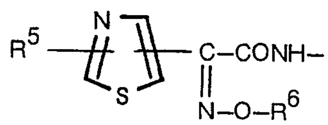

(wherein $R^5$ and $R^6$ are each as defined above) and the anti isomer in the 7-position means the other geometrical isomer having the partial structure represented by the following formula:

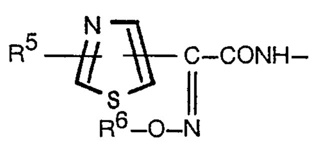

(wherein $R^5$ and $R^6$ are each as defined above).

On the other hand, the syn isomer in the 3-position means one geometrical isomer having the partial structure represented by the following formula:

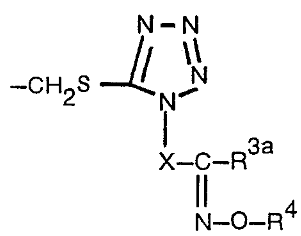

(wherein $R^{3a}$, $R^4$ and X are each as defined above) and the anti isomer in the 3-position means one geometrical isomer having the partial structure represented by the following formula:

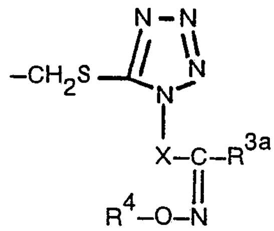

(wherein $R^{3a}$, $R^4$ and X are each as defined above).

Regarding the other object and starting compounds as mentioned above, the syn isomer and the anti isomer can also be referred to the same geometrical isomers as illustrated for the compound (I).

In this regard, the above formula:

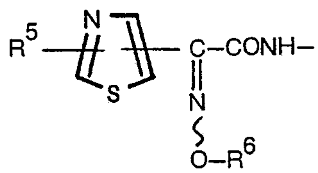

and

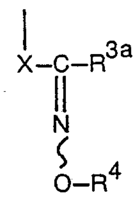

(wherein $R^{3a}$, $R^4$, $R^5$, $R^6$ and X are each as defined above) are intended to represent both of the syn isomer and anti isomer as well as a mixture thereof and both of the respective isomers as well as a mixture thereof are included within the scope of the present invention.

Concerning the starting thiol compounds (III), (IIIa), (IIIb), (IIIc), (IIId), (IIIe), (IIIf), (IIIg), (IIIh), (IIIi), (IV), (V), and (XXV), there are tautomeric isomers as shown by the following equilibrium:

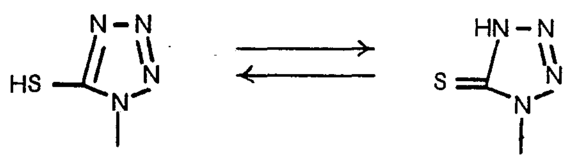

These types of tautomerism between 1-substituted 1H-tetrazole-5-thiol compounds and 1-substituted 4,5-dihydro-1H-tetrazole-5-thione compounds as stated above have been well known in the arts, and it is obvious to a person skilled in the art that both of the tautomeric isomers are equilibrated and lie in the reciprocally convertible state, and accordingly it is to be understood that such isomers are included within the same category of the compound *per se*. Accordingly, both of the tautomeric forms are clearly included within the scope of the present invention. In the present specification, starting compounds including the group of such tautomeric isomers are represented by using one of the expressions therefor, i.e. 1-substituted 1H-tetrazole-5-thiol and the formula:

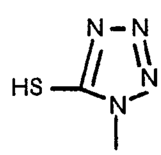

only for convenience.

Suitable "aliphatic and aromatic group" for $R^6$ may include, for example,

lower alkyl (e.g. methyl, ethyl, propyl, isopropyl, butyl, isobutyl, tert-butyl, pentyl, neopentyl, tert-pentyl, hexyl, etc.);

lower alkenyl (e.g. vinyl, 1-propenyl, allyl, 1-methylallyl, 1 or 2 or 3-butenyl, 1 or 2 or 3 or 4-pentenyl, 1 or 2 or 3 or 4 or 5-hexenyl, etc.);

lower alkynyl (e.g. ethynyl, 1-propynyl, 2-propynyl, 1-methyl-2-propynyl, 1 or 2 or 3-butynyl, 1 or 2 or 3 or 4-pentynyl, 1 or 2 or 3 or 4 or 5-hexynyl, etc.);

cycloalkyl (e.g. cyclopropyl, cyclobutyl, cyclopentyl, cyclohexyl, cycloheptyl, etc.);

cyclo(lower)alkenyl (e.g. cyclopropenyl, cyclobutenyl, cyclopentenyl, cyclohexenyl, etc.);

aryl (e.g. phenyl, tolyl, xylyl, cumenyl, naphthyl, etc.);

ar(lower)alkyl such as phenyl(lower)alkyl (e.g. benzyl, phenethyl, phenylpropyl, etc.);

ar(lower)alkyl having at least one suitable substituent(s) such as halogen as mentioned above (e.g. chlorobenzyl, dichlorobenzyl, trichlorobenzyl, fluorobenzyl, fluorophenetyl, fluorophenylpropyl, etc.) or the like.

Suitable esterified carboxy for $R^2$, $R^{2'}$ and $R^{3a}$ may include lower alkyl ester (e.g. methyl ester, ethyl ester, propyl ester, isopropyl ester, butyl ester, isobutyl ester, pentyl ester, hexyl ester, 1-cyclopropylethyl ester, etc.) which may have at least one suitable substituent(s), for example, lower alkanoyloxy(lower)alkyl ester (e.g. acetoxymethyl ester, propionyloxymethyl ester, butyryloxymethyl ester, valeryloxymethyl ester, pivaloyloxymethyl ester, 2-acetoxyethyl ester, 2-propionyloxyethyl ester, hexanoyloxymethyl ester, etc.), lower alkanesulfonyl(lower)alkyl ester (e.g. 2-mesylethyl ester, etc.) or mono(or di or tri)-halo(lower)alkyl ester (e.g. 2-iodoethyl ester, 2,2,2-trichloroethyl ester, etc.); lower alkenyl ester (e.g. vinyl ester, allyl ester, etc.); lower alkynyl ester (e.g. ethynyl ester, propynyl ester, etc.); ar(lower)alkyl ester which may have at least one suitable substituent(s) (e.g. benzyl ester, 4-methoxybenzyl ester, 4-nitrobenzyl ester, phenethyl ester, trityl ester, diphenylmethyl ester, bis-(methoxyphenyl)methyl ester, 3,4-dimethoxybenzyl ester, 4-hydroxy-3,5-ditertiarybutylbenzyl ester, etc.); aryl ester which may have at least one suitable substituent(s) (e.g. phenyl ester, 4-chlorophenyl ester, tolyl ester, tertiarybutylphenyl ester, xylyl ester, mesityl ester, cumenyl ester, etc.), and the like.

Preferable examples of the esterified carboxy as mentioned above may include ($C_1$ to $C_6$)alkoxycarbonyl (e.g. methoxycarbonyl, ethoxycarbonyl, propoxycarbonyl, isopropoxycarbonyl, butoxycarbonyl, isobutoxycarbonyl, tert-butoxycarbonyl, pentyloxycarbonyl, tert-pentyloxycarbonyl, hexyloxycarbonyl, 1-cyclopropylethoxycarbonyl, etc.) and phenyl ($C_1$ to $C_6$)alkoxycarbonyl (e.g. benzyloxycarbonyl, diphenylmethoxycarbonyl, etc.).

Suitable "($C_1$ to $C_6$)alkylene" in the terms "hydroxy($C_1$ to $C_6$)alkylene", "amino($C_1$ to $C_6$)alkylene", "protected amino($C_1$ to $C_6$)alkylene", "hydroxyimino($C_1$ to $C_6$)alkylene wherein the hydrogen atom of the hydroxyimino group may be replaced with a ($C_1$ to $C_6$)aliphatic hydrocarbon group" for $A_1$, a protected amino($C_1$ to $C_6$)alkylene for $A_1'$, amino($C_1$ to $C_6$)alkylene for $A_1''$ ($C_1$ to $C_6$)alkylene for X and $A_2'$, and ($C_1$ to $C_6$)alkylene which may have an oxo group for $A_2$ may include straight or branched one and may include methylene, ethylene, trimethylene, propylene, tetramethylene, pentamethylene, hexamethylene and the like.

Suitable ($C_1$ to $C_6$)aliphatic hydrocarbon group" in the terms "hydroxyimino($C_1$ to $C_6$)alkylene wherein the hydrogen atom of the hydroxyimino group may be replaced with a ($C_1$ to $C_6$)aliphatic hydrocarbon group" for $A_1$ and "a ($C_1$ to $C_6$)aliphatic hydrocarbon group" for $R^4$ may include a monovalent radical of a saturated or unsaturated, and straight or branched acyclic aliphatic hydrocarbon, and particularly may include ($C_1$ to $C_6$)alkyl, alkenyl with up to 6C-atoms, alkynyl with up to 6C-atoms and the like, each of which is exemplified for $R^6$.

Suitable "alkenylene with up to 6C-atoms" for $A_1$, B and B' means straight or branched one and may include alkenylene with up to 6C-atoms (e.g. vinylene, 1-propenylene, 2-propenylene, 1-methyl-2-propenylene, 1 or 2 or 3-butenylene, 1 or 2 or 3 or 4-pentenylene, 1 or 2 or 3 or 4 or 5-hexenylene, etc.) and lower alkenylidene (e.g. vinylidene, propenylidene, butenylidene, pentenylidene, hexenylidene, etc.).

In this connection, with regard to the object compounds (I), (Ia), (Ib), (Ic) and (Id) and starting compounds (III) and (IIIe), it is to be noted that when the group represented by $A_1$ is lower alkenylene, there is a possibility that these compounds exist as geometrical isomers, i.e. cis- and trans-isomers and a mixture thereof. For example, with regard to the object compound (I) wherein the group represented by $—A_1—R^{3a}$ is 3-carboxy-1-propenyl, the cis isomer means one geometrical isomer having the partial structure represented by the following formula:

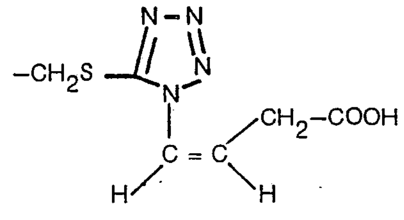

and the trans isomer means the other geometrical isomer having the partial structure represented by the following formula:

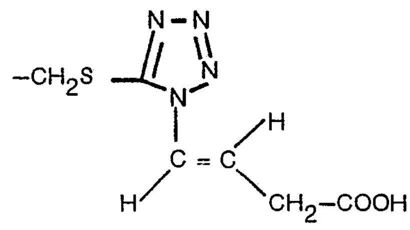

The cis- and trans-isomers as well as a mixture thereof are included within the scope of the present invention.

Suitable "carboxy, esterified carboxy, an aliphatic or aromatic group" for $R^{3b}$ may include for example,

lower alkyl (e.g. methyl, ethyl, propyl, isopropyl, butyl, isobutyl,ryl, isopropyl, butyl, isobutyl, tert-butyl, pentyl, neopentyl, tert-pentyl, hexyl, etc.);

lower alkenyl (e.g. vinyl, 1-propenyl, allyl, 1-methylallyl, 1 or 2 or 3-butenyl, 1 or 2 or 3 or 4-pentenyl, 1 or 2 or 3 or 4 or 5-hexenyl, etc.);

aryl (e.g. phenyl, tolyl, xylyl, cumenyl, naphthyl, etc.);

ar(lower)alkyl such as phenyl(lower)alkyl (e.g. benzyl, phenethyl, phenylpropyl, etc.);

hydroxy(lower)alkyl (e.g., hydroxymethyl, hydroxyethyl, hydroxypropyl, hydroxybutyl, etc.); and the like.

Suitable "lower alkoxy" for Q and Q' may include methoxy, ethoxy, propoxy, isopropoxy, butoxy and the like.

Suitable example of Y may include an acid residue (e.g. azido, halogen as mentioned above, acyloxy as mentioned above, etc.) and the like.

Suitable "amino having a protective group" for $R^8$ and $R^{8'}$ may include phthalimido, succinimido, ethoxycarbonylamino and the like, and preferably phthalimido.

Suitable "lower alkyl" for $R^9$ means straight or branched one and may include methyl, ethyl, propyl, isopropyl, butyl, isobutyl, tert-butyl, pentyl, hexyl and the like.

Suitable "mercapto-protective group" for $R^{10}$ and $R^{10a}$ may include a conventional protective group such as lower alkyl as mentioned above, ar(lower)alkyl, for example, phenyl(lower)alkyl (e.g., benzyl, phenethyl, phenylpropyl, etc.) and the like.

Suitable "acid residue" for $R^{11}$ and $R^{12}$ may include halogen (e.g., chlorine, bromine, iodine etc.), azide, and the like.

The preferable examples of the object compound (I) are exemplified as follows:

Preferable examples of $R^1$ are amino or acylamino, for example, 2-carboxy-2-(hydroxyphenyl)acetamido or a group of the formula:

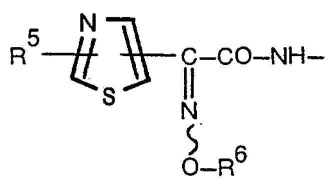

wherein

$R^5$ is amino, lower alkanoylamino or halogen substituted lower alkanoylamino;

$R^6$ is lower alkyl, lower alkenyl, lower alkynyl, cycloalkyl, cyclo(lower)alkenyl, aryl [more preferably, phenyl], ar(lower)alkyl [more preferably, phenyl(lower)alkyl] or ar(lower)alkyl having halogen [more preferably, phenyl(lower)alkyl having halogen];

$R^2$ is carboxy;

R is a group of the formula: $—A_1—R^{3a}$

wherein

$A_1$ is hydroxy(lower)alkylene, amino(lower)alkylene, acylamino(lower)alkylene [more preferably lower alkoxycarbonylamino(lower)alkylene], hydroxyimino(lower)alkylene, lower alkoxyimino(lower)alkylene, lower alkenyloxyimino(lower)alkylene, lower alkenylene or lower alkenylidene,

$R^{3a}$ is carboxy; or a group of the formula:

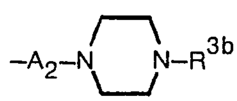

wherein

$A_2$ is lower alkylene or lower alkylene having oxo,

$R^{3b}$ is carboxy, esterified carboxy [more preferably lower alkoxycarbonyl], lower alkyl, lower alkenyl, hydroxy(lower)alkyl, ar(lower)alkyl [more preferably phenyl(lower)alkyl], aryl [more preferably phenyl];

Q is hydrogen or lower alkoxy.

The use of the term "lower" hereinabove shall be understood to refer to a C-atom number of up to six.

The processes for preparing the object compounds of the present invention are explained in detail in the following.

*Process 1:*

The object compound (I) or a salt thereof can be prepared by reacting the compound (II) or a salt thereof with the compound (III) or its reactive derivative at the mercapto group or a salt thereof.

Suitable salts of the compound (II) and (III) are each referred to the ones exemplified for the compound (I).

Suitable reactive derivative at the mercapto group in the compound (III) may include a metal salt such as an alkali metal salt (e.g. sodium salt, potassium salt, etc.), an alkaline earth metal salt (e.g., magnesium salt, etc.) or the like.

The reaction is usually carried out in a solvent such as water, acetone, chloroform, nitrobenzene, methylene chloride, ethylene chloride, dimethylformamide, methanol, ethanol, ether, tetrahydrofuran or any other conventional solvents which do not adversely influence the reaction, preferably in ones having strong polarity, which may be used as a mixture with water.

When the compound (II) and/or the compound (III) are used in free form in the reactions, the reaction is preferably carried out in the presence of a base such as alkali metal hydroxide, alkali metal carbonate, alkali metal bicarbonate, trialkylamine, pyridine, or a Lewis acid such as boron trifluoride or the like, and preferably carried out around neutral conditions. The reaction temperature is not critical and the reaction is usually carried out at ambient temperature or under warming.

The present invention includes, within its scope, the cases that a protected amino and/or a protected carboxy group are converted into the corresponding free amino and/or the free carboxy group during the reaction or the post-treating step of the present process.

*Process 2*

The object compound (Ia) or a salt thereof can be prepared by reacting the compound (Ib) or its reactive derivatives at the amino group or a salt thereof with an acylating agent.

Suitable reactive derivatives at the amino group of the compound (Ib) may include conventional ones such as Schiff's base type imino or its tautomeric enamine type derivatives formed by the reaction of the compound (Ib) with a carbonyl compound (e.g. aldehyde, ketone, etc.), isocyanate;

silyl derivatives formed by the reaction of the compound (Ib) with a silyl compound [e.g. bis(trimethylsilyl)acetamide, trimethylsilylacetamide, etc.];

derivatives formed by the reaction of the compound (Ib) with phosphorus trichloride or phosgene, or the like.

Suitable salts of the compound (Ib) can be referred to the ones as exemplified for the compound (I).

The acylating agent to be used for the present reaction may include one of the formulae:

$$R^7—OH \qquad (VI)$$

wherein $R^7$ is acyl, or its reactive derivatives or a salt thereof.

Suitable acyls can be referred to those exemplified hereinbefore.

Suitable reactive derivatives of the compound (VI) may include an acid halide, an acid anhydride, an activated amide, an activated ester, and the like.

The suitable example may be an acid chloride; an acid azide;

a mixed acid anhydride with an acid such as substituted phosphoric acid (e.g. dialkylphosphoric acid, phenylphosphoric acid, diphenylphosphoric acid, dibenzylphosphoric acid, halogenated phosphoric acid, etc.), dialkylphosphorous acid, sulfurous acid, thiosulfuric acid, sulfuric acid, alkylcarbonic acid, aliphatic carboxylic acid (e.g. pivalic acid, pentanoic acid, isopentanoic acid, 2-ethylbutyric acid or trichloroacetic acid, etc.) or aromatic carboxylic acid (e.g. benzoic acid, etc.);

a symmetrical acid anhydride;

an activated amide with imidazole, 4-substituted imidazole, dimethylpyrazole, triazole or tetrazole; an activated ester [e.g. cyanomethyl ester, methoxymethyl ester, dimethyliminomethyl

$$[(CH_3)_2\overset{+}{N}=CH—] \text{ ester,}$$

vinyl ester, propargyl ester, p-nitrophenyl ester, 2,4-dinitrophenyl ester, trichlorophenyl ester, pentachlorophenyl ester, mesyl-phenyl ester, phenylazophenyl ester, phenyl thioester, p-nitrophenyl thioester, p-cresyl thioester, carboxymethyl thioester, pyranyl ester, pyridyl ester, piperidyl ester, 8-quinolyl thioester, or an ester with N,N-dimethylhydroxylamine, 1-hydroxy-2-(1H)pyridone, N-hydroxysuccinimide, N-hydroxyphthalimide or 1-hydroxy-6-chloro-1H-benzotriazole, and the like.

These reactive derivatives can optionally be selected from them according to the kind of the compound (VI) to be used.

The salts of the compound (VI) may be salt with an inorganic base such as an alkali metal salts (e.g. sodium or potassium salt) or an alkaline earth metal salt (e.g. calcium or magnesium salt), a salt with an organic base such as trimethylamine, triethylamine, dicyclohexylamine or the like.

The present reaction is usually carried out in a conventional solvent such as water, acetone, dioxane, acetonitrile, chloroform, methylene chloride, ethylene chloride, tetrahydrofuran, ethyl acetate, N,N-dimethylformamide, pyridine or any other organic solvents which do not adversely influence the reaction.

Among these solvents, hydrophilic solvents may be used in a mixture with water.

When the acylating agent is used in a free acid form or its salt form in the reaction, the reaction is preferably carried out in the presence of a conventional condensing agent such as carbodiimide compound (e.g. N,N'-dicyclohexylcarbodiimide, N-cyclohexyl-N'-morpholinoethylcarbodiimide, N-cyclohexyl-N'-(4-diethylaminocyclohexyl)carbodiimide, N,N'-diethylcarbodiimide, N,N'-diisopropylcarbodiimide, N-ethyl-N'-(3-dimethylaminopropyl)carbodiimide, etc.), N,N'-carbonylbis(2-mesylimidazole), pentamethylene-ketene-N-cyclohexylimine, diphenylketone-N-cyclohexylimine, alkoxyacetylene, 1-alkoxy-1-chloroethylene, trialkyl phosphite, ethyl polyphosphate, isopropyl polyphosphate, phosphorus

oxychloride, phosphorus trichloride, thionyl chloride, oxalyl chloride, triphenylphosphine, N-ethylbenzisooxazolium salt, N-ethyl-5-phenyl-isoxazolium-3'-sulfonate, 1-(p-chlorobenzenesulfonyloxy)-6-chloro-1H-benzotriazole, Vilsmeier reagent [e.g. (chloromethylene)dimethylammonium chloride, a compound formed by the reaction of dimethylformamide with phosphorus oxychloride, etc.] or the like.

The reaction may also be carried out in the presence of an inorganic or an organic base such as an alkali metal bicarbonate, alkali metal carbonate, tri(lower)alkylamine, pyridine, di(lower)alkylpyridine, N-(lower)alkylmorphorine, N,N-di(lower)alkylbenzylamine, N,N-di(lower)alkylaniline, or the like. When the base or the condensing agent is in liquid, it can be used also as a solvent. The reaction temperature is not critical, and the reaction is usually carried out under cooling or at ambient temperature.

*Process 3*

The object compound (Ic) or a salt thereof can be prepared by subjecting the compound (Id) or a salt thereof to the elimination reaction of the amino protective group.

Suitable salts of the compound (Id) may include a metal salt, ammonium salt, an organic amine salt and the like as aforementioned.

The present elimination reaction is carried out in accordance with a conventional method such as hydrolysis; reduction; elimination using a Lewis acid; a method by reacting the compound (Id), wherein the protective group is an acyl group, with an iminohalogenating agent and then with an iminoetherifying agent, and, if necessary, subjecting the resulting compound to hydrolysis; or the like.

The hydrolysis may include a method using an acid or base or hydrazine and the like. These methods may be selected depending on the kind of the protective groups to be eliminated.

Among these methods, hydrolysis using an acid is one of the common and preferable methods for elimination of an acyl group.

Suitable acid may include an organic or an inorganic acid, for example, formic acid, trifluoroacetic acid, benzenesulfonic acid, p-toluenesulfonic acid, hydrochloric acid and the like, and preferable acids are those which can easily be removed from the reaction mixture by a conventional manner such as distillation under reduced pressure, for example, formic acid, trifluoroacetic acid, hydrochloric acid, etc. The acid suitable for the reaction can be selected according to the kind of protective groups to be eliminated. When the elimination reaction is conducted with an acid, it can be carried out in the presence or absence of a solvent. Suitable solvents may include an organic solvent, water or a mixed solvent thereof. When trifluoroacetic acid is used, the elimination reaction may preferably be carried out in the presence of anisole.

The hydrolysis using hydrazine is commonly applied for eliminating the protective groups, for example, succinyl or phthaloyl.

The hydrolysis using a base is preferably applied for elimination of an acyl group. Suitable base may include, for example, an inorganic base such as alkali metal hydroxide (e.g. sodium hydroxide, potassium hydroxide, etc.), alkaline earth metal hydroxide (e.g. magnesium hydroxide, calcium hydroxide, etc.), alkali metal carbonate (e.g. sodium carbonate, potassium carbonate, etc.), alkaline earth metal carbonate (e.g. magnesium carbonate, calcium carbonate, etc.), alkali metal bicarbonate (e.g. sodium bicarbonate, potassium bicarbonate, etc.), alkali metal acetate (e.g. sodium acetate, potassium acetate, etc.), alkaline earth metal phosphate (e.g. magnesium phosphate, calcium phosphate, etc.), alkali metal hydrogen phosphate (e.g. disodium hydrogen phosphate, dipotassium hydrogen phosphate, etc.), or the like, and an organic base such as trialkylamine (e.g. trimethylamine, triethylamine, etc.), picoline, N-methylpyrrolidine, N-methylmorpholine, 1,5-diazabicyclo[4,3,0]non-5-ene, 1,4-diazabicyclo[2,2,2]octane, 1,5-diazabicyclo[5,4,0]undecene-5 or the like. The hydrolysis using a base is often carried out in water or a hydrophilic organic solvent or a mixed solvent thereof.

Among the protective group, the acyl group can generally be eliminated by hydrolysis as mentioned above or by the other conventional hydrolysis. In case that the acyl group is halo(lower)alkoxycarbonyl or 8-quinolyloxycarbonyl, they are eliminated by treating with a heavy metal such as copper, zinc or the like.

The reductive elimination is generally applied for eliminating the protective group, for example, halo(lower or higher)alkoxycarbonyl (e.g. trichloroethoxycarbonyl, etc.), substituted or unsubstituted ar(lower)alkoxycarbonyl (e.g. benzyloxycarbonyl, etc.), etc. Suitable reduction may include, for example, reduction with an alkali metal borohydride (e.g. sodium borohydride, etc.), reduction using a combination of a metal (e.g. zinc, zinc amalgam, etc.) and an organic or inorganic acid (e.g. acetic acid, propionic acid, hydrochloric acid, etc.), catalytic reduction, and the like.

Suitable iminohalogenating agents used in a method as mentioned above may include phosphorus halide (e.g. phosphorus trichloride, phosphorus pentachloride, phosphorus tribromide, phosphorus pentabromide, etc.), phosphorus oxychloride, thionyl chloride, phosgene and the like. The reaction temperature is not critical, and the reaction is usually carried out at ambient temperature or under cooling. Suitable iminoetherifying agents reacted with thus obtained reaction product may include an alcohol, metal alkoxide and the like. Suitable alcohol may include alkanol (e.g. methanol, ethanol, propanol, isopropanol, butanol, tert-butanol, etc.) which may be substituted with alkoxy (e.g. methoxy, ethoxy, propoxy, isopropoxy, butoxy, etc.). Suitable metal alkoxide may include alkali metal alkoxide (e.g. sodium alkoxide, potassium alkoxide, etc.), alkaline earth metal alkoxide (e.g. calcium alkoxide, barium alkoxide, etc.) and the like.

The reaction temperature is not critical, and the reaction is usually carried out under cooling at ambient temperature or under warming.

Process 4

The object compound (Ie) or a salt thereof can be prepared by subjecting the compound (If) or a salt thereof to the elimination reaction of the amino protective group.

Suitable salts of the compound (If) can be referred to the ones as exemplified for the compound (I).

The present elimination reaction is carried out substantially in the same manner as illustrated in Process 3.

The processes for preparing the starting compounds (III) are explained in detail as follows.

Suitable reactive derivative at the mercapto group of the compounds (V), (IV), (IIIa), (IIIb), (IIIc), (IIId), (IIIe) and (IIIf) can be referred to the ones as exemplified for the compound (III).

Suitable salts of the compounds (IV), (IIIa), (IIIb), (IIIc), (IIIe) and (XXV) are each referred to the ones as exemplified for the compound (VI).

Suitable salts of the compounds (IIa), (IIb), (IIc), (IIId), (IIIg), (IIIi), (VIa), (IX), (XV) and (XXVI) are each referred to the ones as exemplified for the compound (I).

Suitable salts of the compounds (IIIh), (VIII), (X), (XIII), (XVI), (XVII), (XIX), (XX), (XXII) and (XXIV) may include an inorganic acid salt (e.g. hydrochloride, hydrobromide, sulfate, etc.), an organic acid salt (e.g. acetate, p-toluenesulfonate, etc.) and the like.

Process A. (V) → (IV)

The compound (IV) or its reactive derivative at the mercapto group or a salt thereof can be prepared by reacting the compound (V) or its reactive derivative at the mercapto group with the compound (VII) or its ester.

Suitable esters of the compound (VII) are referred to the ones exemplified for the ester moiety of the esterified carboxy groups represented by $R^2$ and $R^3$.

The present reaction may be carried out in the presence of a base such as alkyl alkali metal (e.g. n-butyllithium, etc.), alkali metal acetate (e.g. sodium acetate, etc.), alkali metal alkoxide (e.g. sodium methoxide, potassium tert-butoxide, etc.), alkali metal hydride (e.g. sodium hydride, etc.), alkali metal hydroxide, alkaline earth metal hydroxide or the like.

The present reaction is usually carried out in a solvent such as tetrahydrofuran or any other solvents which do not adversely affect the reaction.

The reaction temperature is not critical and the reaction is preferably carried out under cooling or at ambient temperature.

Process B

i): optional process

The compound (IV) or its reactive derivative at the mercapto group or a salt thereof wherein $R^3$ is a protected carboxy group may, if desired, be converted into its free form by elimination of the carboxy protective group,

prior to: ii) introduction of the substituent: $=N-OR^4$; or
iii) conversion of the —CO— group to the —CH(OH) group.

In the present elimination reaction, conventional methods used in the elimination reaction of the carboxy protective group, for example, hydrolysis, reduction, elimination using Lewis acid, etc. are applicable. When the carboxy protective group is an ester, it can be eliminated by hydrolysis or elimination using Lewis acid. The hydrolysis is preferably carried out in the presence of a base (e.g. sodium hydroxide, etc.) or an acid (e.g. hydrochloric acid, formic acid, etc.).

The present reaction is usually carried out in a solvent such as alcohol (e.g. methanol, etc.) or any other solvents which do not adversely affect the reaction.

The reaction temperature is not critical and the reaction is preferably carried out under cooling, at ambient temperature or under warming.

ii) (IV) + (VIII) → (IIIa)

The compound (IIIa) or its reactive derivative at the mercapto group or a salt thereof can be prepared by reacting the compound (IV) or its reactive derivative at the mercapto group or a salt thereof with the compound (VIII) or a salt thereof.

The reaction is usually carried out in a conventinal solvent such as water, alcohol (e.g., methanol, ethanol, etc.), a mixture thereof or any other ones which do not adversely influence the reaction.

When the compound (VIII) is used in its salt form, the reaction is preferably carried out in the presence of an organic or an inorganic base as exemplified before.

The reaction temperature is not critical, and the reaction is usually carried out from cooling to heating.

iii) (IV) → (IIIb)

i) The compound (IIIb) or its reactive derivative at the mercapto group or a salt thereof can be prepared

by reducing the compound (IV) or its reactive derivative at the mercapto group or a salt thereof.

The present reduction may be carried out by a conventional method which is applied to the reduction of the —CO— group to the corresponding —CH(OH)— group, for example, by using a combination of a metal (e.g. zinc, etc.) and an acid (e.g. formic acid, acetic acid, hydrochloric acid, etc.) or a base (e.g. sodium hydroxide, etc.), alkali metal borohydride (e.g. lithium borohydride, etc.), metal amalgam (e.g. aluminum amalgam, etc.) or the like.

The present reaction is preferably carried out in a solvent such as alcohol (e.g. ethanol, etc.) or any other solvents which do not adversely affect the reaction.

The reaction temperature is not critical and the reaction is preferably carried out under cooling, at ambient temperature or under warming.

Process C

i) (IIIa) → (IIId)

The compound (IIId) or its reactive derivative at the mercapto group or a salt thereof can be prepared by reducing the compound (IIIa) or its reactive derivative at the mercapto group or a salt thereof.

The present reduction can be carried out by the methods explained in Process B iii), among which the method of using a combination of a metal (e.g. zinc, etc.) and an acid (e.g. formic acid, hydrochloric acid, etc.) are preferred.

ii) (IIId) → (IIIc)

a) The compound (IIIc) or its reactive derivative at the mercapto group or a salt thereof can be prepared by subjecting the compound (IIId) or its reactive derivative at the mercapto group or a salt thereof to the introduction reaction of the amino group.

The present introduction reaction can be carried out by reacting the compound (IIId) or its reactive derivative at the mercapto group or a salt thereof with an acylating agent. Suitable acylating agent may include a) $R^{13}$—OH (XXVII) wherein $R^{13}$ is acyl as defined above) or its reactive derivatives or a salt thereof as explained in Process 2.

b) an introducing agent of lower alkoxycarbonyl group, for example, 2-(lower)alkoxycarbonyloxyimino-2-cyanoacetamide (e.g., 2-ethoxycarbonyloxyimino-2-cyanoacetamide, etc.), di(lower)alkyl 2-(lower)alkoxycarbonyloxyiminomalonate (e.g. diethyl 2-tertbutoxycarbonyloxyiminomalonate, etc.), lower alkyl 2-(lower)alkoxycarbonyloxyimino-2-cyanoacetate (e.g. ethyl 2-isobutoxycarbonyloxyimino-2-cyanoacetate, etc.), lower alkyl 2-(lower)alkoxycarbonyloxyiminoacetoacetate (e.g. ethyl 2-tert-butoxycarbonyloxyiminoacetoacetate, etc.), lower alkoxycarbonyloxyimino-2-phenylacetonitrile (e.g. tert-butoxycarbonyloxyimino-2-phenylacetonitrile, etc.) or the like, and the like.

The substantially same reaction conditions as set out in Process 2 can be applied in this reaction.

Process D (IIIf) → (IIIe)

The compound (IIIe) or its reactive derivative at the mercapto group or a salt thereof can be prepared by subjecting the compound (IIIf) or its reactive derivative at the mercapto group to the reaction to introduce a carboxy group; whereafter, if necessary, the introduction of a carboxy protective group is carried out.

The present reaction can be carried out by a conventional method which is applied for the introduction of a carboxy group to the alkenyl group, for example by reacting the compound (IIIf) with dry ice or carbon dioxide, or the like.

The present reaction is usually carried out in the presence of a base as exemplified in Process A.

The present reaction is usually carried out in a solvent such as tetrahydrofuran, hexan or any other solvents which do not adversely affect the reaction. The reaction temperature is not critical and the reaction is preferably carried out under cooling or at ambient temperature.

Process E

i) (XI) → (X)

The compound (X) or a salt thereof can be prepared by subjecting a compound (XI) to elimination reaction of the amino protective group.

This elimination reaction of the amino protective group of the compound (XI) can be carried out in a similar manner to that of aforementioned Process 3.

Suitable solvents include water, ethanol, chloroform, diethyl ether and the like. The reaction temperature is not critical and the reaction is usually carried out under warming or heating.

ii) (X) + (IX) → (VIa)

The compound (VIa) or a salt thereof can be prepared by reacting a compound (X) or a salt thereof with a compound (IX) or a salt thereof.

The reaction is usually carried out in a conventional solvent such as water, alcohol (e.g. methanol, ethanol, etc.), a mixture thereof or any other ones which do not adversely influence the reaction.

When the compound (X) is used in its salt form, the reaction is preferably carried out in the presence of an organic or an inorganic base as exemplified before.

The reaction temperature is not critical, and the reaction is usually carried out from cooling to heating.

In the present reaction, a syn isomer of the compound (VIa) can be obtained preferably by conducting the present reaction under around neutral conditions.

Process F

1) (XIX) + (XVIII) → (XVII)

The compound (XVII) or a salt thereof can be prepared by reacting the compound (XIX) or a salt thereof with the compound (XVIII).

The present reaction may be carried out in the presence of an organic or inorganic base such as alkali metal bicarbonate, alkali metal carbonate, alkaline earth metal bicarbonate, alkaline earth metal carbonate, alkali metal hydroxide, alkaline earth metal hydroxide, tri(lower)alkylamine, N, N-di(lower)alkyl arylamine, pyridine or the like.

The present reaction is usually carried out in a solvent such as acetone, N, N-dimethylformamide or any other solvents which do not adversely affect the reaction.

The reaction temperature is not critical and the reaction is preferably carried out from at ambient temperature to under heating.

2) (XVII) → (XVI)

The compound (XVI) or a salt thereof can be prepared by subjecting the compound (XVII) or a salt thereof to the elimination of the amino-protective group.

This elimination reaction can be carried out in a similar manner to that of aforementioned Process 3.

3) (XVI) → (XV)

The compound (XV) or a salt thereof can be prepared by reacting the compound (XVI) or a salt thereof with carbon disulfide.

The present reaction is usually carried out in a solvent such as alcohol (e.g., methanol, etc.) or any other solvents which do not adversely affect the reaction.

The reaction temperature is not critical and the reaction is preferably carried out under cooling to warming.

4) (XV) + (XIV) → (XIII)

The compound (XIII) or a salt thereof can be prepared by reacting the compound (XV) or a salt thereof with a lower alkylating agent (XIV).

Suitable "lower alkylating agent" may include lower alkyl halide (e.g. methyl iodide, methyl chloride, methyl bromide, ethyl iodide, etc.) and the like.

The present reaction is preferably carried out in a solvent such as alcohol (e.g. ethanol, etc.) or any other solvents which do not adversely affect the reaction.

The reaction temperature is not critical and the reaction is preferably carried out under cooling to warming.

5) (XIII) + (XII) → (III g)

The compound (III g) or a salt thereof can be prepared by reacting the compound (XIII) or a salt thereof with a hydrazoic acid salt (XII).

Suitable "hydrazoic acid salt" may include an alkali metal azido (e.g. sodium azido, potassium azido, etc.) and the like.

The present reaction is usually carried out in a solvent such as water, alcohol (e.g. methanol, ethanol, etc.) or a mixed solvent thereof or any other solvents which do not adversely affect the reaction.

The reaction temperature is not critical and the reaction is preferably carried out from at ambient to under heating.

Process G

1) (XXIV) + (XXIII) → (XXII)

The compound (XXII) or a salt thereof can be prepared by reacting the compound (XXIV) or a salt thereof with the compound (XXIII).

The present reaction is usually carried out in a solvent such as water, alcohol (e.g., methenol, ethanol, etc.) or a mixed solvent thereof, or any other solvents which do not adversely affect the reaction.

The reaction temperature is not critical and the reaction is preferably carried out under cooling to warming.

2) (XXII) → (XXI)

The compound (XXI) can be prepared by subjecting the compound (XXII) or a salt thereof to the diazotization reaction.

The present diazotization can be carried out by using a conventional diazotizing agent such as nitrous acid, [which may be prepared in situ by reaction of an alkali metal nitrite with an acid (e.g. hydrochloric acid, etc.)] nitrosyl-chloride, lower alkyl nitrite (e.g. t-butyl nitrite, etc.) or the like.

The present reaction may be carried out in the presence of an organic or inorganic acid such as hydrochloric acid, acetic acid or the like.

The present reaction may be carried out in a solvent such as alcohol (e.g., methanol, ethanol, etc.) or any other solvents which do not adversely affect the reaction.

The reaction temperature is not critical and the reaction is preferably carried out under cooling or at ambient temperature.

(3) (XXI) + (XIX) → (XX)

The compound (XX) or a salt thereof can be prepared by reacting the compound (XXI) or its reactive derivative at the hydroxy group with the compound (XIX) or a salt thereof.

Suitable reactive derivative at the hydroxy group of the compound (XXI) may include the compound (XXI) wherein the hydroxy group is transformed into an acid residue such as halogen (e.g., chlorine, bromine, etc.), arenesulfonyloxy (e.g., p-toluenesulfonyloxy, p-nitrobenzenesulfonyl, etc.), haloformyloxy (e.g., chloroformyloxy, etc.) or the like.

The present reaction can be carried out in a similar manner to that of aforementioned Process F-1).

4) (XX) → (IIIg)

The compound (IIIg) or a salt thereof can be prepared by subjecting the compound (XX) or a salt thereof to the elimination reaction of the mercapto-protective group.

The present elimination reaction may be carried out in accordance with a conventional method such as hydrolysis using an organic or inorganic acid (e.g. acetic acid, hydrobromic acid, etc.) or the like.

The present reaction is usually carried out in a solvent such as water or any other solvents which do not adversely affect the reaction.

The reaction temperature is not critical and the reaction is preferably carried out under cooling to heating.

Process H (IIIh) → (IIIg)

The compound (IIIg) or a salt thereof can be prepared by isomerizing the compound (IIIh) or a salt thereof.

The present isomerization may be carried out in the presence of an organic or inorganic acid (e.g., hydrochloric acid, acetic acid, etc.).

The present reaction is usually carried out in a solvent such as water or any other solvents which do not adversely affect the reaction.

The reaction temperature is not critical and the reaction is preferably carried out under cooling to warming.

Process I (XXV) + (XIX) → (IIIi)

The compound (IIIi) or a salt thereof can be prepared by reacting the compound (XXV) or its reactive derivative at the carboxy group or a salt thereof with the compound (XIX) or its reactive derivative at the amino group or a salt thereof.

The present reaction can be carried out in a similar manner to that of aforementioned Process 2.

Process J (XXVI) → (IIa)

The compound (IIa) or a salt thereof can be prepared by reacting the compound (XXVI) or its reactive derivative at the amino group or a salt thereof with an acylating agent.

The present reaction can be carried out in a similar manner to that of aforementioned Process 2.

Process K (IIc) → (IIb)

The compound (IIb) or a salt thereof can be prepared by subjecting the compound (IIc) or a salt thereof to the elimination reaction of the carboxy-protective group.

The present elimination reaction is carried out in accordance with a connectional method such as hydrolysis, elimination using Lewis acid [preferably in the presence of cation trapping agents (e.g., anisole, etc.)], reduction or the like.

The present reaction is usually carried out in a solvent such as nitroalkane (e.g., nitromethane, etc.), alkylene halide (e.g. methylene chloride, etc.) or any other solvents which do not adversely affect the reaction.

The reaction temperature is not critical and the reaction is preferably carried out under cooling or warming.

The present invention includes, within its scope, the cases that protected amino and/or protected carboxy and/or protected hydroxy group(s) are transformed into the corresponding free amino and/or carboxy and/or hydroxy group(s) according to the reaction conditions and kinds of the protective groups in the course of the aforementioned reactions and/or in post-treatment of the reactions in Processes 1 to 4 and A to K.

In the aforementioned reactions and/or the post-treating of the reactions in Processes 1 to 4 and A to K of the present invention, the aforementioned geometrical isomer and/or tautomeric isomer may

occasionally be transformed into the other geometrical isomer and/or tautomeric isomer and such cases are to be also included in the scope of the present invention.

In case that the object compound (I) has a free carboxy group and/or a free amino group, it may be transformed into its pharmaceutically acceptable salt as aforementioned by a conventional method.

The object compound (I) of the present invention exhibits high antimicrobial activity and inhibits the growth of a number of microorganisms including pathogenic Gram-positive and Gram-negative bacteria.

For therapeutic administration, the cephalosporin compounds according to the present invention are used in the form of pharmaceutical preparation which contain said compounds in admixture with a pharmaceutically acceptable carriers such as an organic or inorganic solid or liquid excipient suitable for oral, parenteral or external administration. The pharmaceutical preparations may be in solid form such as capsule, tablet, dragee, ointment or suppository, or in liquid form such as solution, suspension, or emulsion. If desired, there may be included in the above preparations auxiliary substances, stabilizing agents, wetting or emulsifying agents, buffers and other commonly used additives.

While the dosage of the compounds may vary from and also depend upon the age and condition of the patient, an average single dose of about 50 mg., 100 mg., 250 mg., and 500 mg. of the compounds according to the present invention has proved to be effective for treating of infectious diseases caused by a number of pathogenic bacteria. In general amounts, daily dose between 1 mg/body and about 1000 mg/body or even more may be administered.

Now, in order to show the utility of the object compounds (I), test data on anti-microbial activity of a representative compound of the present invention are shown below.

Test method

One loopful of an overnight culture of each test strain in Trypticase-soy broth ($10^8$ viable cells per ml.) was streaked on heart infusion agar (HI-agar) containing graded concentrations of antibiotics, and the minimal inhibitory concentration (MIC) was expressed in terms of µg/ml after incubation at 37°C for 20 hours.

Test compound

(1) 7 - [2 - Methoxyimino - 2 - (2 - aminothiazol - 4 - yl) - acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position).

(2) 7 - [2 - (2 - Cyclopenten - 1 - yl)oxyimino - 2 - (2 - aminothiazol - 4 - yl)acetamido] - 3 - [1 - (2 - carboxy - 2 - hydroxyiminoethyl) - 1H - tetrazol - 5 - yl]thiomethyl - 3 - cephem - 4 - carboxylic acid (syn isomer: in the 7-position).

(3) 7 - [2 - Methoxyimino - 2 - (2 - aminothiazol - 4 - yl) - acetamido] - 3 - [1 - (2 - carboxy - 2 - aminoethyl) - 1H - tetrazol - 5 - yl]thiomethyl - 3 - cephem - 4 - carboxylic acid (syn isomer: in the 7-position).

(4) 7 - [2 - methoxyimino - 2 - (2 - aminothiazol - 4 - yl)acetamido] - 3 - [1 - [3 - (4 - methyl - 1 - piperazinyl)propyl] - 1H - tetrazol - 5 - yl]thiomethyl - 3 - cephem - 4 - carboxylic acid (syn isomer).

(5) 7 - [2 - methoxyimino - 2 - (2 - aminothiazol - 4 - yl)acetamido] - 3 - [1 - [2 - (4 - methyl - 1 - piperazinyl)ethyl] - 1H - tetrazol - 5 - yl]thiomethyl - 3 - cephem - 4 - carboxylic acid (syn isomer).

(6) 7 - [2 - methoxyimino - 2 - (2 - aminothiazol - 4 - yl) - acetamido] - 3 - [1 - (4 - methyl - 1 - piperazinyl)carbonylmethyl - 1H - tetrazol - 5 - yl]thiomethyl - 3 - cephem - 4 - carboxylic acid (syn isomer).

TEST RESULT

| Test Microorganism | M.I.C. (µg/ml) | | | | | |
|---|---|---|---|---|---|---|
| | Compound (1) | Compound (2) | Compound (3) | Compound (4) | Compound (5) | Compound (6) |
| Klebsiella pneumoniae 20 | 0.20 | 0.39 | 0.39 | 0.10 | 0.10 | 0.10 |
| Proteus vulgaris 2 | 0.05 | 0.39 | 0.10 | 0.39 | 0.39 | 0.10 |

The following preparations and examples are given for the purpose of illustrating the present invention.

Preparation 1

A solution of 1-methyl-1H-tetrazole-5-thiol (23 g) in dry tetrahydrofuran (80 ml) was added over 20 minutes with stirring under dry nitrogen atmosphere to a n-butyllithium (250 ml, 15% in hexane) solution in dry tetrahydrofuran (250 ml) precooled to −15 to −10°C. After stirring at the same temperature for 40 minutes, the mixture was cooled to −60° ± 5°C and thereto was added dropwise a solution of diethyl oxalate (28.95 g) in dry tetrahydrofuran (80 ml) over 30 min. The reaction mixture was stirred at −60° ± 5°C for 1.5 hours. 10% Aqueous hydrochloric acid (72 ml) was added with stirring and the separated aqueous layer (pH 7.5) was adjusted to pH 2.5 with 10% hydrochloric acid and extracted with ethyl acetate (200 ml × 3). The combined extracts were dried over magnesium sulfate and evaporated to give oily residue of 1-ethoxalylmethyl-1H-tetrazole-5-thiol (34.3 g).

I.R. (Nujol): 1730 $cm^{-1}$

N.M.R. ($CDCl_3$, δ): 1.43 (3H, t, J=7Hz), 4.46 (2H, q, J=7Hz), 5.67 (2H, s)

Preparation 2

To a solution of 1-ethoxalylmethyl-1H-tetrazole-5-thiol (18.6 g) in methanol (186 ml) was added 1N aqueous solution of sodium hydroxide (172 ml) at room temperature and the mixture was stirred for 40 minutes. After adjusting to pH 7.5 with 10% hydrochloric acid, the solution was evaporated in vacuo to remove methanol and the remained aqueous solution was washed with ethyl acetate (100 ml). The aqueous solution was adjusted to pH 4.0 and washed again with ethyl acetate to remove impurities. The aqueous solution was adjusted to pH 2.5, and extracted with ethyl acetate, dried over magnesium sulfate and evaporated to give pale yellow powder of 1-oxalomethyl-1H-tetrazole-5-thiol (9.7 g), mp 140 to 150°C (dec.).

I.R. (Nujol): 1750, 1710 $cm^{-1}$

N.M.R. (DMSO-$d_6$, δ): 5.71 (2H, s)

Preparation 3

A solution of 1-oxalomethyl-1H-tetrazole-5-thiol (10.0 g) and hydroxylamine hydrochloride (4.8 g) in water (100 ml) was adjusted to pH 6.0 with 2N aqueous solution of sodium hydroxide and stirred at room temperature for 3.5 hours. The solution was acidified with 10% hydrochloric acid to pH 2.0, extracted with a mixture of tetrahydrofuran and ethyl acetate (1:1). The extract was washed with a saturated aqueous solution of sodium chloride, dried over magnesium sulfate and evaporated to give 1-(2-carboxy-2-hydroxyiminoethyl)-1H-tetrazole-5-thiol (5.61 g).

N.M.R. (DMSO-$d_6$, δ): 5.19 (2H, s)

Preparation 4

A solution of 1-oxalomethyl-1H-tetrazole-5-thiol (1.09 g) and methoxyamine hydrochloride (0.47 g) in water (15 ml) was adjusted to pH 6.5 with sodium bicarbonate and stirred at room temperature for 2 hours. After washing with ethyl acetate, the aqueous solution was adjusted to pH 1.8 with 10% hydrochloric acid, extracted with ethyl acetate, dried over magnesium sulfate and evaporated to give 1-(2-carboxy-2-methoxyiminoethyl)-1H-tetrazole-5-thiol (1.05 g), mp 84 to 86°C.

I.R. (Nujol): 1685 $cm^{-1}$

N.M.R. (DMSO-$d_6$, δ): 3.97 (3H, s), 5.17 (2H, s)

Preparation 5

The following compound was prepared according to the similar manners to those of Preparation 1 to 4.

1-(2-Carboxy-2-allyloxyiminoethyl)-1H-tetrazole-5-thiol.

I.R. (Nujol): 1700 $cm^{-1}$

N.M.R. (DMSO-$d_6$, δ): 4.58 (2H, m), 4.98—5.33 (4H, m), 5.58—6.07 (1H, m)

Preparation 6

1-Oxalomethyl-1H-tetrazole-5-thiol (0.94 g) was dissolved in aqueous 60% ethanol (15 ml) containing sodium hydroxide (1.4 g) and to this solution was added portionwise zinc powder (0.98 g). The mixture was stirred at room temperature for 3.5 hours. After removal of zinc powder by filtration, ethanol was removed under reduced pressure and the remained aqueous solution was adjusted to pH 2.0 with 10% hydrochloric acid. Extraction with ethyl acetate, drying over magnesium sulfate and evaporation gave an oil which was triturated with a mixture of diisopropyl ether and n-hexane (1:1). The crystals were filtered and washed with a mixture of diisopropyl ether and n-hexane (1:1) to give 1-(2-carboxy-2-hydroxyethyl)-1H-tetrazole-5-thiol (0.40 g), mp 143 to 146°C,

I.R. (Nujol): 3320, 3180, 1715 $cm^{-1}$

N.M.R. (DMSO-$d_6$, δ): 4.17—4.57 (3H, m)

Preparation 7

A mixture of 1-(2-carboxy-2-hydroxyiminoethyl)-1H-tetrazole-5-thiol (19.3 g), 50% formic acid (255 ml), zinc powder (22.3 g) in ethanol (230 ml) was stirred for 5 hours at room temperature. After the addition of trifluoroacetic acid (100 ml), the mixture was stirred for 5 minutes and filtered. The filtrate was evaporated under reduced pressure. The residue was adjusted to pH 13.0 with 4N aqueous solution of sodium hydroxide and then filtered. The filtrate containing disodium 1-(2-carboxylate-2-aminoethyl)-1H-tetrazole-5-thiolate was adjusted to pH 6.0 and thereto were added dioxane (300 ml), 2-tert-butoxycarbonyloxyimino-2-phenylacetonitrile (35.1 g) and triethylamine (26.2 g). The resulting mixture was stirred overnight at room temperature. The reaction mixture was adjusted to pH 7.0 and washed with ethyl acetate. The washed layer was adjusted to pH 2.0 with phosphoric acid and then extracted with ethyl acetate. The extract was washed with a saturated aqueous solution of sodium chloride, dried over magnesium sulfate and evaporated. The residue was pulverized in diisopropyl ether to give 1-(2-carboxy-2-tert-butoxycarboxamidoethyl)-1H-tetrazole-5-thiol (21.2 g).

I.R. (Nujol): 3400, 1718, 1690 $cm^{-1}$

N.M.R. (DMSO-$d_6$, δ): 1.34 (9H, s), 4.33—4.80 (3H, m), 7.27 (1H, broad s)

Preparation 8

15% solution of n-butyl lithium in hexane (371 ml) was dissolved in dry tetrahydrofuran (150 ml) and cooled to −20°C. To the solution was added dropwise a solution of 1-allyl-1H-tetrazole-5-thiol (38.7 g) in tetrahydrofuran (220 ml) with stirring under a stream of nitrogen gas during a period of 30 minutes. After the addition of solid carbon dioxide, the mixture was stirred for 30 minutes at −10°C. To the mixture was added water (275 ml) and then the resulting mixture was stirred. The aqueous layer was separated adjusted to pH 4.5, washed with ethyl acetate, adjusted to pH 3.8 to 4.0 and extracted with ethyl acetate. The extract was washed with a saturated aqueous solution of sodium chloride, dried over magnesium sulfate and then evaporated. The residue was washed with diisopropyl ether to give an oil of 1-(3-carboxy-1-propenyl)-1H-tetrazole-5-thiol (5.5 g).

I.R. (Nujol): 1700 $cm^{-1}$

N.M.R. (DMSO-$d_6$, δ): 3.57 (2H, m), 6.00 (1H, m), 7.05 (1H, m)

The remaining aqueous layer was adjusted to pH 1.5 and extracted with ethyl acetate. The extract was washed with a saturated aqueous solution of sodium chloride, dried over magnesium sulfate and evaporated to give 1-(1-carboxyallyl)-1H-tetrazole-5-thiol (21.3 g).

I.R. (Film): 1710, 1620 $cm^{-1}$

N.M.R. (DMSO-$d_6$, δ): 5.30—5.70 (2H, m), 5.90—6.67 (2H, m)

Preparation 9

To a solution of 2-(2-formamidothiazol-4-yl)-glyoxylic acid (30 g) and sodium bicarbonate (12.6 g) in water (1300 ml) was added allyloxyamine hydrochloride (19.8 g), and the mixture was stirred for 7 hours at ambient temperature at pH 6. To the reaction mixture was added ethyl acetate (500 ml). After the mixture was adjusted to pH 1.9 with 10% hydrochloric acid, the ethyl acetate layer was separated. The ethyl acetate layer was washed with an aqueous solution of sodium chloride, dried over magnesium sulfate and then the solvent was distilled off. The residue was pulverized in diisopropyl ether, collected by filtration and then dried to give 2-allyloxyimino-2-(2-formamidothiazol-4-yl)acetic acid (syn isomer) (25.3 g).

I.R. (Nujol): 3110, 1730, 1660, 1540 $cm^{-1}$

N.M.R. ($d_6$-DMSO, δ): 4.70 (2H, m), 5.13—5.60 (2H, m), 5.73—6.27 (1H, m), 7.57 (1H, s), 8.35 (1H, s)

Preparation 10

(1) Ethyl 2-(2-propynyloxyimino)-3-oxobutyrate (syn isomer) (71.2 g) was obtained by reacting ethyl 2-hydroxyimino-3-oxobutyrate (syn isomer) (56.7 g) with 2-propynyl bromide (43 g) in the presence of potassium carbonate (72.3 g) and N,N-dimethylformamide (280 ml).

I.R. (Film): 3280, 3220, 2120, 1735, 1670 $cm^{-1}$

(2) Ethyl 2-(2-propynyloxyimino)-3-oxo-4-chlorobutyrate (syn isomer) (61.6 g) was obtained by reacting ethyl 2-(2-propynyloxyimino)-3-oxobutyrate (syn isomer) (71.2 g) with sulfuryl chloride (50.2 g) in acetic acid (81 ml).

I.R. (Film): 3300, 2130, 1745, 1720, 1675 $cm^{-1}$

(3) Ethyl 2-(2-propynyloxyimino)-2-(2-aminothiazol-4-yl)acetate (syn isomer) (35.6 g) was obtained by reacting ethyl 2-(2-propynyloxyimino)-3-oxo-4-chlorobutyrate (syn isomer) (61 g) with thiourea (20 g) in the presence of sodium acetate trihydrate (35.8 g), water (150 ml) and ethanol (180 ml).

I.R. (Nujol): 3290, 2220, 1729 $cm^{-1}$

(4) 2-(2-Propynyloxyimino)-2-(2-aminothiazol-4-yl)acetic acid (syn isomer) (1.924 g) was obtained by hydrolyzing ethyl 2-(2-propynyloxyimino)-2-(2-aminothiazol-4-yl)acetate (syn isomer) (2.8 g) in the

presence of 1N aqueous solution of sodium hydroxide (22.17 ml), methanol (23 ml) and tetrahydrofuran (20 ml).

I.R. (Nujol): 2190, 1740 $cm^{-1}$

(5) 2-(2-Propynyloxyimino)-2-[2-(2,2,2-trifluoroacetamido)-thiazol-4-yl]acetic acid (syn isomer) (11.4 g) was obtained by reacting 2-(2-propynyloxyimino)-2-(2-aminothiazol-4-yl)acetic acid (syn isomer) (10.0 g) with 2,2,2-trifluoroacetic anhydride (22.5 g) in the presence of bis(trimethylsilyl)acetamide (22.3 g) and dry ethyl acetate (100 ml).

I.R. (Nujol): 3280, 3130, 2140, 1710, 1575, 1360, 1260, 1210, 1165, 1075, 1020, 980, 750 $cm^{-1}$

N.M.R. (DMSO-$d_6$, δ): 3.53 (1H, t, J=2Hz), 4.83 (2H, d, J=2Hz), 7.73 (1H, s).

Preparation 11

(1) The following compounds were obtained according to a similar manner to that of Preparation 10(1).

i) Ethyl 2-cyclopentyloxyimino-3-oxobutyrate (syn isomer), oil.

I.R. (Film): 1740, 1670, 1495, 1430 $cm^{-1}$

N.M.R. ($CCl_4$, δ): 1.32 (3H, t, J=7Hz), 1.4—2.2 (8H, m), 2.33 (3H, s), 4.27 (2H, q, J=7Hz) 4.87 (1H, m)

ii) Ethyl 2-benzyloxyimino-3-oxobutyrate (syn isomer).

iii) Ethyl 2-(4-fluorobenzyloxyimino)-3-oxobutyrate (syn isomer).

I.R. $\nu_{max}^{film}$: 3000, 2940, 1730, 1690, 1600 $cm^{-1}$

N.M.R. δ(DMSO-$d_6$, ppm): 1.21 (3H, t, J=7.0Hz), 2.34 (3H, s), 4.26 (2H, q, J=7.0Hz), 5.32 (2H, s), 6.97—7.73 (4H, m)

(2) The following compounds were obtained according to a similar manner to that of Preparation 10(2).

i) Ethyl 2-cyclopentyloxyimino-3-oxo-4-chlorobutyrate (syn isomer), oil.

I.R. (Film): 1750, 1735, 1465, 1435 $cm^{-1}$

N.M.R. ($CCl_4$, δ): 1.33 (3H, t, J=7Hz), 1.3—2.4 (8H, m), 4.28 (2H, q, J=7Hz), 4.46 (2H, s), 4.86 (1H, m)

ii) Ethyl 2-benzyloxyimino-3-oxo-4-chlorobutyrate (syn isomer).

iii) Ethyl 2-(4-fluorobenzyloxyimino)-3-oxo-4-chlorobutyrate (syn isomer).

I.R. $\nu_{max}^{film}$: 1720, 1600 $cm^{-1}$

N.M.R. δ(DMSO-$d_6$, ppm): 1.20 (3H, t, J=7.0Hz), 4.28 (2H, q, J=7.0Hz), 4.87 (2H, s), 5.36 (2H, s), 7.00—7.75 (4H, m)

(3) The following compounds were obtained according to a similar manner to that of Preparation 10(3).

i) Ethyl 2-cyclopentyloxyimino-2-(2-aminothiazol-4-yl)acetate (syn isomer), mp 134 to 136°C.

I.R. (Nujol): 3490, 3450, 3250, 3120, 1735, 1540, 1460 $cm^{-1}$

N.M.R. (DMSO-$d_6$, δ): 1.25 (3H, t, J=7Hz), 1.62 (8H, broad s), 4.27 (2H, q, J=7Hz), 4.70 (1H, m), 6.85 (1H, s), 7.20 (2H, s)

ii) Ethyl 2-benzyloxyimino-2-(2-aminothiazol-4-yl)acetate (syn isomer).

I.R. $\nu_{max}^{Nujol}$: 3440, 3240, 3100, 1730, 1680 $cm^{-1}$

iii) Ethyl 2-(4-fluorobenzyloxyimino)-2-(2-aminothiazol-4-yl)acetate (syn isomer).

I.R. $\nu_{max}^{Nujol}$: 3450, 3150, 3100, 1710, 1620 $cm^{-1}$

N.M.R. δ(DMSO-$d_6$, ppm): 1.23 (3H, t, J=7.0Hz), 4.30 (2H, q, J=7.0Hz), 5.15 (2H, s), 6.90 (1H, s), 6.95—7.60 (4H, m)

(4) The following compounds were obtained according to a similar manner to that of Preparation 10(4).

i) 2-Cyclopentyloxyimino-2-(2-aminothiazol-4-yl)acetic acid (syn isomer), mp 186°C (dec.).

I.R. (Nujol): 3330, 3120, 1635, 1450 $cm^{-1}$

N.M.R. (DMSO-$d_6$, δ): 1.1—2.2 (8H, m), 4.68 (1H, m), 6.81 (1H, s), 7.18 (2H, broad s)

ii) 2-Benzyloxyimino-2-(2-aminothiazol-4-yl)acetic acid (syn isomer).

I.R. $\nu_{max}^{Nujol}$: 3330, 3200, 3100, 1660, 1590 $cm^{-1}$

N.M.R. δ(DMSO-$d_6$, ppm): 5.20 (2H, s), 6.90 (1H, s), 7.40 (5H, s)

iii) 2-(4-Fluorobenzyloxyimino)-2-(2-aminothiazol-4-yl)acetic acid (syn isomer).

I.R. $\nu_{max}^{Nujol}$: 3650, 3450, 3300, 3150, 1630 $cm^{-1}$

N.M.R. δ(DMSO-$d_6$, ppm): 5.16 (2H, s), 6.88 (1H, s), 7.04—7.66 (4H, m)

(5) The following compounds were obtained according to a similar manner to that of Preparation 10(5).

i) 2-Cyclopentyloxyimino-2-[2-(2,2,2-trifluoroacetamido)thiazol-4-yl]acetic acid (syn isomer).

I.R. (Nujol): 3200, 3130, 1720, 1590, 1580 $cm^{-1}$

N.M.R. (DMSO-$d_6$, δ): 1.34—2.22 (8H, m), 4.81 (1H, m), 7.71 (1H, s)

ii) 2-Benzyloxyimino-2-[2-(2,2,2-trifluoroacetamido)thiazol-4-yl]acetic acid (syn isomer).

I.R. (Nujol): 3130, 1730, 1600, 1580 $cm^{-1}$

N.M.R. (DMSO-$d_6$, δ): 5.23 (2H, s), 7.36 (5H, s), 7.67 (1H, s)

iii) 2-(4-Fluorobenzyloxyimino)-2-[2-(2,2,2-trifluoroacetamido)thiazol-4-yl]acetic acid (syn isomer), mp 180—182°C.

I.R. $\nu_{max}^{Nujol}$: 3200, 3150, 1730 $cm^{-1}$
N.M.R. δ(DMSO-$d_6$, ppm): 5.25 (2H, s), 7.02—7.60 (4H, m), 7.72 (1H, s)

Preparation 12

A mixture of 3-chlorocyclopentene (36.9 g), N-hydroxyphthalimide (58.2 g) and triethylamine (53.9 g) in acetonitrile (370 ml) was refluxed for 2 hours. The reaction mixture was poured into ice-water. The precipitated crystals were collected by filtration, washed with water and dried over phosphorus pentoxide to give N-(2-cyclopenten-1-yl)oxyphthalimide (56.5 g).

I.R. (Nujol): 1780, 1730, 1610 $cm^{-1}$
N.M.R. (DMSO-$d_6$, δ): 1.98—2.9 (4H, m), 5.42 (1H, s), 6.00 (1H, m), 6.28 (1H, m), 7.92 (4H, s)

Preparation 13

A mixture of N-phenoxyphthalimide (5.5 g) and hydrazine hydrate (1.15 g) in ethanol (120 ml) was stirred for 1.5 hours at 75 to 80°C. After the addition of conc. hydrochloric acid (4 ml) and water (40 ml) to the reaction mixture under ice-cooling, the resulting mixture was filtered and the filtrate was evaporated. The remaining mixture containing phenoxyamine hydrochloride was adjusted to pH 7.0 with 10% aqueous solution of sodium hydroxide and thereto were added 2-(2-formamidothiazol-4-yl)glyoxylic acid (3.54 g).

The resulting mixture was adjusted to pH 5.0 to 5.5 with 10% hydrochloric acid and stirred for 2 hours at room temperature. The reaction mixture was evaporated under reduced pressure and to the residue was added ethyl acetate. The resulting mixture was adjusted to pH 7.0 with a saturated aqueous solution of sodium bicarbonate. The aqueous layer was separated, adjusted to pH 2.0 with 10% hydrochloric acid and extracted with ethyl acetate. The extract was washed with a saturated aqueous solution of sodium chloride. The solution was dried over magnesium sulfate and concentrated under reduced pressure. The residue was pulverized in diethyl ether to give 2-phenoxyimino-2-(2-formamidothiazol-4-yl)acetic acid (syn isomer).

I.R. (Nujol): 3200, 1700, 1690, 1590, 1560 $cm^{-1}$
N.M.R. (DMSO-$d_6$, δ): 12.60 (1H, broad s), 8.58 (1H, s), 7.85 (1H, s), 7.73—6.67 (5H, m)

Preparation 14

The following compound was obtained according to a similar manner to that of Preparation 13. 2-(2-Cyclopenten-1-yl)oxyimino-2-(2-formamidothiazol-4-yl)acetic acid (syn isomer).

I.R. (Nujol): 3200, 1740, 1710, 1600, 1560 $cm^{-1}$
N.M.R. (DMSO-$d_6$, δ): 1.52—2.67 (4H, m), 5.45 (1H, m), 6.00 (1H, m), 6.28 (1H, m), 7.67 (1H, s), 8.73 (1H, s), 13.0 (1H, broad s)

Preparation 15

(1) To the solution of N-(3-bromopropyl) phthalimide (402 g) in acetone (2.5 l) were added 1-methylpiperazine (225 g) and potassium carbonate (415 g). The resulting mixture was refluxed with stirring for 3.5 hours. The reaction mixture was cooled and then filtered. The filter cake was washed with acetone (500 ml). The filtrate and washing were combined and evaporated under reduced pressure. The remaining starting materials in the residual oil were azeotropically removed with benzene to give an oil of N-[3-(4-methyl-1-piperazinyl)propyl]phthalamide (502 g). I.R. (Film): 1770, 1700 $cm^{-}$

(2) To a solution of N-[3-(4-methyl-1-piperazinyl)propyl]phthalimide (502 g) in ethanol (3.0 l) was added 100% hydrazine hydrate (187.6 g). The resulting mixture was refluxed with stiving for 1.5 hours. The reaction mixture was cooled and then filtered. The filter cake was washed with ethanol (1l). The filtrate and washing were combined and evaporated, under reduced pressure. The residual oil was distilled under reduced pressure to give 3-(4-methyl-1-piperazinyl) propylamine (146.6 g), bp 34 mmHg/127 to 128°C.

(3) To a solution of potassium hydroxide (57.3 g) in methanol (250 ml) was added 3-(4-methyl-1-piperazinyl) propylamine (146 g) and thereto was added carbon disulfide (70.6 g) with stirring under ice cooling over a period of 40 minutes. The resulting mixture was stirred for 3.5 hours under ice cooling. The reaction mixture was evaporated under reduced pressure. The residual oil was dissolved in water (400 ml) and washed with diethyl ether twice. The washed aqueous layer was ice-cooled and thereto was added methyl iodide ((132.1 g) with stirring. The resulting mixture was stirred for 2 hours under ice-cooling, and extracted with ethyl acetate (400 ml × 3) and chloroform (400 ml × 2). The extracts were combined, dried over magnesium sulfate and then evaporated under reduced pressure to give methyl N-[3-(4-methyl-1-peperazinyl)propyl]dithiocarbamate (139.3 g).

Thus obtained product was used directly in the next step reaction without further purification.

(4) To a solution of methyl N-[3-(4-methyl-1-piperazinyl) propyl dithiocarbamate obtained in Preparation 1 (3) (139.3 g) in a mixture of water (420 ml) and ethanol (280 ml) was added sodium azide (47.6 g). The resulting mixture was refluxed with stirring for 3 hours. The reaction mixture was concentrated under reduced pressure. The concentrate was washed successively with ethyl acetate and diethyl ether and then evaporated. To the residue was added ethanol and the mixture was filtered. The filtrate was evaporated under reduced pressure, and to the residual oil was added 6N hydrochloric acid. The mixture was evaporated under reduced pressure. The residue was recrystallized from isopropyl alcohol containing water to give 1-[3-(4-methyl-1-piperazinyl)propyl]-1H-tetrazole-5-thiol dihydrochloride (48.1 g), mp 239 to 243°C.

N.M.R. ($D_2O$, δ): 2.3—2.8 (2H, m), 3.07 (3H, s) 3.3—3.7 (2H, m), 3.75 (8H, s), 4.42 (2H, t, J = 6Hz).

Preparation 16

(1) To the solution of N-(3-bromopropyl)phthalimide (20.1 g) in acetone (225 ml) were added). 1-benzyl piperazine (19.8 g) and potassium carbonate (31.1 g). The resulting mixture was refluxed with stirring for 3 hours. The reaction mixture was cooled and then filtered. The filter cake was washed with acetone. The filtrate and washing were combined and evaporated under reduced pressure to give the oily crude product of the object compound (36.2 g). This oily product was subjected to column chromatography on silica gel. The fractions containing the object compound were collected to give an oil of N-[3-(4-benzyl-1-piperazinyl)propyl]phthalimide (22.4 g).

(2) To a solution of N-[3-(4-benzyl-1-piperazinyl)propyl] phthalimide (22.4 g) in ethanol (250 m) was added 100% hydrazine hydrate (8.02 g). The resulting mixture was refluxed with stirring for 2.5 hours. The reaction mixture was cooled and then filtered. The filter cake was washed with ethanol. The filtrate and washing were combined and evaporated under reduced pressure. To the residue was added 5% aqueous solution of potassium hydroxide and the mixture was extracted with chloroform. The extract was washed with water, dried over magnesium sulfate and then evaporated under reduced pressure to give an oil of 3-(4-benzyl-1-piperazinyl)propylamine (12.4 g).

N.M.R. ($CDCl_3$, S): 1.4 (2H, s), 1.6 (2H, tt, J = 7Hz), 2.4 (2H, t, J = 7Hz), 2.47 (8H, s), 2.73 (2H, t, J = 7Hz), 3.5 (2H, s), 7.27 (5H, s)

(3) To a solution of potassium hydroxide (1.3 g) in methanol (9 ml) was added 3-(4-benzyl-1-piperazinyl)-propylamine (4.9 g) and thereto was added carbon disulfide (1.8 g) under ice cooling over a period of 10 minutes. The resulting mixture was stirred for 4 hours under ice cooling. The reaction mixture was evaporated under reduced pressure. The residual oil was dissolved in water (20 ml) and washed with diethyl ether. The washed aqueous layer was ice-cooled and thereto was added methyl iodide (3.0 g) with stirring under ice-cooling over a period of 10 minutes. The resulting mixture was stirred for 4 hours under ice-cooling. The reaction mixture was extracted with ethyl acetate, dried over magnesium sulfate and then evaporated under reduced pressure to give an oil of methyl N-[3-(4-benzyl-1-piperazinyl)propyl]dithiocarbamate (4.6 g).

(4) To a solution of methyl N-[3-(4-benzyl-1-piperazinyl)propyl]dithiocarbamate (4.6 g) in a mixture of water (12 ml) and ethanol (8 ml) was added sodium azide (1.2 g). The resulting mixture was refluxed with stirring for 3 hours. The reaction mixture was concentrated under reduced pressure. The concentrate was washed successively with ethyl acetate and diethyl ether and then evaporated. To the residue was added ethanol and the mixture was filtered. The filtrate was evaporated under reduced pressure. To the residue was added 6N hydrochloric acid. The mixture was evaporated under reduced pressure and the residue was recrystallized from isopropyl alcohol containing water to give 1-[3-(4-benzyl-1-piperazinyl)propyl]-1H-tetrazole-5-thiol dihydrochloride (2.5 g), mp 234 to 237°C.

N.M.R. (DMSO-$d_6$, δ): 2.1—3.0 (2H, m), 3.1—4.1 (10H, m), 4.42 (2H, s), 4.4 (2H, t), 7.3-8.0 (5H, m).

Preparations 17

(1) To the solution of N-(3-bromopropyl)phthalimide (38.8 g) in acetone (400 ml) were added. 1-ally-piperazine (18.5 g) and potassium carbonate (60.8 g). The resulting mixture was refluxed with stirring for 5 hours. The reaction mixture was cooled and then filtered. The filter cake was washed with acetone. The filtrate and washing were combined and evaporated under reduced pressure. The residual oil was subjected to column chromatography on silica gel. The fractions containing the object compound were collected and then evaporated under reduced pressure to give an oil of N-[3-(4-allyl-1-piperazinyl)propyl]phthalimide (35.6 g).

(2) To a solution of N-[3-(4-allyl-1-piperazinyl)propyl] phthalimide (35.6 g) in ethanol (400 ml) was added 100% hydrazine hydrate (14.3 g). The resulting mixture was refluxed with stirring for 2.5 hours. The reaction mixture was cooled and then filtered. The filter cake was washed with ethanol. The filtrate and washing were combined and evaporated under reduced pressure. The residual oil was subjected to column chromatography (aluminum oxide), eluting with a mixture of chloroform and methanol (10:1). The fractions containing the object compound were collected and then evaporated under reduced pressure to give an oil of 3-(4-allyl-1-piperazinyl)propylamine (18.8 g).

N.M.P. ($ccl_4$, δ): 1.43 (2H, s), 1.52 (2H, tt, J = 7Hz), 2.4 (8H, broad s), 2.33 (2H, t, J = 7Hz), 2.67 (2H, t, J = 7Hz), 2.92 (2H, d, J = 6Hz), 5.0—6.0 (3H, m).

(3) To a solution of potassium hydroxide (6.3 g) in methanol (40 m) was added 3-(4-allyl-1-piperazinyl)propylamine (18.8 g) and thereto was added carbon disulfide (7.8 g) under ice cooling over a period of 10 minutes. The resulting mixture was stirred for 2 hours under ice cooling. The reaction mixture was evaporated under reduced pressure. The residual oil was dissolved in water and washed with diethyl ether. The washed aqueous layer was ice-cooled and thereto was added methyl iodide (14.6 g) with stirring. The resulting mixture was stirred for two hours under ice-cooling. The reaction mixture was extracted with ethyl acetate, the extract was washed with a saturated aqueous solution of sodium chloride, dried over magnesium sulfate and then evaporated under reduced pressure to give an oil of methyl N-[3-(4-allyl-1-piperazinyl)propyl]dithiocarbamate (17.7 g).

(4) To a solution of methyl N-[3-(4-allyl-1-piperazinyl)propyl]dithiocarbamate (17.7 g) in a mixture of

water (60 ml) and ethanol (40 ml) was added sodium azide (5.5 g). The resulting mixture was refluxed with stirring for 3 hours. The reaction mixture was concentrated under reduced pressure. The concentrate was washed successively with ethyl acetate and diethyl ether and then evaporated. To the residue was added ethanol and the mixture was filtered. The filtrate was evaporated under reduced pressure. To the residue was added 6N hydrochloric acid. The mixture was evaporated under reduced pressure. The residue was recrystallized from methanol containing water to give 1-[3-(4-allyl-1-piperazinyl)propyl]-1H-tetrazole-5-thiol dihydrochloride (2.5 g), mp 211 to 215°C (dec.)

N.M.R. ($D_2O$, δ): 2.2—2.8 (2H, m), 3.78 (8H, s), 3.67 (2H, t, J = 6Hz), 3.97 (2H, d, J = 7Hz), 4.50 (2H, t, J = 7Hz), 5.5—6.5 (3H, m).

Preparation 18

(1) To the solution of N-(3-bromopropyl) phthalimide (40.5 g) in acetone 375 ml) were added 1-(2-hydroxyethyl) piperazine (31.5 g) and potassium carbonate (63.0 g). The resulting mixture was refluxed with stirring for 3 hours. The reaction mixture was filtered and the filtrate was evaporated. The residual oil was subjected to column chromatography on silica gel, eluting with a mixture of chloroform and methanol (9:1). The fractions containing the object compound were collected and then evaporated to give an oil of N-[3-[4-(2-hydroxyethyl)-1-piperazinyl]propyl]phthalimide (25.5 g).

(2) To a solution of N - [3 - [4 - (2 - hydroxyethyl - 1 - piperazinyl]propyl]phthalimide (14.4 g) in ethanol (145 ml) was added 100% hydrazine hydrate (6.6 g). The resulting mixture was refluxed with stirring for 2 hours. The reaction mixture was cooled and then filtered. The filter cake was washed with ethanol. The filtrate and washing were combined and evaporated under reduced pressure. The residual oil was subjected to column chromatography (Aluminum oxide), eluting with a mixture of chloroform and methanol (20:1). The fractions containing the object compound were collected and then evaporated to give an oil of 3 - [4 - (2 - hydroxyethyl) - 1 - piperazinyl)]propylamine (5.0 g).

N.M.R. (DMSO-$d_6$, S): 1.48 (2H, tt, J = 7Hz), 2.28 (2H, t, J = 7Hz), 2.53 (2H, t, J = 7Hz), 2.1—2.7 (10H, m), 3.03 (3H, s), 3.16 (2H, t, J = 7Hz).

(3) To a solution of potassium hydroxide (1.5 g) in methanol (11 ml) was added 3-[4-(2-hydroxyethyl)-1-piperazinyl]propylamine (5.0 g) and thereto was added carbon disulfide (2.0 g) under ice cooling over a period of 10 minutes. The resulting mixture was stirred for 2 hours under ice cooling. The reaction mixture was evaporated under reduced pressure. The residual oil was dissolved in water (10 ml), washed with diethyl ether. The washed aqueous layer was ice-cooled and thereto was added methyl iodide (3.8 g) with stirring. The resulting mixture was stirred for 2 hours under ice-cooling, extracted with ethyl acetate, dried over magnesium sulfate and then evaporated to give methyl N - [3 - [4 - (2 - hydroxy - ethyl) - 1 - piperazinyl]propyl]dithio-carbamate (4.8 g).

(4) To a solution of methyl N - [3 - [4 - (2 - hydroxy-ethyl) - 1 - piperazinyl] - propyl] - dithiocarbamate (4.8 g) in a mixture of water (15 ml) and ethanol (10 ml) was added sodium azide (1.5 g). The resulting mixture was refluxed with stirring for 4 hours. The reaction mixture was concentrated under reduced pressure. The concentrate was dissolved in water and the solution was washed successively with ethyl acetate and diethyl ether and then evaporated. To the residue was added ethanol and the mixture was filtered. The filtrate was evaporated under reduced pressure. The residue was recrystallized from isopropyl alcohol to give 1 - [3 - [4 - (2 - hydroxy - ethyl) - 1 - piperazinyl]propyl] - 1H - tetrazole - 5 - thiol (1.35 g).

N.M.R. ($D_2O$, δ): 2.0—3.0 (14H, m), 3.79 (2H, t, J = 7Hz), 4.40 (2H, t, J = 7Hz).

Preparation 19

(1) To the solution of N-(3-bromopropyl)phthalimide (53.0 g) in acetone (400 ml) were added.

1-ethoxycarbonyl piperazine (50.0 g) and potassium carbonate (82.0 g). The resulting mixture was refluxed with stirring for 15 hours. The reaction mixture was cooled and then filtered. The filter cake was washed with acetone. The filtrate and washing were combined and evaporated under reduced pressure. The residue was recrystallized from a mixture of benzene and n-hexane to give N - [3 - (4 - ethoxycarbonyl - 1 - piperazinyl)propyl] - phthalimide (65.2 g), mp 77 to 84°C.

(2) To a solution of N - [3 - (4 - ethoxycarbonyl - 1 - piperazinyl)propyl]phthalimide (28.9 g) in ethanol (300 ml) was added 100% hydrazine hydrate (8.4 g). The resulting mixture was refluxed with stirring for 4 hours. The reaction mixture was cooled and then filtered. The filter cake was washed with ethanol. The filtrate and washing were combined and evaporated under reduced pressure. After the addition of an aqueous solution of sodium hydroxide to the residue, the mixture was extracted with chloroform. The extract was washed with water, dried over magnesium sulfate and then evaporated under reduced pressure to give an oil of 3 - (4 - ethoxycarbonyl - 1 - piperazinyl)propylamine (10.9 g).

N.M.R. ($CDCl_3$, δ): 1.27 (3H, t, J = 7Hz), 1.63 (2H, tt, J = 7Hz), 2.3—2.6 (6H, m), 2.80 (2H, t, J = 7Hz), 3.4—3.7 (4H, m), 4.17 (2H, q, J = 7Hz).

(3) To a solution of potassium hydroxide (2.9 g) in methanol (40 m) was added 3-(4-ethoxycarbonyl-1-piperazinyl)propylamine (10.9 g) and thereto was added carbon disulfide (3.9 g) under ice cooling over a period of 5 minutes. The resulting mixture was stirred for 4 hours under ice cooling. The reaction mixture was evaporated under reduced pressure. The residual oil was dissolved in water (40 ml) and washed with diethyl ether. The washed aqueous layer was ice-cooled and thereto was added methyl iodide (7.2 g) with stirring. The resulting mixture was stirred for 2 hours under ice-cooling, and extracted with ethyl acetate.

The extract was dried over magnesium sulfate and then evaporated under reduced pressure to give an oil of methyl N - [3 - (4 - ethoxy - carbonyl - 1 - piperazinyl)propyl]dithiocarbamate (11.7 g).

(4) To a solution of methyl N - [3 - (4 - ethoxy - carbonyl - 1 - piperazinyl)propyl]dithiocarbamate (11.7 g) in a mixture of water (40 ml) and ethanol (30 ml) was added sodium azide (3.3 g). The resulting mixture was refluxed with stirring for 4 hours. The reaction mixture was concentrated under reduced pressure. After the addition of water to the residue, the mixture was washed with diethyl ether and then evaporated. To the residue was added ethanol and the mixture was filtered. The filtrate was evaporated under reduced pressure. After the addition of a mixture of hydrochloric acid and ethanol to the residue, the resulting mixture was evaporated under reduced pressure. The residue was triturated with acetone to give crude crystals of the object compound (5.7 g), which were recrystallized from ethanol to give 1 - [3 - (4 - ethoxycarbonyl - 1 - piperazinyl)propyl] - 1H - tetrazole - 5 - thiol hydrochloride (2.1 g), mp 192 to 197°C (dec.).

N.M.R. (DMSO-$d_6$, δ): 1.22 (3H, t, J = 7Hz), 2.1—2.5 (2H, m), 3.0—3.5 (6H, m), 3.5—3.9 (4H, m), 4.13 (2H, g, J = 7Hz), 4.37 (2H, t, J = 7Hz).

Preparation 20

(1) To the solution of N-(3-bromopropyl)phthalimide (30.1 g) in acetone (200 ml) were added 1-isopropyl piperazine (23.0 g) and potassium carbonate (41.4 g). The resulting mixture was refluxed with stirring for 4 hours. The reaction mixture was cooled and then filtered. The filter cake was washed with acetone. The filtrate and washing were combined and evaporated under reduced pressure to give an oil of N - [3 - (4 - isopropyl - 1 - piperazinyl)propyl]phthalimide (43.5 g).

(2) To a solution of N - [3 - (4 - isopropyl - 1 - piperazinyl)propyl]phthalimide (43.1 g) in ethanol (350 ml) was added 100% hydrazine hydrate (11.2 g). The resulting mixture was refluxed with stirring for 3 hours. The reaction mixture was cooled and then filtered. The filter cake was washed with ethanol. The filtrate and washing were combined and concentrated under reduced pressure. The residual oil was subjected to column chromatography (aluminum oxide), eluting with a mixture of chloroform and methanol (20:1). The fractions containing the object compound were collected and then evaporated under reduced pressure. The residual oil was distilled under reduced pressure to give 3-(4-isopropyl-1-piperazinyl)propylamine (15.0 g), (bp 45 mmHg/150°C).

(3) To a solution of potassium hydroxide (4.9 g) in methanol (40 ml) was added 3-(4-isopropyl-1-piperazinyl)propylamine (14.7 g) and thereto was added carbon disulfide (6.0 g) under ice cooling over a period of 20 minutes. The resulting mixture was stirred for 3.5 hours under ice cooling. The reaction mixture was evaporated under reduced pressure. The residual oil was dissolved in water (40 ml) and washed with diethyl ether. The washed aqueous layer was ice-cooled and thereto was added methyl iodide (11.3 g) with stirring over a period of about 5 minutes. The resulting mixture was stirred for 2 hours under ice-cooling extracted with cloroform. The extract was washed with water, dried over magnesium sulfate and then evaporated to give an oil of methyl N - [3 - (4 - isopropyl - 1 - piperazinyl)propyl]dithiocarbamate (15.6 g).

(4) To a solution of methyl N - [3 - (4 - isopropyl - 1 - piperazinyl) - propyl]dithiocarbamate (15.1 g) in a mixture of water (50 ml) and ethanol (40 ml) was added sodium azide (4.6 g). The resulting mixture was refluxed with stirring for 4.5 hours. The reaction mixture was concentrated under reduced pressure. The concentrate was washed successively with diethyl ether and ethyl acetate and then evaporated. To the residue was added ethanol and the mixture was filtered. The filtrate was evaporated under reduced pressure. After the addition of a little amount of mixture of hydrochloric acid and ethanol to the residual oil, the product was recrystallized from methanol to give 1 - [3 - (4 - isopropyl - 1 - piperazinyl)propyl] - 1H - tetrazole - 5 - thiol dihydrochloride (3.6 g).

N.M.R. (DMSO-$d_6$, δ): 1.28 (6H, d, J = 6Hz), 2.1—2.5 (2H, m), 3.1—3.8 (3H, m), 3.58 (8H, s), 4.33 (2H, t, J = 8Hz).

Preparation 21

(1) To the solution of N-(3-bromopropyl)phthalimide (15.6 g) in acetone (150 ml) were added 1-phenylpiperazine (10.4 g) and potassium carbonate (12.0 g). The resulting mixture was refluxed with stirring for 4.5 hours. The reaction mixture was cooled and then filtered. The filter cake was washed with acetone. The filtrate and washing were combined and evaporated under reduced pressure. The residue was recrystallized from ethanol to give an oil of N - [3 - (4 - phenyl - 1 - piperazinyl)propyl]phthalimide (18.3 g), mp 129 to 134°C.

(2) To a solution of N - [3 - (4 - phenyl - 1 - piperazinyl)propyl]phthalimide (18.3 g) in ethanol (300 ml) was added 100% hydrazine hydrate (5.3 g). The resulting mixture was refluxed with stirring for 2 hours. The reaction mixture was evaporated under reduced pressure. After the addition of dil aqueous solution of sodium hydroxide to the residue, the mixture was extracted with chloroform. The extract was washed with water, dried over magnesium sulfate and then evaporated under reduced pressure to give 3-(4-phenyl-1-piperazinyl)propylamine (11.2 g).

N.M.R. ($CCl_4$ δ): 1.47 (2H, broad s), 1.57 (2H, tt, J = 7Hz), 2.38 (2H, t, J = 7Hz), 2.72 (2H, t, J = 7Hz), 2.4—2.7 (4H, m), 3.0—3.2 (4H, m), 6.6—7.4 (5H, m).

(3) To a solution of potassium hydroxide (2.85 g) in methanol (25 ml) was added 3-(4-phenyl-1-piperazinyl)propylamine (11.1 g) and thereto was added carbon disulfide (3.85 g) under ice cooling over a period of 10 minutes. The resulting mixture was stirred for 3 hours under ice cooling. The reaction mixture was evaporated under reduced pressure. The residual oil was dissolved in water (40 ml) and washed with diethyl ether. The washed aqueous layer was ice-cooled and thereto was added methyl iodide (7.2 g) with stirring. The resulting mixture was stirred for 2 hours under ice-cooling and extracted with ethyl acetate. The extract was washed with water, dried over magnesium sulfate and then evaporated to give methyl N - [3 - (4 - phenyl - 1 - piperazinyl)propyl]dithiocarbamate (12.9 g), mp 75 to 79°C.

(4) To a solution of methyl N - [3 - (4 - phenyl - 1 - piperazinyl)propyl]dithiocarbamate (12.9 g) in a mixture of water (20 m) and ethanol (30 m) was added sodium azide (3.6 g). The resulting mixture was refluxed with stirring for 4.5 hours. The reaction mixture was concentrated under reduced pressure. After the addition of water to the residue, the mixture was washed successively with ethyl acetate and diethyl ether and then evaporated. To the residue was added ethanol (100 m) and the mixture was filtered. The filtrate was evaporated under reduced pressure. The residue was recrystallized from isopropyl alcohol twice to give 1 - [3 - (4 - phenyl - 1 - piperazinyl)propyl] - 4,5 - dihydro - 1H - tetrazole - 5 - thione (3.2 g), mp. 200 to 205°C.

I.R. (Nujol): 3270, 1600 $cm^{-1}$.

N.M.P. (DMSO-$d_6$, δ): 1.8—2.1 (2H, m), 3.0—3.2 (4H, m), 4.17 (2H, t, J = 6Hz), 6.8—7.5 (5H, m).

A solution of the above obtained product in a little amount of water was acidified with dil hydrochloric acid and then adjusted to pH 7.0 with an aqueous solution of sodium bicarbonate. The precipitates were collected by filtration, washed with water and dried to give 1 - [3 - (4 - phenyl - 1 - piperazinyl)propyl] - 1H - tetrazole - 5 - thiol, mp 220 to 233°C (dec.).

I.R. (Nujol): 2350, 1590 $cm^{-1}$.

N.M.R. (DMSO-$d_6$, δ): 2.0—2.3 (2H, m), 4.3 (2H, t, J = 6Hz), 6.8—7.5 (5H, m).

Preparation 22

The following compounds were prepared according to the similar manners to those of Preparation 15—21.

(1) 1 - [2 - (4 - Methyl - 1 - piperazinyl)ethyl] - 1H - tetrazole - 5 - thiol·hydrobromide, mp 216 to 222°C, N.M.P. ($D_2O$, δ): 2.80 (3H, s), 2.80 (4H, m), 3.04 (2H, t, J = 6Hz), 3.28 (4H, m), 4.48 (2H, t, J = 6Hz).

(2) 1 - (4 - Methyl - 1 - piperazinyl)carbonylmethyl) - 1H - tetrazole - 5 - thiol, mp > 250°C.

| Elemental Analysis: | C | H | N |
|---|---|---|---|
| Calcd | 39.65 | 5.82 | 34.69 |
| Found | 39.33 | 5.68 | 34.52 |

Preparation 23

(1) A mixture of 4-(2-hydroxyethyl) thiosemicarbazide (10.3 g), benzyl chloride (10.6 g), water (30 ml) and methanol (60 ml) was stirred for 20 hours at room temperature to give a homogeneous solution containing benzyl N-(2-hydroxyethyl) thiocarbazimidate. The solution was ice-cooled and thereto was little by little added sodium nitrite (5.24 g) with stirring and then dropwise added conc. hydrochloric acid (3.5 ml) over a period of 30 minutes. The resulting mixture was stirred for an hour under ice-cooling. To the reaction mixture were added potassium carbonate (18 g) and water (30 ml). The mixture was stirred and then concentrated under reduced pressure. The concentrate was extracted with chloroform and the extract was washed with water, dried over magnesium sulfate and then evaporated under reduced pressure. The residual oil was subjected to column chromatography on silica gel. The fractions containing the object compound were collected to give an oil of 1 - (2 - hydroxyethyl) - 5 - benzylthio - 1H - tetrazole (12.1 g).

I.R. (Film): 3380, 1595, 1490 $cm^{-1}$.

N.M.R. ($CDCl_3$, δ): 3.1 (1H, m), 4.1 (2H, m), 4.23 (2H, m), 4.50 (2H, s), 7.33 (5H, s).

(2) To a solution of 1 - (2 - hydroxyethyl) - 5 - benzylthio - 1H - tetrazole (7.0 g) in pyridine (50 ml) was added p-toluenesulfonyl chloride (6.3 g) and the mixture was stirred for 3 hours. After the addition of water, the reaction mixture was extracted with ethyl acetate. The extract was washed with water, dried over magnesium sulfate and then concentrated under reduced pressure. To the residual oil containing 1-(2-tosyloxyethyl)-5-benzylthio-1H-tetrazole were added 1-methylpiperazine (12.0 g), potassium carbonate (12.2 g) and N.N-dimethylformamide (55 m) and the mixture was stirred for 5 hours at 50°C. After the addition of water, the reaction mixture was extracted with ethyl acetate. The extract was washed with water, dried over magnesium sulfate and evaporated under reduced pressure. The crude product was subjected to column chromatography on silica gel. The fractions containing the object compound was collected to give an oil of 1 - [2 - (4 - methyl - 1 - piperazinyl)ethyl] - 5 - benzylthio - 1H - tetrazole (6.5 g).

I.R. (Film: 1600, 1498, 1460, 1164 $cm^{-1}$.

N.M.R. ($CDCl_3$, δ): 2.23 (3H, s), 2.1—2.7 (8H, m), 2.7 (2H, t, J = 6.0 Hz), 4.17 (2H, t, J = 6.0 Hz), 4.5 (2H, s), 7.27 (5H, s).

(3) A mixture of 1 - [2 - (4 - methyl - 1 - piperazinyl)ethyl] - 5 - benzylthio - 1H - tetrazole (5.4 g), acetic acid (40 ml) and conc. hydrobromic acid (40 ml) was heated at 70 to 80°C for 8 hours. After the

concentration of the reaction mixture, the concentrate was diluted with water. The mixture was washed with diethyl ether twice and evaporated under reduced pressure. The residue was recrystallized from methanol. A solution of the obtained crystals in a little amount of water was adjusted to pH 6.0 with 1N aqueous solution of potassium hydroxide., washed with ethyl acetate and then evaporated under reduced pressure. To the residue was added hot ethanol and insoluble materials were filtered out. The filtrate was cooled and the precipitated crystals were collected by filtration to give 1 - [2 - (4 - methyl - 1 - piperazinyl)ethyl] - 1H - tetrazole - 5 - thiol hydrobromide (1.1 g), mp 216 to 222°C.

N.M.R. ($D_2O$, δ): 2.80 (3H, s), 280 (4H. m), 3.04 (2H, t, J = 6Hz), 3.28 (4H, m), 4.48 (2H, t, J = 6Hz).

Preparation 24

The following compounds were prepared according to the similar manner to that of Preparation 23.

(1) 1 - [3 - (4 - Methyl - 1 - piperazinyl)propyl] - 1H - tetrazole - 5 - thiol·dihydrochloride, mp 239 to 243°C.

(2) 1 - [3 - (4 - Benzyl - 1 - piperazinyl)propyl] - 1H - tetrazole - 5 - thiol·dihydrochloride, mp 234 to 237°C.

(3) 1 - [3 - (4 - Allyl - 1 - piperazinyl)propyl] - 1H - tetrazole - 5 - thiol·dihydrochloride, mp 211 to 215°C (dec.).

4. 1 - [3 - [4 - (2 - hydroxyethyl) - 1 - piperazinyl]propyl] - 1H - tetrazole - 5 - thiol.

N.M.R. ($D_2O$, δ): 2.0—3.0 (14H, m), 3.79 (2H, t, J = 7Hz), 4.40 (2H, t, J = 7Hz).

(5) 1 - [3 - (4 - Ethoxycarbonyl - 1 - piperazinyl)propyl] - 1H - tetrazole - 5 - thiol, mp 192 to 197°C (dec.).

(6) 1 - [3 - (4 - Isopropyl - 1 - piperazinyl)propyl] - 1H - tetrazole - 5 - thiol·dihydrochloride.

N.M.R. (DMSO-$d_6$): 1.28 (6H, d, J = 6Hz), 2.1—2.5 (2H, m), 3.1—3.8 (3H, m), 3.58 (8H, s), 4.33 (2H, t, J = 8Hz).

(7) 1 - [3 - (4 - Phenyl - 1 - piperazinyl)propyl] - 1H - tetrazole - 5 - thiol, mp 220 to 223°C (dec.).

(8) 1 - (4 - Methyl - 1 - piperazinyl)carbonylmethyl - 1H - tetrazole - 5 - thiol, mp > 250°C.

Preparation 25

To a solution of 1-carboxymethyl-1H-tetrazole-5-thiol (3.2 g) in dry acetone (60 ml) was added triethyl amine (4.2 g). After the addition of a solution of pivaloyl chloride (4.8 g) in dry acetone (10 ml) with stirring at −15°C over a period of 5 minutes, the mixture was stirred for one hour and 15 minutes at the same temperature. A solution of 1-methylpiperazine (4.0 g) in dry acetone (10 ml) was dropwise added over a period of 5 minutes and the resulting mixture was stirred at −15°C for 40 minutes and under ice-cooling for a further 2.5 hours. After the evaporation of the reaction mixture under reduced pressure, water was added to the residue, 4N aqueous solution of sodium hydroxide was added until the crystals were dissolved. The resulting solution was evaporated under reduced pressure and the residue was crystallized from water to give 1 - (4 - methyl - 1 - piperazinyl)carbonylmethyl - 1H - tetrazole - 5 - thiol (2.2 g). mp > 250°C.

| Elemental Analysis: | C | H | N |
|---|---|---|---|
| Calcd. | 39.65 | 5.82 | 34.69 |
| Found. | 39.33 | 5.68 | 34.52 |

Preparation 26

(1) To a solution of benzhydryl 2-carboxy-2-(4-benzyloxyphemyl) acetate (10 g) in methylene chloride (70 ml) precooled at 0°C was added oxalyl chloride (1.54 ml) and the mixture was stirred for 30 minutes at 0°C. The resulting mixture was dropwise added to a mixture of benzhydryl 7α-methoxy-7β-amino-cephalosporanate (8.5 g), pyridine (1.46 ml) and methylene chloride (80 ml) over a period of about 2 minutes keeping the temperature below 4°C and then the mixture was stirred for 1.5 hours at 0°C. The reaction mixture was concentrated and to the concentrate were added water and ethyl acetate. The ethyl acetate layer was separated and thereto was added water. The mixture was adjusted to pH 1 with dil. hydrochloric acid and to the separated ethyl acetate layer was added water. The resulting mixture was adjusted to pH 8 with an aqueous solution of sodium bicarbonate. The ethyl acetate layer was separated, washed with a saturated aqueous solution of sodium chloride dried over magnesium sulfate and then filtered. The filtrate was evaporated to give a dark brown solid (15.7 g). A solution of the solid in methylene chloride was subjected to column chromatography (silica gel: 235 g), eluting with methylene chloride. The fractions containing the object compound were collected and then concentrated. The residual oil was pulverized in diisopropyl ether to give a powder of benzhydryl 7α - methoxy - 7β - [2 - benzhydryloxy-carbonyl - 2 - 4 - (4 - benzyloxyphenyl)acetamido]cephalosporanate (4.0 g).

I.R. (Nujol): 3350, 1780, 1740, 1610, 1510 $cm^{-1}$.

N.M.R. (DMSO-$d_6$, δ): 1.98 (3H, s), 3.30 (3H, s), 3.42 (2H, broad s), 4.6—4.9 (2H, broad s), 5.13 (3H, s), 5.24 (1H, s), 6.8—7.6 (31H, m).

(2) To a solution of benzhydryl 7α - methoxy - 7β - [2 - benzhydryloxycarbonyl - 2 - (4 - benzyloxyphenyl)acetamido]cephalosporanate (1.5 g), anisole (3.6 ml) in methylene chloride (15 ml) was dropwise added a solution of aluminum chloride (2.21 g) in methyl nitrite (13 ml) over a period of 5 minutes and the mixture was stirred for 1 hour and 40 minutes under ice-cooling. After the addition of water and ethyl acetate to the reaction mixture, the ethyl acetate layer was separated and thereto was added water.

The mixture was adjusted to pH7 with an aqueous solution of sodium bicarbonate and then poured into water. The aqueous layer was separated and thereto was added ethyl acetate. The mixture was adjusted to pH1 with dil. hydrochloric acid and the ethyl acetate layer was separated, dried over magnesium sulfate and then filtered. The filtrate was concentrated and the concentrate was pulverized in diisopropyl ether to give 7α - methoxy - 7β - [D,L - 2 - carboxy - 2 - (4 - hydroxyphenyl)acetamido]cephalosporanic acid (0.7 g).

I.R. (KBr): 3250, 3000, 1770, 1720, 1610, 1510, 1440, 1380 $cm^{-1}$.

N.M.R. (DMSO-$d_6$, δ): 2.0 (3H, s), 3.42 (3H, s), 3.3—3.6 (2H, m), 4.70 (1H, s), 4.6—4.9 (2H, m), 5.07 (1H, s), 6.67, 7.13 (4H, ABq, J = 8Hz), 9.4 (1H, broad s).

Example 1

To a suspension of 7-aminocephalosporanic acid (12.1 g) and 1 - (2 - carboxy - 2 - hydroxyiminoethyl) - 1H - tetrazole - 5 - thiol (9.0 g) in dry acetonitril (60 ml) was added boron trifluoride etherate (25.1 g) and the resulting solution was stirred at 50° for 50 minutes. Water (60 ml) was added and the solution was adjusted to pH 3.5 with conc. aqueous ammonia to give precipitates which were filtered, washed successively with water and acetone, and air-dried to give 7 - amino - 3 - [1 - (2 - carboxy - 2 - hydroxyiminoethyl) - 1H - tetrazol - 5 - yl] - thiomethyl - 3 - cephem - 4 - carboxylic acid (13.92 g).

I.R. (Nujol): 3160, 1800, 1620 $cm^{-1}$.

N.M.R. (DMSO-$d_6$, δ): 3.62 (2H, m), 4.30 (2H, q, J = 14.0 Hz), 4.77 (1H, d, J = 5.0Hz), 4.96 (1H, d, J = 5.0Hz), 5.24 (2H, broad s).

Example 2

To a suspension of 7-aminocephalosporanic acid (0.99 g) and 1 - (2 - carboxy - 2 - methoxyiminoethyl) - 1H - tetrazole - 5 - thiol (0.95 g) in dry acetonitrile (10 ml) was added boron trifluoride etherate (1.55 g) and the resulting solution was stirred at 50°C for 2 hours. Water (10 ml) was added to the reaction mixture and the solution was adjusted to pH 3.5 with conc. aqueous ammonia under ice-cooling to give precipitates which were filtered, washed with water and dried to give 7 - amino - 3 - [1 - (2 - carboxy - 2 - methoxyiminoethyl) - 1H - tetrazol - 5 - yl]thiomethyl - 3 - cephem - 4 - carboxylic acid (1.0 g), mp 170 to 180°C (dec.).

I.R. (Nujol): 1800, 1705, 1615 $cm^{-1}$.

N.M.R. (DMSO-$d_6$, δ): 3.63 (2H, ABq, J = 18Hz), 3.96 (3H, s), 4.30 (2H, ABq, J = 14Hz), 4.78 (1H, d, J = 5Hz), 4.97 (1H, d, J = 5Hz), 5.27 (2H, s).

Example 3

To a suspension of 7 - [2 - methoxyimino - 2 - (2 - aminothiazol - 4 - yl)acetamido]cephalosporanic acid (syn isomer) (1.14 g) and 1 - (2 - carboxy - 2 - methoxyiminoethyl) - 1H - tetrazole - 5 - thiol (0.81 g) in water (20 ml) was added sodium bicarbonate and the solution was adjusted to pH 6.5 and then stirred for 5 hours at 60°C. After cooling at 10 to 15°C, the solution was adjusted to pH 2.5 with with 10% hydrochloric acid. The precipitates were collected by filtration, washed with water and then dried to give 7 - [2 - methoxyimino - 2 - (2 - aminothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position).

I.R. (Nujol): 3270, 1770, 1645 $cm^{-1}$.

Example 4

To a solution of 1 - [3 - (4 - methyl - 1 - piperazinyl)propyl] - 1H - tetrazole - 5 - thiol·dihydrochloride (75.32 g) in water (6 l) was added 7-aminocephalosporanic acid (60.0 g) and the mixture was adjusted to pH 5.4 with a saturated aqueous solution of sodium bicarbonate and stirred for 2 hours at 64°C. The reaction mixture was cooled and adjusted to pH 6.84 with a saturated aqueous solution of sodium bicarbonate. The resulting mixture was subjected to column chromatography (CM-Cephadex, H-type: 3 l), eluting with water, 0.5% aqueous solution of sodium chloride and 2% aqueous solution of sodium chloride. The factions containing the object compound were collected. The resulting mixture (about 7 l) was adjusted to pH 4.5 with a saturated aqueous solution of sodium bicarbonate and then subjected to column chromatography (Non-ion adsorption resin, Diaion HP 20 prepared by Mitsubishi Chemical Industries: 3 l) and eluting with water, 2% isopropyl alcohol and 15% isopropyl alcohol. The fractions containing the object compound were collected and then concentrated. The concentrate was lyophilized to give a powder of 7 - amino - 3 - [1 - [3 - (4 - methyl - 1 - piperazinyl)propyl] - 1H - tetrazol - 5 - yl]thiomethyl - 3 - cephem - 4 - carboxylic acid (34.6 g).

I.R. (KBr): 3400, 1760, 1600 $cm^{-1}$.

N.M.R. ($D_2O$, δ); 2.30 (2H, m), 2.5—3.5 (10H, m), 2.90 (3H, s), 3.57, 3.83 (2H, ABq, J = 18Hz), 4.20, 4.40 (2H, Abq, J = 14Hz), 4.52 (2H, t, J = 7Hz), 4.88 (1H, d, J = 5Hz), 5.13 (1H, d, J = 5Hz).

Example 5

A solution of 7 - [2 - methoxyimino - 2 - (2 - aminothizol - 4 - yl)acetamido]cephalosporanic acid (syn isomer) (20.0 g) and 1 - [3 - (4 - methyl - 1 - piperazinyl)propyl] - 1H - tetrazole - 5 - thiol·dihydrochloride (16.6 g) in water (1 l) was adjusted to pH 6.0 with a saturated aqueous solution of sodium bicarbonate. The mixture was stirred for 3 hours and 20 minutes at 63 to 65°C while the pH was maintained at 6.0 to 6.2 with

5% hydrochloric acid. The reaction mixture was ice-cooled and adjusted to pH 7.3 with a saturated aqueous solution of sodium bicarbonate and thereto was added cold water (3 l). The resulting solution was subjected to column chromatography (CM-Cephadex, H-type: 1 l), eluting with water (200 ml), 1% (2 l), 2% (2 l) and 4% (4 l) aqueous solution of sodium chloride. The fractions containing the object compound were collected. The resulting mixture (about 2 l) was adjusted to pH 4.5 with a saturated aqueous solution of sodium bicarbonate and subjected to column chromatography (Non ion adsorption resin, Diaion HP 20 prepared by Mitsubishi Chemical Industries: 500 ml), eluting with water (0.5l), 5% (1 l) and 15% (2.5 l) isopropyl alcohol. The fractions containing the object compound were collected and then concentrated. The concentrate was lyophilized to give 7 - [2 - methoxyimino - 2 - (2 - aminothiazol - 4 - yl)acetamido] - 3 - [1 - [3 - (4 - methyl - 1 - piperazinyl)propyl] - 1H - tetrazol - 5 - yl]thiomethyl - 3 - cephem - 4 - carboxylic acid (syn isomer). (9.2 g).

I.R. (Nujol): 3300, 1760, 1660, 1600, 1530 $cm^{-1}$.

N.M.R. ($DMSO-d_6$, δ): 2.0 (2H, m), 2.3—3.0 (10H, m), 2.55 (3H, s), 3.47, 3.73 (2H, ABq J = 18Hz), 3.84 (3H, s), 4.32 (4H, broad s), 5.05 (1H, d, J = 5Hz), 5.67 (1H, dd, J = 5 and 8Hz), 6.75 (1H, s), 7.22 (2H, broad s), 9.51 (1H, d, J = 8Hz).

Example 6

A mixture of 7 - [2 - methoxyimino - 2 - (2 - aminothiazol - 4 - yl)acetamido]cephalosporanic acid (syn isomer) (1.2 g), 1 - [2 - (4 - methyl - 1 - piperazinyl)ethyl] - 1H - tetrazole - 5 - thiol·hydrobromide (1.2 g) and phosphate buffer (PH6.4: 100 ml) was adjusted to pH 6.7 with a saturated aqueous solution of sodium bicarbonate. The mixture was stirred for 6 hours at 65°C, cooled to room temperature and then adjusted to pH 4.5 with 5% hydrochloric acid. The resulting mixture was subjected to column chromatography (Non-ion adsorption resin, Diaion HP20 prepared by Mitsubishi Chemical Industries: 60 ml), eluting with water, 5%, 10% and 20% isopropyl alcohol. The fractions containing the object compound were collected, and then concentrated. The concentrate was lyophilized to give powder of 7 - [2 - methoxyimino - 2 - (2 - aminothiazol - 4 - yl)acetamido] - 3 - [1 - [2 - (4 - methyl - 1 - piperazinyl)ethyl] - 1H - tetrazol - 5 - yl]thiomethyl - 3 - cephem - 4 - carboxylic acid (syn isomer) (0.70 g).

I.R. (Nujol): 3300, 1765, 1660, 1600, 1530 $cm^{-1}$.

N.M.R. ($DMSO-d_6$, δ): 2.5—3.1 (10H, m), 2.60 (3H, s), 3.63 (2H, broad s), 3.87 (3H, s), 4.47 (4H, broad s), 5.08 (1H, d, J = 5Hz), 5.70 (1H, dd, J = 5 and 8Hz), 6.77 (1H, s), 7.23 (2H, broad s), 9.56 (1H, d, J = 8Hz).

Example 7

A mixture of 7 - [2 - methoxyimino - 2 - (2 - aminothiazol - 4 - yl)acetamido]cephalosporanic acid (syn isomer) (1.5 g), 1 - (4 - methyl - 1 - piperazinyl)carbonylmethyl - 1H - tetrazole - 5 - thiol (1.03 g), phosphate buffer (pH 6.4, 50 ml) and water (50 ml) was adjusted to pH 7.0 with a saturated aqueous solution of sodium bicarbonate and the mixture was stirred for 5 hours and 50 minutes at 60°C. The reaction mixture was cooled and adjusted to pH 4.5 with 5% hydrochloric acid. The resulting mixture was subjected to column chromatography (Non-ion adsorption resin, Diaion HP 20 prepared by Mitsubishi Chemical Industries: 75 ml), eluting with water, 5% and 10% isopropyl alcohol. The fractions containing the object compound were collected and concentrated. The concentrate was lyophilized to give a powder of 7 - [2 - methoxyimino - 2 - (2 - aminothiazol - 4 - yl)acetamido] - 3 - [1 - (4 - methyl - 1 - piperazinyl)carbonylmethyl - 1H - tetrazol - 5 - yl]thiomethyl - 3 - cephem - 4 - carboxylic acid (syn isomer).

I.R. (Nujol): 3300, 1760, 1660, 1600, 1530 $cm^{-1}$.

N.M.R. ($DMSO-d_6$, δ): 2.5 (3H, s), 2.5—3.0 (4H.m), 3.4—3.9 (6H, m), 3.82 (3H, s), 4.27 (2H, broad s), 5.08 (1H, d, J = 5Hz), 5.57 (2H, broad s), 5.65 (1H, dd, J = 5 and 8Hz), 6.75 (1H, s), 7.17 (2H, broad s), 9.55 (1H, d, J = 5Hz).

Example 8

A solution of 7α - methoxy - 7β - [D,L - 2 - carboxy - 2 - (4 - hydroxyphenyl)acetamido]cephalosporanic acid (0.28 g), 1 - [3 - (4 - methyl - 1 - piperazinyl)propyl] - 1H - tetrazole - 5 - thiol·dihydrochloride (1.3 g) in phosphate buffer (pH 6.4: 100 ml) was stirred for 2 hours and 50 minutes at 65°C. The reaction mixture was adjusted to pH 4.5 with 6N hydrochloric acid and then subjected to column chromatography (Non-ion adsorption resin, Diaion HP-20 prepared by Mitsubishi Chemical Industries: 50 ml), eluting with water (50 ml), 3% (100 ml), 4% (100 ml) and 25% (200 ml) isopropyl alccohol. The fractions containing the object compound were collected and concentrated. The concentrate was lyophilized to give a powder of 7α - methoxy - 7β - [D,L - 2 - carboxy - 2 - (4 - hydroxyphenyl)acetamido] - 3 - [1 - [3 - (4 - methyl - 1 - piperazinyl)propyl] - 1H - tetrazol - 5 - yl]thiomethyl - 3 - cephem - 4 - carboxylic acid (0.15 g).

I.R. (Nujol): 3200, 1760, 1670, 1600, 1505 $cm^{-1}$.

N.M.R. ($D_2O—N_aHCO_3$, δ): 2.5—3.2 (12H, m), 2.75 (3H, s), 3.5 (3H, s), 3.6 (2H, broad s), 4.2—4.6 (4H, m), 5.13, 5.16 (1H, each s), 5.35 (1H, s), 6.97, 7.40 (4H, ABq, J = 8Hz).

Example 9

The following compounds were prepared according to the similar manners to those of Examples 1 to 8.

(1) 7 - amino - 3 - [1 - (2 - carboxy - 2 - allyloxyiminoethyl) - 1H - tetrazol - 5 - yl]thiomethyl - 3 - cephem - 4 - carboxylic acid, mp 165 to 170°C (dec.).

I.R. (Nujol): 1790, 1695, 1605 $cm^{-1}$.

N.M.R. (DMSO-$d_6$, δ): 3.60 (2H, ABq, J = 18Hz), 4.30 (2H, ABq, J = 14Hz), 4.67—5.10 (6H, m), 5.29 (2H, s), 5.60—6.23 (1H, m).

(2) 7 - amino - 3 - [1 - (2 - carboxy - 2 - hydroxyethyl) - 1H - tetrazol - 5 - yl]thiomethyl - 3 - cephem - 4 - carboxylic acid.

I.R. (Nujol): 1795, 1725, 1615 $cm^{-1}$.

N.M.R. (DMSO-$d_6$, δ): 3.67 (2H, m), 4.23—4.73 (5H, m), 4.91 (1H, d, J = 5Hz), 5.10 (1H, d, J = 5Hz).

(3) 7 - [2 - Methoxyimino - 2 - (2 - formamidothiazol - 4 - yl) - acetamido] - 3 - [1 - (2 - carboxy - 2 - hydroxyiminoethyl) - 1H - tetrazol - 5 - yl]thiomethyl - 3 - cephem - 4 - carboxylic acid (syn isomer: in the 7-position).

I.R. (Nujol): 1760, 1650 $cm^{-1}$.

(4) 7 - [2 - Cyclopentyloxyimino - 2 - {2 - (2,2,2 - trifluoroacetamido)thiazol - 4 - yl}acetamido] - 3 - [1 - (2 - carboxy - 2 - hydroxyiminoethyl) - 1H - tetrazol - 5 - yl]thiomethyl - 3 - cephem - 4 - carboxylic acid (syn isomer: in the 7-position).

I.R. (Nujol): 3150, 1770, 1710, 1660 $cm^{-1}$.

(5) 7 - [2 - (2 - Cyclopenten - 1 - yl)oxyimino - 2 - (2 - formamidothiazol - 4 - yl)acetamido] - 3 - [1 - (2 - carboxy - 2 - hydroxyiminoethyl) - 1H - tetrazol - 5 - yl] - thiomethyl - 3 - cephem - 4 - carboxylic acid (syn isomer: in the 7-position).

I.R. (Nujol): 3200, 1780, 1660 $cm^{-1}$.

(6) 7 - [2 - (4 - Fluorobenzyl)oxyimino - 2 - {2 - (2,2,2 - trifluoroacetamido)thiazol - 4 - yl}acetamido] - 3 - [1 - (2 - carboxy - 2 - hydroxyiminoethyl) - 1H - tetrazol - 5 - yl]thiomethyl - 3 - cephem - 4 - carboxylic acid (syn isomer: in the 7-position).

I.R. (Nujol): 3300, 1780, 1720, 1670 $cm^{-1}$.

(7) 7 - [2 - Methoxyimino - 2 - {2 - (2,2,2 - trifluoroacetamido) - thiazol - 4 - yl}acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 164 to 170°C (dec.).

I.R. (Nujol): 1780, 1715, 1655 $cm^{-1}$.

(8) 7 - [2 - Isopropoxyimino - 2 - {2 - (2,2,2 - trifluoroacetamido)thiazol - 4 - yl}acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position).

I.R. (Nujol): 1780, 1715 $cm^{-1}$.

(9) 7 - [2 - Allyloxyimino - 2 - (2 - formamidothiazol - 4 - yl) - acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position).

I.R. (Nujol): 1770, 1670 $cm^{-1}$.

(10) 7 - [2 - (2 - Propynyloxyimino) - 2 - {2 - (2,2,2 - trifluoroacetamido)thiazol - 4 - yl}acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position).

I.R. (Nujol): 3225, 2110, 1770, 1715, 1670 $cm^{-1}$.

(11) 7 - [2 - (2 - Cyclopenten - 1 - yl)oxyimino - 2 - (2 - formamidothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 156 to 161°C (dec.).

I.R. (Nujol): 1770, 1665 $cm^{-1}$.

(12) 7 - [2 - Benzyloxyimino - 2 - {2 - (2,2,2 - trifluoroacetamido) - thiazol - 4 - yl}acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 165 to 170°C (dec.).

I.R. (Nujol): 1775, 1710, 1650 $cm^{-1}$.

(13) 7 - [2 - (4 - Fluorobenzyl)oxyimino - 2 - {2 - (2,2,2 - trifluoroacetamido)thiazol - 4 - yl}acetamido] - 3 - [1 - (2 - carboxy - 2 - methoxyiminoethyl) - 1H - tetrazole - 5 - yl]thiomethyl - 3 - cephem - 4 - carboxylic acid (syn isomer: in the 7-position), mp 153°C (dec.).

I.R. (Nujol): 1765, 1710, 1650 $cm^{-1}$.

(14) 7 - [2 - (2 - Cyclopenten - 1 - yl)oxyimino - 2 - (2 - formamidothiazol - 4 - yl)acetamido] - 3 - [1 - (2 - carboxy - 2 - allyloxyiminoethyl) - 1H - tetrazol - 5 - yl]thiomethyl - 3 - cephem - 4 - carboxylic acid (syn isomer: in the 7-position), mp 150 to 160°C (dec.).

I.R. (Nujol): 1775, 1670 $cm^{-1}$.

(15) 7 - [2 - Benzyloxyimino - 2 - {2 - (2,2,2 - trifluoroacetamido) - thiazol - 4 - yl}acetamido] - 3 - [1 - (2 - carboxy - 2 - allyloxyiminoethyl) - 1H - tetrazol - 5 - yl]thiomethyl - 3 - cephem - 4 - carboxylic acid (syn isomer: in the 7-position).

I.R. (Nujol): 1775, 1710, 1650 $cm^{-1}$.

(16) 7 - [2 - (4 - Fluorobenzyl)oxyimino - 2 - {2 - (2,2,2 - trifluoroacetamido)thiazol - 4 - yl}acetamido] - 3 - [1 - (2 - carboxy - 2 - hydroxyethyl) - 1H - tetrazol - 5 - yl] - thiomethyl - 3 - cephem - 4 - carboxylic acid (syn isomer: in the 7-position), mp 90 to 100°C (dec.).

I.R. (Nujol): 1770, 1710, 1650 $cm^{-1}$.

(17) 7 - [2 - Methoxyimino - 2 - (2 - aminothiazol - 4 - yl) - acetamido] - 3 - [1 - (2 - carboxy - 2 -

hydroxyiminoethyl) - 1H - tetrazol - 5 - yl]thiomethyl - 3 - cephem - 4 - carboxylic acid (syn isomer: in the 7-position), mp 169 to 173°C (dec.).

I.R. (Nujol): 3300, 3200, 1770, 1660, 1630 $cm^{-1}$.

(18) 7 - [2 - Cyclopentyloxyimino - 2 - (2 - aminothiazol - 4 - yl) - acetamido] - 3 - [1 - (2 - carboxy - 2 - hydroxyiminoethyl) - 1H - tetrazol - 5 - yl]thiomethyl - 3 - cephem - 4 - carboxylic acid (syn isomer: in the 7-position), mp 168 to 171°C (dec.).

I.R. (Nujol): 3250, 3150, 1760, 1650 $cm^{-1}$.

(19) 7 - [2 - (2 - Cyclopenten - 1 - yl)oxyimino - 2 - (2 - aminothiazol - 4 - yl)acetamido] - 3 - [1 - (2 - carboxy - 2 - hydroxyiminoethyl) - 1H - tetrazol - 5 - yl]thiomethyl - 3 - cephem - 4 - carboxylic acid (syn isomer: in the 7-position), mp 150 to 160°C (dec.).

I.R. (Nujol): 3270, 3160, 1770, 1650 $cm^{-1}$.

(20) 7 - [2 - (4 - Fluorobenzyl)oxyimino - 2 - (2 - aminothiazol - 4 - yl)acetamido] - 3 - [1 - (2 - carboxy - 2 - hydroxyiminoethyl) - 1H - tetrazol - 5 - yl]thiomethyl - 3 - cephem - 4 - carboxylic acid (syn isomer: in the 7-position), mp 160 to 163°C (dec.).

I.R. (Nujol): 3200, 1770, 1640 $cm^{-1}$.

(21) 7 - [2 - Isopropoxyimino - 2 - (2 - aminothiazol - 4 - yl) - acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 140 to 150°C (dec.).

I.R. (Nujol): 3260, 1770, 1660 $cm^{-1}$.

(22) 7 - [2 - Allyloxyimino - 2 - (2 - aminothiazol - 4 - yl) - acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 130 to 140°C (dec.).

I.R. (Nujol: 3250, 1770, 1650 $cm^{-1}$.

(23) 7 - [2 - (2 - Propynyloxyimino) - 2 - (2 - aminothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position).

I.R. (Nujol): 3255, 1775, 1670, 1630 $cm^{-1}$.

(24) 7 - [2 - (2 - Cyclopenten - 1 - yl)oxyimino - 2 - (2 - aminothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 140 to 150°C (dec.).

I.R. (Nujol): 3260, 1770, 1650 $cm^{-1}$.

(25) 7 - [2 - Benzyloxyimino - 2 - (2 - aminothiazol - 4 - yl) - acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 168 to 172°C (dec.).

I.R. (Nujol): 3260, 1770, 1655 $cm^{-1}$.

(26) 7 - [2 - (4 - Fluorobenzyl)oxyimino - 2 - (2 - aminothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 130 to 140°C (dec.).

I.R. (Nujol): 3270, 1770, 1625 $cm^{-1}$.

(27) 7 - [2 - (2 - Cyclopenten - 1 - yl)oxyimino - 2 - (2 - aminothiazol - 4 - yl)acetamido] - 3 - [1 - (2 - carboxy - 2 - allyloxyiminoethyl) - 1H - tetrazol - 5 - yl]thiomethyl - 3 - cephem - 4 - carboxylic acid (syn isomer: in the 7-position), mp 140 to 150°C (dec.).

I.R. (Nujol): 3270, 1770, 1660, 1630 $cm^{-1}$.

(28) 7 - [2 - Benzyloxyimino - 2 - (2 - aminothiazol - 4 - yl) - acetamido] - 3 - [1 - (2 - carboxy - 2 - allyloxyiminoethyl) - 1H - tetrazol - 5 - yl]thiomethyl - 3 - cephem - 4 - carboxylic acid (syn isomer: in the 7-position), mp 180 to 190°C (dec.).

I.R. (Nujol): 3290, 1775, 1705, 1655 $cm^{-1}$.

(29) 7 - [2 - (4 - Fluorobenzyl)oxyimino - 2 - (2 - aminothiazol - 4 - yl)acetamido] - 3 - [1 - (2 - carboxy - 2 - hydroxyethyl) - 1H - tetrazol - 5 - yl]thiomethyl - 3 - cephem - 4 - carboxylic acid (syn isomer: in the 7-position).

I.R. (Nujol): 3280, 1765, 1630 $cm^{-1}$.

(30) Disodium 7 - amino - 3 - [1 - (2 - carboxylate - 2 - tert - butoxycarboxaminoethyl) - 1H - tetrazol - 5 - yl] - thiomethyl - 3 - cephem - 4 - carboxylate.

I.R. (Nujol): 1750, 1685, 1600 $cm^{-1}$.

N.M.R. ($D_2O$, δ): 1.30 (9H, s), 3.70 (2H, ABq, J = 17Hz), 4.05—4.87 (6H, m), 5.04 (1H, d, J = 5Hz).

(31) 7 - [2 - Methoxyimino - 2 - {2 - (2,2,2 - trifluoroacetamido) - thiazol - 4 - yl}acetamido] - 3 - [1 - (2 - carboxy - 2 - hydroxyethyl) - 1H - tetrazol - 5 - yl]thiomethyl - 3 - cephem - 4 - carboxylic acid (syn isomer: in the 7-position), mp > 140°C (dec.).

I.R. (Nujol): 3180, 1765, 1715, 1650 $cm^{-1}$.

(32) 7 - [2 - Methoxyimino - 2 - (2 - aminothiazol - 4 - yl) - acetamido] - 3 - [1 - (2 - carboxy - 2 - hydroxyethyl) - 1H - tetrazol - 5 - yl]thiomethyl - 3 - cephem - 4 - carboxylic acid (syn isomer: in the 7-position), mp 140 to 150°C (dec.).

I.R. (Nujol): 3320, 1770, 1635 $cm^{-1}$.

(33) 7 - [2 - Methoxyimino - 2 - {2 - (2,2,2 - trifluoroacetamido) - thiazol - 4 - yl}acetamido] - 3 - [1 - (2 - carboxy - 2 - tert - butoxycarboxamidoethyl) - 1H - tetrazol - 5 - yl] - thiomethyl - 3 - cephem - 4 - carboxylic

acid (syn isomer in the 7-position), mp 189 to 196°C (dec.).

I.R. (Nujol): 1780, 1695 $cm^{-1}$.

(34) 7 - [2 - Methoxyimino - 2 - {2 - (2,2,2 - trifluoroacetamido) - thiazol - 4 - yl}acetamido] - 3 - [1 - (2 - carboxy - 2 - aminoethyl) - 1H - tetrazol - 5 - yl]thiomethyl - 3 - cephem - 4 - carboxylic acid formate (syn isomer: in the 7-position).

I.R. (Nujol): 3160, 1760, 1650 $cm^{-1}$.

(35) 7 - [2 - Methoxyimino - 2 - (2 - aminothiazol - 4 - yl)acetamido] - 3 - [1 - (2 - carboxy - 2 - aminoethyl) - 1H - tetrazol - 5 - yl] - thiomethyl - 3 - cephem - 4 - carboxylic acid (syn isomer: in the 7-position).

I.R. (Nujol): 1765, 1640 $cm^{-1}$.

(36) 7 - Amino - 3 - [1 - (3 - carboxy - 1 - propenyl) - 1H - tetrazol - 5 - yl]thiomethyl - 3 - cephem - 4 - carboxylic acid.

I.R. (Nujol): 1795 $cm^{-1}$.

N.M.R. (DMSO-$d_6$, δ): 3.35 (2H, m), 3.63 (2H, ABq, J = 18Hz), 4.33 (2H, ABq, J = 14Hz), 4.75 (1H, d, J = 5Hz), 4.94 (1H, d, J = 5Hz), 6.18 (1H, m), 6.96 (1H, m).

(37) 7 - Amino - 3 - [1 - (1 - carboxyallyl) - 1H - tetrazol - 5 - yl] - thiomethyl - 3 - cephem - 4 - carboxylic acid.

I.R. (Nujol): 1795 $cm^{-1}$.

N.M.R. (DMSO-$d_6$, δ): 3.57 (2H, m), 4.32 (2H, m), 4.84 (2H, m), 5.18—5.40 (2H, m), 5.80—6.47 (2H, m).

(38) 7 - [2 - Methoxyimino - 2 - (2 - aminothiazol - 4 - yl) - acetamido] - 3 - [1 - (3 - carboxy - 1 - propenyl) - 1H - tetrazol - 5 - yl]thiomethyl - 3 - cephem - 4 - carboxylic acid (syn isomer: in the 7-position).

I.R. (Nujol): 3300, 1760, 1660 $cm^{-1}$.

(39) 7 - [2 - (2 - Propynyloxyimino) - 2 - (2 - aminothiazol - 4 - yl) - acetamido] - 3 - [1 - (3 - carboxy - 1 - propenyl) - 1H - tetrazol - 5 - yl]thiomethyl - 3 - cephem - 4 - carboxylic acid (syn isomer: in the 7-position).

I.R. (Nujol): 3320, 2150, 1775, 1675 $cm^{-1}$.

(40) 7 - [2 - Methoxyimino - 2 - (2 - aminothiazol - 4 - yl) - acetamido] - 3 - [1 - (1 - carboxyallyl) - 1H - tetrazol - 5 - yl]thiomethyl - 3 - cephem - 4 - carboxylic acid (syn isomer: in the 7-position).

I.R. (Nujol): 3350, 1765, 1660, 1605 $cm^{-1}$.

(41) 7 - [2 - (2 - Cyclopenten - 1 - yl)oxyimino - 2 - (2 - formamidothiazol - 4 - yl)acetamido] - 3 - [1 - (3 - carboxy - 1 - propenyl) - 1H - tetrazol - 5 - yl]thiomethyl - 3 - cephem - 4 - carboxylic acid (syn isomer: in the 7-position).

I.R. (Nujol): 1770, 1660 $cm^{-1}$.

(42) 7 - [2 - (2 - Cyclopenten - 1 - yl)oxyimino - 2 - (2 - aminothiazol - 4 - yl)acetamido] - 3 - [1 - (3 - carboxy - 1 - propenyl) - 1H - tetrazol - 5 - yl]thiomethyl - 3 - cephem - 4 - carboxylic acid (syn isomer: in the 7-position).

I.R. (Nujol): 3280, 1770, 1660 $cm^{-1}$.

(43) 7 - [2 - Phenoxyimino - 2 - (2 - formamidothiazol - 4 - yl) - acetamido] - 3 - [1 - (3 - carboxy - 1 - propenyl) - 1H - tetrazol - 5 - yl]thiomethyl - 3 - cephem - 4 - carboxylic acid (syn isomer: in the 7-position).

N.M.R. (DMSO-$d_6$, δ): 3.33—3.90 (6H, m), 4.10—4.67 (2H, m), 5.23 (1H, d, J =5 Hz), 5.80—6.33 (2H, m), 6.90—7.57 (6H, m), 7.73 (1H, s), 8.60 (1H, s), 9.03 (1H, d, J = 8Hz), 12.86 (1H, broad s).

(44) 7 - [2 - Phenoxyimino - 2 - (2 - aminothiazol - 4 - yl) - acetamido] - 3 - [1 - (3 - carboxy - 1 - propenyl) - 1H - tetrazol - 5 - yl]thiomethyl - 3 - cephem - 4 - carboxylic acid (syn isomer: in the 7-position).

I.R. (Nujol): 3150, 1770, 1650, 1585 $cm^{-1}$.

(45) 7 - [2 - methoxyimino - 2 - (2 - aminothiazol - 4 - yl)acetamido] - 3 - [1 - [3 - (4 - isopropyl - 1 - piperazinyl)propyl] - 1H - tetrazol - 5 - yl]thiomethyl - 3 - cephem - 4 - carboxylic acid (syn isomer), powder.

I.R. (Nujol): 3300, 1765, 1660, 1600, 1530 $cm^{-1}$.

N.M.R. (DMSO-$d_6$): 1.22 (6H, d, J = 6Hz), 2.0 (2H, m), 2.3—3.1 (11H, m), 3.87 (3H, s), 4.37 (4H, broad s), 5.10 (1H, d, J = 5Hz), 5.73 (1H, dd, J = 5 and 8Hz), 6.77 (1H, s), 7.23 (2H, broad s), 9.57 (1H, d, J = 8Hz).

(46) Sodium 7 - [2 - methoxyimino - 2 - (2 - aminothiazol - 4 - yl)acetamido] - 3 - [1 - [3 - (4 - benzyl - 1 - piperazinyl)propyl] - 1H - tetrazol - 5 - yl]thiomethyl - 3 - cephem - 4 - carboxylate (syn isomer), powder.

I.R. (Nujol): 3300, 1760, 1660, 1600 $cm^{-1}$.

N.M.R. (DMSO-$d_6$, δ): 2.0 (2H, m), 2.5—2.8 (10H, broad s), 3.60 (2H, broad s), 3.73 (2H, broad s), 3.86 (3H, s), 4.30 (4H, broad s), 5.10 (1H, d, J=5Hz), 5.74 (1H, dd, J = 5 and 8Hz), 6.77 (1H, s), 7.40 (7H, broad s), 9.56 (1H, d, J = 8Hz).

(47) Sodium 7 - [2 - methoxyimino - 2 - (2 - aminothiazol - 4 - yl)acetamido] - 3 - [1 - [3 - (4 - allyl - 1 - piperazinyl)propyl] - 1H - tetrazol - 5 - yl]thiomethyl - 3 - cephem - 4 - carboxylate (syn isomer), powder.

I.R. (Nujol): 3350, 1770, 1670, 1600, 1530 $cm^{-1}$.

N.M.R. (DMSO-$d_6$, δ): 3.83 (3H, s), 5.00 (1H, d, J = 5Hz), 6.73 (1H, s), 7.20 (2H, broad s), 9.50 (1H, d, J = 8Hz).

(48) Sodium 7 - [2 - methoxyimino - 2 - (2 - aminothiazol - 4 - yl)acetamido] - 3 - [1 - [3 - (4 - phenyl - 1 - piperazinyl)propyl] - 1H - tetrazol - 5 - yl]thiomethyl - 3 - cephem - 4 - carboxylate (syn isomer), powder.

I.R. (Nujol): 3300, 1765, 1665, 1600, 1530 $cm^{-1}$.

N.M.R. (DMSO-$d_6$, δ): 2.1 (2H, m), 2.5—3.4 (10H, m), 3.63 (2H, broad s), 3.80 (3H, s), 4.25 (2H, broad s), 4.33 (2H, broad s), 5.05 (1H, d, J = 5Hz), 5.70 (1H, dd, J = 5 and 8Hz), 6.68 (1H, s), 6.7—7.3 (7H, m), 9.48 (1H, d, J = 8Hz).

(49) 7 - [2 - methoxyimino - 2 - (2 - aminothiazol - 4 - yl)acetamido] - 3 - [1 - [3 - [4 - (2 - hydroxyethyl) - 1 -

piperazinyl]propyl] - 1H - tetrazol - 5 - yl]thiomethyl - 3 - cephem - 4 - carboxylic acid (syn isomer), powder.

I.R. (Nujol): 3350, 3250, 1775, 1670, 1610, 1540 $cm^{-1}$.

N.M.R. (DMSO-$d_6$, δ): 1.9—2.1 (2H, m), 2.3—3.0 (12H, m), 3.62 (4H, broad s), 3.80 (3H, s), 4.30 (4H, broad s), 5.00 (1H, d, J = 5Hz), 5.62 (1H, dd, J = 5 and 8Hz), 6.70 (1H, s), 7.16 (2H, broad s), 9.44 (1H, d, J = 8Hz).

(50) 7 - [2 - methoxyimino - 2 - (2 - aminothiazol - 4 - yl)acetamido] - 3 - [1 - [3 - (4 - ethoxycarbonyl - 1 - piperazinyl)propyl] - 1H - tetrazol - 5 - yl]thiomethyl - 3 - cephem - 4 - carboxylic acid (syn isomer), powder.

I.R. (Nujol): 3300, 1770, 1670, 1600, 1530 $cm^{-1}$.

N.M.R. (DMSO-$d_6$, δ): 1.17 (3H, t, J = 6.0Hz), 2.0 (2H, m), 2.3—2.6 (6H, m), 3.2—3.5 (4H, m), 3.67 (2H, broad s), 3.83 (3H, s), 4.03 (2H, q, J = 6Hz), 4.33 (4H, broad s), 5.13 (1H, d, J = 5Hz), 5.77 (1H, dd, J = 5 and 8Hz), 6.78 (1H, s), 7.15 (2H, broad s), 9.50 (1H, d, J = 8Hz).

(51) 7 - [2 - (2 - cyclopenten - 1 - yl)oxyimino - 2 - (2 - formamidothiazol - 4 - yl)acetamido] - 3 - [1 - [3 - (4 - methyl - 1 - piperazinyl)propyl] - 1H - tetrazol - 5 - yl]thiomethyl - 3 - cephem - 4 - carboxylic acid (syn isomer), powder.

I.R. (Nujol): 3350, 1775, 1670, 1600, 1550 $cm^{-1}$.

(52) 7 - [2 - (2 - cyclopenten - 1 - yl)oxyimino - 2 - (2 - aminothiazol - 4 - yl)acetamido] - 3 - [1 - [3 - (4 - methyl - 1 - piperazinyl)propyl] - 1H - tetrazol - 5 - yl]thiomethyl - 3 - cephem - 4 - carboxylic acid (syn isomer), powder.

I.R. (Nujol): 3300, 1770, 1660, 1620, 1530 $cm^{-1}$.

## Example 10

2 - (2 - Cyclopenten - 1 - yl)oxyimino - 2 - (2 - formamidothiazol - 4 - yl)acetic acid (syn isomer) (1.00 g) was added at 0°C to vilsmeier reagent which had been prepared from N,N-dimethylformamide (0.30 g) and phosphoryl chloride (0.62 g) in ethyl acetate (1.2 ml) and tetrahydrofuran (10 ml), and the mixture was stirred at the same temperature for 30 minutes to afford the activated solution. The activated solution was added dropwise at −5 to 0°C to a solution of 7 - amino - 3 - [1 - (2 - carboxy - 2 - hydroxyiminoethyl) - 1H - tetrazol - 5 - yl]thiomethyl - 3 - cephem - 4 - carboxylic acid (1.30 g) in water (8 ml) and acetone (8 ml), adjusted to pH 8.0 with sodium bicarbonate and the mixture was stirred at the same temperature for 30 minutes. To the resultant mixture was added ethyl acetate (10 ml), and the aqueous phase was separated from the mixture. To the aqueous solution was added ethyl acetate (60 ml) and the mixture was adjusted to pH 2.0 with 10% hydrochloric acid with stirring, and the ethyl acetate layer was separated, washed with water, treated with activated carbon, dried over magnesium sulfate and evaporated. The residue was pulverized with diethyl ether to give 7 - [2 - (2 - cyclopenten - 1 - yl)oxyimino - 2 - (2 - formamidothiazol - 4 - yl)acetamido] - 3 - [1 - (2 - carboxy - 2 - hydroxyiminoethyl - 1H - tetrazol - 5 - yl]thiomethyl - 3 - cephem - 4 - carboxylic acid (syn isomer: in the 7-position) (1.17 g).

I.R. (Nujol): 3200, 1780, 1660 $cm^{-1}$.

N.M.R. (DMSO-$d_6$): 1.75—2.73 (4H, m), 3.73 (2H, m), 4.39 (2H, q, J = 14.0Hz), 5.03—5.53 (4H, m), 5.62—6.36 (3H, m), 7.43 (1H, s), 8.56 (1H, s), 9.63 (1H, d, J=8.0Hz).

## Example 11

2 - Methoxyimino - 2 - [2 - (2,2,2 - trifluoroacetamido)thiazol - 4 - yl]acetic acid (syn isomer) (0.97 g) was added at 0°C to vilsmeier reagent which had been prepared from N,N-dimethylformamide (0.31 g) and phosphoryl chloride (0.65 g) in ethyl acetate (1.2 ml) and tetrahydrofuran (7.4 ml), and the mixture was stirred at the same temperature for 30 minutes to afford the activated solution. The activated solution was added dropwise at −5 to 0°C to a solution of 7 - amino - 3 - [1 - (2 - carboxy - 2 - methoxyiminoethyl) - 1H - tetrazol - 5 - yl]thiomethyl - 3 - cephem - 4 - carboxylic acid (1.40 g) in water (10 ml) and acetone (8 ml), adjusted to pH 8.0 with sodium bicarbonate and the mixture was stirred at the same temperature for 45 minutes. To the resultant mixture was added ethyl acetate (10 ml), and the aqueous phase was separated from the mixture. To the aqueous solution was added ethyl acetate (70 ml) and the mixture was adjusted to pH 2.0 with 10% hydrochloric acid with stirring, and the ethyl acetate layer was separated, washed with a saturated aqueous solution of sodium chloride, dried over magnesium sulfate and evaporated. The residue was pulverized with diisopropyl ether to give 7 - [2 - methoxyimino - 2 - {2 - (2,2,2 - trifluoroacetamido)thiazol - 4 - yl}acetamido] - 3 - [1 - (2 - carboxy - 2 - methoxyiminoethyl) - 1H - tetrazol - 5 - yl] - thiomethyl - 3 - cephem - 4 - carboxylic acid (syn isomer: in the 7-position) (1.77 g), mp 164 to 170°C (dec.).

I.R. (Nujol): 1780, 1715, 1655 $cm^{-1}$.

N.M.R. (DMSO-$d_6$, δ): 3.70 (2H, m), 3.91 (3H, s), 3.97 (3H, s), 4.39 (2H, m), 5.14 (1H, d, J = 4.5Hz), 5.27 (2H, s), 5.81 (1H, dd, J = 4.5 and 8Hz), 7.52 (1H, s), 9.73 (1H, d, J = 8Hz).

## Example 12

2 - (2 - Propynyloxyimino) - 2 - [2 - (2,2,2 - trifluoroacetamido)thiazol - 4 - yl]acetic acid (syn isomer) (1.05 g) was added at 0°C to vilsmeier reagent which had been prepared from N,N-dimethylformamide (0.31 g) and phosphoryl chloride (0.65 g) in ethyl acetate (1.2 ml) and tetrahydrofuran (7.4 ml), and the mixture was stirred at the same temperature for 30 minutes to afford the activated solution. The activated solution was added dropwise at −5 to 0°C to a solution of 7 - amino - 3 - [1 - (2 - carboxy - 2 -

methoxyiminoethyl) - 1H - tetrazol - 5 - yl]thiomethyl - 3 - cephem - 4 - carboxylic acid (1.40 g) in water (10 ml) and acetone (8 ml), adjusted to pH 8.0 with sodium bicarbonate and the mixture was stirred at the same temperature for 35 minutes. To the resultant mixture was added ethyl acetate (10 ml), and the aqueous layer was separated from the mixture. To the aqueous solution was added ethyl acetate (70 ml) and the mixture was adjusted to pH 1.8 with 10% hydrochloric acid with stirring, and the ethyl acetate layer was separated, washed with a saturated aqueous solution of sodium chloride, dried over magnesium sulfate and evaporated. In the residue was pulverized with diisopropyl ether to give 7 - [2 - (2 - propynyloxyimino) - 2 - {2 - (2,2,2 - trifluoroacetamido)thiazol - 4 - yl}acetamido] - 3 - [1 - (2 - carboxy - 2 - methoxyiminoethyl) - 1H - tetrazol - 5 - yl] - thiomethyl - 3 - cephem - 4 - carboxylic acid (syn isomer: in the 7-position) (2.05 g).

I.R. (Nujol): 3225, 2110, 1770, 1715, 1670 $cm^{-1}$.

N.M.R. (DMSO-$d_6$, δ): 3.47 (1H, m), 3.69 (2H, m), 3.97 (3H, s), 4.35 (2H, m), 4.77 (2H, m), 5.14 (1H, d, J = 5Hz), 5.26 (2H, s), 5.80 (1H, dd, J = 5 and 8Hz), 7.55 (1H, s), 9.80 (1H, d, J = 8Hz).

## Example 13

To a solution of N,N-dimethylformamido (277 mg) in tetrahydrofuran (1 ml) was dropwise added a solution of phosphorus oxychloride (583 mg) in tetrahydrofuran (9 ml) at −10°C and the mixture was stirred at room temperature. After the addition of 2 - (2 - cyclopenten - 1 - yl)oxyimino - 2 - (2 - formamidothiazol - 4 - yl) acetic acid (syn isomer) (900 mg) at −10°C, the resulting mixture was stirred for 30 minutes at 0°C and then the temperature was raised to 10°C to give a solution (Solution A). On the other hand, a solution of 7 - amino - 3 - [1 - [3 - (4 - methyl - 1 - piperazinyl)propyl] - 1H - tetrazol - 5 - yl] thiomethyl - 3 - cephem - 4 - carboxylic acid (1.2 g) in a mixture of water (12 ml) and acetone (12 ml) was adjusted to pH 7.0 with a saturated aqueous solution of sodium bicarbonate and then cooled to 0°C. To the solution was dropwise added the above obtained solution A over a period of 5 minutes while the pH was maintained at 7. The resulting solution was allowed to warm to room temperature, stirred for 1.5 hours, adjusted to pH 6.5 with dil, hydrochloric acid and then concentrated under reduced pressure. The precipitates were collected by filtration to give 7 - [2 - (2 - cyclopenten - 1 - yl)oxyimino - 2 - (2 - formamidothiazol - 4 - yl)acetamido] - 3 - [1 - [3 - (4 - methyl - 1 - piperazinyl)propyl] - 1H - tetrazol - 5 - yl]thiomethyl - 3 - cephem - 4 - carboxylic acid (syn isomer) (300 mg). The filtrate was subjected to column chromatography (Non-ion adsorption resin, Diaion HP20 prepared by Mitsubishi Chemical Industries, 60 ml, eluting with water, 5%, 15% and 25% isopropyl alcohol. The fractions containing the object compound were collected and concentrated. The concentrate was lyophilized to give the same object compound (0.85 g), powder.

I.R. (Nujol): 3350, 1775, 1670, 1600, 1550 $cm^{-1}$.

N.M.R. (DMSO-$d_6$, δ): 1.8—3.0 (19H, m), 3.6 (2H, broad s), 4.43 (4H, broad s), 5.07 (1H, d, J = 5Hz), 5.2—5.5 (1H, m), 5.6—6.2 (3H, m), 7.38 (1H, s), 8.53 (1H, s), 9.55 (1H, d, J = 8H).

## Example 14

The following compounds were prepared according to the similar manners to those of examples 10 to 13.

(1) 7 - [2 - Methoxyimino - 2 - (2 - formamidothiazol - 4 - yl) - acetamido] - 3 - [1 - (2 - carboxy - 2 - hydroxyiminoethyl) - 1H - tetrazol - 5 - yl]thiomethyl - 3 - cephem - 4 - carboxylic acid (syn isomer: in the 7-position).

I.R. (Nujol): 1760, 1650 $cm^{-1}$.

N.M.R. (DMSO-$d_6$, δ): 3.72 (2H, m), 3.90 (3H, s), 4.36 (2H, m), 5.04—5.46 (3H, m), 5.83 (1H, dd, J = 4.0 and 8.0Hz), 7.43 (1H, s), 8.55 (1H, s), 9.78 (1H, d, J = 8.0Hz).

(2) 7 - [2 - Cyclopentyloxyimino - 2 - {2 - (2,2,2 - trifluoroacetamido)thiazol - 4 - yl}acetamido] - 3 - [1 - (2 - carboxy - 2 - hydroxyiminoethyl) - 1H - tetrazol - 5 - yl]thiomethyl - 3 - cephem - 4 - carboxylic acid (syn isomer: in the 7-position).

I.R. (Nujol): 3150, 1770, 1710, 1660 $cm^{-1}$.

N.M.R. (DMSO-$d_6$, δ): 1.31—2.01 (8H, m), 3.69 (2H, m), 4.35 (2H, m), 4.72 (1H, m), 5.14 (1H, d, J = 4.0Hz), 5.27 (2H, s), 5.80 (1H, dd, J = 4.0 and 8.0Hz), 7.48 (1H, s), 9.67 (1H, d, J = 8.0Hz).

(3) 7 - [2 - (4 - Fluorobenzyl)oxyimino - 2 - {2 - (2,2,2 - trifluoroacetamido)thiazol - 4 - yl}acetamido] - 3 - [1 - (2 - (2 - carboxy - 2 - hydroxyiminoethyl) - 1H - tetrazol - 5 - yl]thiomethyl - 3 - cephem - 4 - carboxylic acid (syn isomer: in the 7-position).

I.R. (Nujol): 3300, 1780, 1720, 1670 $cm^{-1}$.

N.M.R. (DMSO-$d_6$, δ): 3.70 (2H, m), 4.41 (2H, m), 5.04—5.45 (5H, m), 5.87 (1H, dd, J = 4.0 and 8.0Hz), 6.96—7.72 (4H, m), 7.58 (1H, s), 9.88 (1H, d, J = 8.0Hz).

(4) 7 - [2 - Isopropoxyimino - 2 - {2 - (2,2,2 - trifluoroacetamido)thiazol - 4 - yl}acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position).

I.R. (Nujol): 1780, 1715 $cm^{-1}$.

N.M.R. (DMSO-$d_6$, δ): 1.22 (3H, s), 1.33 (3H, s), 3.72 (2H, m), 3.98 (3H, s), 4.21—4.67 (3H, m), 5.17 (1H, d, J = 5Hz), 5.28 (2H, s), 5.83 (1H, dd, J = 5 and 8Hz), 7.48 (1H, s), 9.68 (1H, d, J = 8Hz).

(5) 7 - [2 - Allyloxyimino - 2 - (2 - formamidothiazol - 4 - yl) - acetamido] - 3 - [1 - (2 - carboxy - 2 - methoxyiminoethyl) - 1H - tetrazol - 5 - yl]thiomethyl- 3 - cephem - 4 - carboxylic acid (syn isomer: in the 7-position).

I.R. (Nujol): 1770, 1670 $cm^{-1}$.

N.M.R. (DMSO-$d_6$, δ): 3.73 (2H, m), 4.02 (3H, s), 4.43 (2H, m), 4.68 (2H, m), 5.10—5.60 (5H, m), 5.77—6.30 (2H, m), 7.46 (1H, s), 8.57 (1H, s), 9.76 (1H, d, J = 8Hz).

(6) 7 - [2 - (2 - Cyclopenten - 1 - yl)oxyimino - 2 - (2 - formamidothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 156 to 161°C (dec.).

I.R. (Nujol): 1770, 1665 $cm^{-1}$.

N.M.R. (DMSO-$d_6$, δ): 1.70—2.60 (4H, m), 3.67 (2H, m), 3.98 (3H, s), 4.37 (2H, m), 5.02—5.46 (4H, m), 5.71—6.20 (3H, m), 7.39 (1H, s), 8.52 (1H, s), 9.60 (1H, d, J = 8Hz).

(7) 7 - [2 - Benzyloxyimino - 2 - {2 - (2,2,2 - trifluoroacetamido)thiazol - 4 - yl}acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 165 to 170°C (dec.).

I.R. (Nujol): 1775, 1710, 1650 $cm^{-1}$.

N.M.R. (DMSO-$d_6$, δ): 3.67 (2H, m), 4.00 (3H, s), 4.37 (2H, m), 5.13—5.50 (5H, m), 5.91 (1H, dd, J = 5 and 8Hz), 7.40 (5H, m), 7.57 (1H, s), 9.88 (1H, d, J = 8Hz).

(8) 7 - [2 - (4 - Fluorobenzyl)oxyimino - 2 - {2 - (2,2,2 - trifluoroacetamido)thiazol - 4 - yl}acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 153°C (dec.).

I.R. (Nujol): 1765, 1710, 1650 $cm^{-1}$.

N.M.R. (DMSO-$d_6$, δ): 3.65 (2H, m), 3.97 (3H, s), 4.37 (2H, m), 5.08—5.40 (5H, m), 5.83 (1H, dd, J = 5 and 8Hz), 7.03—7.63 (5H, m), 9.88 (1H, d, J = 8Hz).

(9) 7 - [2 - (2 - Cyclopenten - 1 - yl)oxyimino - 2 - (2 - formamidothiazol - 4 - yl)acetamido] - 3 - [1 - (2 - carboxy - 2 - allyloxyiminoethyl) - 1H - tetrazol - 5 - yl]thiomethyl - 3 - cephem - 4 - carboxylic acid (syn isomer: in the 7-position), mp 150 to 160°C (dec.).

I.R. (Nujol): 1775, 1670 $cm^{-1}$.

N.M.R. (DMSO-$d_6$, δ): 1.77—2.70 (4H, m), 3.70 (2H, m), 4.40 (2H, ABq, J = 14Hz), 4.77 (2H, m), 5.03—5.50 (6H, m), 5.63—6.30 (4H, m), 7.40 (1H, s), 8.57 (1H, s), 9.62 (1H, d, J = 8Hz).

(10) 7 - [2 - Benzyloxyimino - 2 - {2 - (2,2,2 - trifluoroacetamido)thiazol - 4 - yl}acetamido] - 3 - [1 - (2 - carboxy - 2 - allyloxyiminoethyl) - 1H - tetrazol - 5 - yl]thiomethyl - 3 - cephem - 4 - carboxylic acid (syn isomer: in the 7-position).

I.R. (Nujol): 1775, 1710, 1650 $cm^{-1}$.

N.M.R. (DMSO-$d_6$, δ): 3.70 (2H, m), 4.30 (2H, m), 4.80 (2H, m), 5.07—5.60 (7H, m), 5.68—6.21 (2H, m), 7.42 (5h, m), 7.56 (1H, s), 9.88 (1H, d, J = 8Hz).

(11) 7 - [2 - (4 - Fluorobenzyl)oxyimino - 2 - {2 - (2,2,2 - trifluoroacetamido)thiazol - 4 - yl}acetamido] - 3 - [1 - (2 - carboxy - 2 - hydroxyethyl) - 1H - tetrazol - 5 - yl]thiomethyl - 3 - cephem - 4 - carboxylic acid (syn isomer: in the 7-position), mp 90 to 100°C (dec.).

I.R. (Nujol): 1770, 1710, 1650 $cm^{-1}$.

N.M.R. (DMSO-$d_6$, δ): 3.73 (2H, m), 4.20—4.80 (5H, m), 5.10—5.47 (3H, m), 5.88 (1H, dd, J = 5 and 8Hz), 6.88—7.70 (5H, m), 9.89 (1H, d, J = 8Hz).

(12) 7 - [2 - Methoxyimino - 2 - (2 - aminothiazol - 4 - yl) - (acetamido] - 3 - [1 - (2 - carboxy - 2 - hydroxyiminoethyl) - 1H - tetrazol - 5 - yl]thiomethyl - 3 - cephem - 4 - carboxylic acid (syn isomer: in the 7-position), mp 169 to 173°C (dec).

I.R. (Nujol): 3300, 3200, 1770, 1660, 1630 $cm^{-1}$.

(13) 7 - [2 - Cyclopentyloxyimino - 2 - (2 - aminothiazol - 4 - yl)acetamido] - 3 - [1 - (2 - carboxy - 2 - hydroxyiminoethyl) - 1H - tetrazol - 5 - yl]thiomethyl - 3 - cephem - 4 - carboxylic acid (syn isomer: in the 7-position), mp 168 to 171°C (dec.).

I.R. (Nujol): 3250, 3150, 1760, 1650 $cm^{-1}$.

(14) 7 - [2 - (2 - cyclopenten - 1 - yl)oxyimino - 2 - (2 - aminothiazol - 4 - yl)acetamido] - 3[1 - (2 - carboxy - 2 - hydroxyiminoethyl) - 1H - tetrazol - 5 - yl]thiomethyl - 3 - cephem - 4 - carboxylic acid (syn isomer: in the 7-position), mp 150 to 160°C (dec.).

I.R. (Nujol): 3270, 3160, 1770, 1650 $cm^{-1}$.

(15) 7 - [2 - (4 - Fluorobenzyl)oxyimino - 2 - (2 - aminothiazol - 4 - yl)acetamido] - 3 - [1 - (2 - carboxy - 2 - hydroxyiminoethyl) - 1H - tetrazol - 5 - yl]thiomethyl - 3 - cephem - 4 - carboxylic acid (syn isomer: in the 7-position), mp 160 to 163°C.

I.R. (Nujol): 3200, 1770, 1640 $cm^{-1}$.

(16) 7 - [2 - Methoxyimino - 2 - (2 - aminothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position).

I.R. (Nujol): 3270, 1770, 1645 $cm^{-1}$.

(17) 7 - [2 - Isopropoxyimino - 2 - (2 - aminothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 140 to 150°C (dec.).

I.R. (Nujol): 3260, 1770, 1660 $cm^{-1}$.

(18) 7 - [2 - Allyloxyimino - 2 - (2 - aminothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 130 to 140°C (dec.).

I.R. (Nujol): 3250, 1770, 1650 $cm^{-1}$.

(19) 7 - [2 - (2 - Propynyloxyimino) - 2 - (2 - aminothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position).

I.R. (Nujol): 3255, 1775, 1670, 1630 $cm^{-1}$.

(20) 7 - [2 - (2 - Cyclopenten - 1 - yl)oxyimino - 2 - (2 - aminothiazol - 4 - yl)acetamido] - 3 - [1 - (2 - carboxy-
2 - methoxyiminoethyl) - 1H - tetrazol - 5 - yl]thiomethyl - 3 - cephem - 4 - carboxylic acid (syn isomer: in the 7-position), mp 140 to 150°C (dec.).

I.R. (Nujol): 3260, 1770, 1650 $cm^{-1}$.

(21) 7 - [2 - Benzyloxyimino - 2 - (2 - aminothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 168 to 172°C (dec.).

I.R. (Nujol): 3260, 1770, 1655 $cm^{-1}$.

(22) 7 - [2 - (4 - Fluorobenzyl)oxyimino - 2 - (2 - aminothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 130 to 140°C (dec.).

I.R. (Nujol): 3270, 1770 1625 $cm^{-1}$.

(23) 7 - [2 - (2 - Cyclopenten - 1 - yl)oxyimino - 2 - (2 - aminothiazol - 4 - yl)acetamido] - 3 - [1 - (2 - carboxy - 2 - allyloxyiminoethyl) - 1H - tetrazol - 5 - yl]thiomethyl - 3 - cephem - 4 - carboxylic acid (syn isomer: in the 7-position), mp 140 to 150°C (dec.).

I.R. (Nujol): 3270, 1770, 1660, 1630 $cm^{-1}$.

(24) 7 - [2 - Benzyloxyimino - 2 - (2 - aminothiazol - 4 - yl) - acetamido - 3 - [ - (2 - carboxy - 2 - allyloxyiminoethyl) - 1H - tetrazol - 5 - yl]thiomethyl - 3 - cephem - 4 - carboxylic acid (syn isomer: in the 7-position), mp 180 to 190°C (dec.).

I.R. (Nujol): 3290, 1775, 1705, 1655 $cm^{-1}$.

(25) 7 - [2 - (4 - Fluorobenzyl)oxyimino - 2 - (2 - aminothiazol - 4 - yl)acetamido] - 3 - [1 - (2 - carboxy - 2 - hydroxyethyl) - 1H - tetrazol - 5 - yl]thiomethyl - 3 - cephem - 4 - carboxylic acid (syn isomer: in the 7-position).

I.R. (Nujol): 3280, 1765, 1630 $cm^{-1}$.

(26) 7 - [2 - Methoxyimino - 2 - {2 - (2,2,2 - trifluoroacetamido)thiazol - 4 - yl}acetamido] - 3 - [1 - (2 - carboxy - 2 - hydroxyiminoethyl) - 1H - tetrazol - 5 - yl]thiomethyl - 3 - cephem - 4 - carboxylic acid (syn isomer: in the 7-position), mp >140°C (dec.).

I.R. (Nujol): 3180, 1765, 1715, 1650 $cm^{-1}$.

N.M.R. ($DMSO-d_6$, δ): 3.70 (2H, m), 3.91 (3H, s), 4.20—4.77 (5H, m), 5.13 (1H, d, J = 5Hz), 5.75 (1H, dd, J = 5 and 8Hz), 7.52 (1H, s), 9.75 (1H, d, J = 8Hz).

(27) 7 - [2 - Methoxyimino - 2 - (2 - aminothiazol - 4 - yl)acetamido] - 3 - [1 - (2 - carboxy - 2 - hydroxyiminoethyl) - 1H - tetrazol - 5 - yl]thiomethyl - 3 - cephem - 4 - carboxylic acid (syn isomer: in the 7-position), mp 140 to 150°C (dec.).

I.R. (Nujol): 3320, 1770, 1635 $cm^{-1}$.

(28) 7 - [2 - Methoxyimino - 2 - {2 - (2,2,2 - trifluoroacetamido)thiazol - 4 - yl}acetamido] - 3 - [1 - (2 - carboxy - 2 - tertbutoxycarboxamidoethyl) - 1H - tetrazol - 5 - yl]thiomethyl - 3 - cephem - 4 - carboxylic acid (syn isomer: in the 7-position), mp 189 to 196°C (dec.).

I.R. (Nujol): 1780, 1695 $cm^{-1}$.

N.M.R. ($DMSO-d_6$, δ): 1.30 (9H, s), 3.70 (2H, m), 3.89 (3H, s), 4.22—4.70 (5H, m), 5.12 (1H, d, J = 5Hz), 5.80 (1H, dd, J = 5 and 8Hz), 7.52 (1H, s), 9.73 (1H, d, J = 8Hz).

(29) 7 - [2 - Methoxyimino - 2 - {2 - (2,2,2 - trifluoroacetamido)thiazol - 4 - yl}acetamido] - 3 - [1 - (2 - carboxy - 2 - aminoethyl) - 1H - tetrazol - 5 - yl]thiomethyl - 3 - cephem - 4 - carboxylic acid formate (syn isomer: in the 7-position).

I.R. (Nujol): 3160, 1760, 1650 $cm^{-1}$.

(30) 7 - [2 - Methoxyimino - 2 - (2 - aminothiazol - 4 - yl)acetamido] - 3 - [1 - (2 - carboxy - 2 - aminoethyl) - 1H - tetrazol - 5 - yl]thiomethyl - 3 - cephem - 4 - carboxylic acid (syn isomer: in the 7-position).

I.R. (Nujol): 1765, 1640 $cm^{-1}$.

(31) 7 - [2 - Methoxyimino - 2 - (2 - aminothiazol - 4 - yl)acetamido] - 3 - [1 - (3 - carboxy - 1 - propenyl) - 1H - tetrazol - 5 - yl]thiomethyl - 3 - cephem - 4 - carboxylic acid (syn isomer: in the 7-position).

I.R. (Nujol): 3300, 1760, 1660 $cm^{-1}$.

N.M.R. ($DMSO-d_6$, δ): 3.63 (2H, m), 3.87 (3H, s), 4.40 (2H, ABq, J = 14Hz), 5.03 (3H, m), 5.67 (2H, m), 6.30 (1H, m), 6.73 (1H, s), 7.27 (2H, broad s), 9.57 (1H, d, J = 8Hz).

(32) 7 - [2 - (2 - Propynyloxyimino) - 2 - {2 - (2,2,2 - trifluoroacetamido)thiazol - 4 - yl}acetamido] - 3 - [1 - (3 - carboxy - 1 - propenyl) - 1H - tetrazol - 5 - yl]thiomethyl - 3 - cephem - 4 - carboxylic acid (syn isomer: in the 7-position).

I.R. (Nujol): 2120, 1780, 1720 $cm^{-1}$.

N.M.R. (DMSO-$d_6$, δ): 3.47—3.70 (5H, m), 4.33—4.47 (2H, m), 4.80 (2H, m), 5.20 (1H, d, J = 5Hz), 5.77—6.34 (2h, m), 6.77—7.10 (1H, m), 7.63 (1H, s), 10.27 (1H, d, J = 8Hz).

(33) 7 - [2 - (2 - Propynyloxyimino) - 2 - (2 - aminothiazol - 4 - yl)acetamido] - 3 - [1 - (3 - carboxy - 1 - propenyl) - 1H - tetrazol - 5 - yl]thiomethyl - 3 - cephem - 4 - carboxylic acid (syn isomer: in the 7-position).

I.R. (Nujol): 3320, 2150, 1775, 1675 $cm^{-1}$.

(34) 7 - [2 - Methoxyimino - 2 - (2 - aminothiazol - 4 - yl)acetamido] - 3 - [1 - (1 - carboxyallyl) - 1H - tetrazol - 5 - yl]thiomethyl - 3 - cephem - 4 - carboxylic acid (syn isomer: in the 7-position).

I.R. (Nujol): 3350, 1765, 1660, 1605 $cm^{-1}$.

N.M.R. (DMSO-$d_6$, δ): 3.55 (2H, m), 3.87 (3H, s), 4.37 (2H, m), 4.67—4.83 (5H, m), 6.75 (1H, s), 7.30 (2H, broad s), 9.60 (1H, d, J = 8Hz).

(35) 7 - [2 - (2 - Cyclopenten - 1 - yl)oxyimino - 2 - (2 - formamidothiazol - 4 - yl)acetamido] - 3 - [1 - (3 - carboxy - 1 - propenyl) - 1H - tetrazol - 5 - yl]thiomethyl - 3 - cephem - 4 - carboxylic acid (syn isomer: in the 7-position).

I.R. (Nujol): 1770, 1660 $cm^{-1}$.

(36) 7 - [2 - (2 - Cyclopenten - 1 - yl)oxyimino - 2 - (2, - aminothiazol - 4 - yl)acetamido] - 3 - [1 - (3 - carboxy - 1 - propenyl) - 1H - tetrazol - 5 - yl]thiomethyl - 3 - cephem - 4 - carboxylic acid (syn isomer: in the 7-position).

I.R. (Nujol): 3280, 1770, 1660 $cm^{-1}$.

(37) 7 - [2 - Phenoxyimino - 2 - (2 - formamidothiazol - 4 - yl)acetamido] - 3 - [1 - (3 - carboxy - 1 - propenyl) - 1H - tetrazol - 5 - yl]thiomethyl - 3 - cephem - 4 - carboxylic acid (syn isomer: in the 7-position).

N.M.R. (DMSO-$d_6$, δ): 3.33—3.90 (6H, m), 4.10—4.67 (2H, m), 5.23 (1H, d, J = 5Hz), 5.80—6.33 (2H, m), 6.90—7.57 (6H, m), 7.73 (1H, s), 8.60 (1H, s), 9.03 (1H, d, J = 8Hz), 12.86 (1H, broad s).

(38) 7 - [2 - Phenoxyimino - 2 - (2 - aminothiazol - 4 - yl)acetamido] - 3 - [1 - (3 - carboxy - 1 - propenyl) - 1H - tetrazol - 5 - yl]thiomethyl - 3 - cephem - 4 - carboxylic acid (syn isomer: in the 7-position).

I.R. (Nujol): 3150, 1770, 1650, 1585 $cm^{-1}$.

(39) 7 - [2 - methoxyimino - 2 - (2 - aminothiazol - 4 - yl)acetamido] - 3 - [1 - [3 - (4 - methyl - 1 - piperazinyl)propyl] - 1H - tetrazol - 5 - yl]thiomethyl - 3 - cephem - 4 - carboxylic acid (syn isomer), powder.

I.R. (Nujol): 3300, 1760, 1660, 1600, 1530 $cm^{-1}$.

(40) 7 - [2 - methoxyimino - 2 - (2 - aminothiazol - 4 - yl)acetamido] - 3 - [1 - [3 - (4 - isopropyl - 1 - piperazinyl)propyl] - 1H - tetrazol - 5 - yl]thiomethyl - 3 - cephem - 4 - carboxylate (syn isomer), powder.

I.R. (Nujol): 3300, 1765, 1660, 1600, 1530 $cm^{-1}$.

(41) Sodium 7 - [2 - methoxyimino - 2 - (2 - aminothiazol - 4 - yl)acetamido] - 3 - [1 - [3 - (4 - benzyl - 1 - piperazinyl)propyl] - 1H - tetrazol - 5 - yl]thiomethyl - 3 - cephem - 4 - carboxylate (syn isomer), powder.

I.R. (Nujol): 3300, 1760, 1660, 1600 $cm^{-1}$.

(42) Sodium 7 - [2 - methoxyimino - 2 - (2 - aminothiazol - 4 - yl)acetamido] - 3 - [1 - [3 - (4 - allyl - 1 - piperazinyl)propyl] - 1H - tetrazol - 5 - yl]thiomethyl - 3 - cephem - 4 - carboxylate (syn isomer), powder.

I.R. (Nujol): 3350, 1770, 1670, 1600, 1530 $cm^{-1}$.

(43) Sodium 7 - [2 - methoxyimino - 2 - (2 - aminothiazol - 4 - yl)acetamido] - 3 - [1 - [3 - (4 - phenyl - 1 - piperazinyl)propyl] - 1H - tetrazol - 5 - yl]thiomethyl - 3 - cephem - 4 - carboxylate (syn isomer), powder.

I.R. (Nujol): 3300, 1765, 1665, 1600, 1530 $cm^{-1}$.

(44) 7 - [2 - methoxyimino - 2 - (2 - aminothiazol - 4 - yl)acetamido] - 3 - [1 - [3 - [4 - (2 - hydroxyethyl) - 1 - piperazinyl]propyl] - 1H - tetrazol - 5 - yl]thiomethyl - 3 - cephem - 4 - carboxylic acid (syn isomer), powder.

I.R. (Nujol): 3350, 3250, 1775, 1670, 1610, 1540 $cm^{-1}$.

(45) 7 - [2 - methoxyimino - 2 - (2 - aminothiazol - 4 - yl)acetamido] - 3 - [1 - [3 - (4 - ethoxycarbonyl - 1 - piperazinyl)propyl] - 1H - tetrazol - 5 - yl]thiomethyl - 3 - cephem - 4 - carboxylic acid (syn isomer), powder.

I.R. (Nujol): 3300, 1770, 1670, 1600, 1530 $cm^{-1}$.

(46) 7 - [2 - methoxyimino - 2 - (2 - aminothiazol - 4 - yl)acetamido] - 3 - [1 - [2 - (4 - methyl - 1 - piperazinyl)ethyl] - 1H - tetrazol - 5 - yl]thiomethyl - 3 - cephem - 4 - carboxylic acid (syn isomer) powder.

I.R. (Nujol): 3300, 1765, 1660, 1600, 1530 $cm^{-1}$.

(47) 7 - [2 - methoxyimino - 2 - (2 - aminothiazol - 4 - yl)acetamido] - 3 - [1 - (4 - methyl - 1 - piperazinyl)carbonylmethyl - 1H - tetrazol - 5 - yl]thiomethyl - 3 - cephem - 4 - carboxylic acid (syn isomer) powder.

I.R. (Nujol): 3300, 1760, 1660, 1600, 1530 $cm^{-1}$.

(48) 7α - methoxy - 7β - [D,L - 2 - carboxy - 2 - (4 - hydroxyphenyl)acetamido)] - 3 - [1 - [3 - (4 - methyl - 1 - piperazinyl)propyl] - 1H - tetrazol - 5 - yl]thiomethyl - 3 - cephem - 4 - carboxylic acid, powder.

I.R. (Nujol): 3200, 1760, 1670, 1600, 1505 $cm^{-1}$.

(49) 7 - [2 - (2-cyclopenten - 1 - yl)oxyimino - 2 - (2 - aminothaizol - 4 - yl)acetamido] - 3 - [1 - [3 - (4 - methyl - 1 - piperazinyl)propyl] - 1H - tetrazol - 5 - yl]thiomethyl - 3 - cephem - 4 - carboxylic acid (syn isomer), powder

I.R. (Nujol): 3300, 1770, 1660, 1620, 1530 $cm^{-1}$.

Example 15

A solution of 7 - [2 - (2 - cyclopenten - 1 - yl)oxyimino - 2 - (2 - formamidothiazol - 4 - yl)acetamido] - 3 - [1 -

(2 - carboxy - 2 - hydroxyiminoethyl) - 1H - tetrazol - 5 - yl]thiomethyl - 3 - cephem - 4 - carboxylic acid (syn isomer: in the 7-position) (1.1 g) and conc. hydrochloric acid (0.12 g) in methanol (10 ml) and tetrahydrofuran (2 ml) was stirred at room temperature for 2.5 hours. After removal of organic solvents under reduced pressure, the residue was dissolved in an aqueous solution of sodium bicarbonate and the solution was adjusted to pH 7.5. The aqueous solution was washed with ethyl acetate (15 ml) and then adjusted to pH 3.0 with 10% hydrochloric acid to give precipitates which were filtered and washed with water to give 7 - [2 - (2 - cyclopenten - 1 - yl)oxyimino - 2 - (2 - aminothiazol - 4 - yl)acetamido] - 3 - [1 - (2 - carboxy - 2 - hydroxyiminoethyl) - 1H - tetrazol - 5 - yl]thiomethyl - 3 - cephem - 4 - carboxylic acid (syn isomer: in the 7-position).

I.R. (Nujol): 3270, 3160, 1770, 1650 $cm^{-1}$.

N.M.R. (DMSO-$d_6$, δ): 1.78—2.80 (4H, m), 3.70 (2H, m), 4.38 (2H, q, J = 14.0Hz), 4.97—5.51 (4H, m), 5.61—6.31 (3H, m), 6.73 (1H, s), 9.53 (1H, d, J = 8.0Hz).

Example 16

A mixture of 7 - [2 - methoxyimino - 2 - {2 - (2,2,2 - trifluoroacetamido)thiazol - 4 - yl}acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position) (1.70 g) and sodium acetate·trihydrate (3.26 g) in water (14 ml) was stirred overnight at room temperature. The solution was adjusted to pH 2.5 with 10% hydrochloric acid under ice-cooling and the resultant precipitates were filtered and washed with water to give 7 - [2 - methoxyimino - 2 - (2 - aminothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position).

I.R. (Nujol): 3270, 1770, 1645 $cm^{-1}$.

N.M.R. (DMSO-$d_6$, δ): 9.60 (1H, d, J = 8Hz), 7.13 (2H, broad s), 6.72 (1H, s), 5.77 (1H, dd, J = 5 and 8Hz), 5.25 (2H, s), 5.09 (1H, d, J = 8Hz), 4.32 (2H, ABq, J=14Hz), 3.95 (3H, s), 3.80 (3H, s), 3.65 (2H, broad s).

Example 17

A mixture of 7 - [2 - (2 - propynyloxyimino) - 2 - {2 - (2,2,2 - trifluoroacetamido)thiazol - 4 - yl}acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position) (2.0 g) and sodium acetate·trihydrate (3.71 g) in water (15 ml) was stirred overnight at room temperature. The solution was adjusted to pH 2.5 with 10% hydrochloric acid under ice-cooling and the resultant precipitates were filtered and washed with water to give 7 - [2 - (2 - propynyloxyimino) - 2 - (2 - aminothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position). (1.12 g).

I.R. (Nujol): 3255, 1775, 1670, 1630 $cm^{-1}$.

N.M.R. (DMSO-$d_6$, δ): 9.57 (1H, d, J = 8Hz), 7.17 (2H, broad s), 6.70 (1H, s), 5.73 (1H, dd, J = 5 and 8Hz), 5.22 (2H, s), 5.07 (1H, d, J = 5Hz), 4.64 (2H, m), 4.33 (2H, m), 3.95 (3H, s), 3.63 (2H, m), 3.40 (1H, m).

Example 18

A mixture of 7 - [2 - (2 - cyclopenten - 1 - yl)oxyimino - 2 - (2 - formamidothiazol - 4 - yl)acetamido] - 3 - [1 - [3 - (4 - methyl - 1 - piperazinyl)propyl] - 1H - tetrazol - 5 - yl]thiomethyl - 3 - cephem - 4 - carboxylic acid (syn isomer) (1.1 g), methanol (85 ml) and conc·hydrochloric acid (0.6 ml) was stirred for 45 minutes at room temperature. The reaction mixture was adjusted to pH 4.5 with a saturated aqueous solution of sodium bicarbonate and then concentrated. The concentrate was adjusted to pH 7 with a saturated aqueous solution of sodium bicarbonate and then subjected to column chromatography (CM-cephadex, H-type: 110 ml), eluted with 1%, 2%, 4% and 5% aqueous solution of sodium chloride. The fractions containing the object compound were collected and adjusted to pH 4.3 with a saturated aqueous solution of sodium bicarbonate. The resulting solution was subjected to column chromatography (Non-ion adsorption resin, Diaion HP20 prepared by Mitsubishi Chemical Industries: 50 ml), eluting with water and 20% isopropyl alcohol. The fractions containing the object compound were collected and concentrated under reduced pressure. The concentrate was lyophilized to give 7 - [2 - (2 - cyclopenten - 1 - yl)oxyimino - 2 - (2 - aminothiazol - 4 - yl)acetamido] - 3 - [1 - [3 - (4 - methyl - 1 - piperazinyl)propyl] -1H - tetrazol - 5 - yl] - thiomethyl - 3 - cephem - 4 - carboxylic acid (syn isomer) (0.3 g).

I.R. (Nujol): 3300, 1770, 1660, 1620, 1530 $cm^{-1}$.

N.M.R. (DMSO-$d_6$, δ): 1.9—3.2 (16H, m), 2.63 (3H, s), 3.65 (2H, broad s), 4.28 (4H, broad s), 5.06 (1H, d, J = 5Hz), 5.2 (1H, broad s), 5.6—6.3 (3H, m), 6.54 (1H, s), 7.2 (2H, broad s), 10.13 (1H, d, J = 8Hz).

Example 19

The following compounds were prepared according to the similar manners to those of Examples 15 to 18.

(1) 7 - [2 - Methoxyimino - 2 - (2 - aminoithiazol - 4 - yl)acetamido] - 3 - [1 - (2 - carboxy - 2 - hydroxyiminoethyl) - 1H - tetrazol - 5 - yl]thiomethyl - 3 - cephem - 4 - carboxylic acid (syn isomer: in the 7-position), mp 169 to 173°C (dec.).

I.R. (Nujol): 3300, 3200, 1770, 1660, 1630 $cm^{-1}$.

N.M.R. (DMSO-$d_6$, δ): 3.70 (2H, m), 3.86 (3H, s), 4.36 (2H, q, J = 14.0Hz), 4.98—5.39 (3H, m), 5.80 (1H, dd, J = 4.0 and 8.0Hz), 6.77 (1H, s), 9.60 (1H, d, J = 8.0Hz).

(2) 7 - [2 - Cyclopentyloxyimino - 2 - (2 - aminothiazol - 4 - yl)acetamido] - 3 - [1 - (2 - carboxy - 2 - hydroxyiminoethyl) - 1H - tetrazol - 5 - yl]thiomethyl - 3 - cephem - 4 - carboxylic acid (syn isomer: in the 7-position), mp 168 to 171°C (dec.).

I.R. (Nujol): 3250, 3150, 1760, 1650 $cm^{-1}$.

N.M.R. (DMSO-$d_6$, δ): 1.28—2.03 (8H, m), 3.67 (2H, m), 4.33 (2H, m), 4.86 (1H, m), 4.99—5.46 (3H, m), 5.73 (1H, dd, J = 5 and 8.0Hz), 6.67 (1H, s), 9.49 (1H, d, J = 8.0Hz).

(3) 7 - [2 - (4 - Fluorobenzyl)oxyimino - 2 - (2 - aminothiazol - 4 - yl)acetamido] - 3 - [1 - (2 - carboxy - 2 - hydroxyiminoethyl) - 1H - tetrazol - 5 - yl]thiomethyl - 3 - cephem - 4 - carboxylic acid (syn isomer: in the 7-position), mp 160 to 163°C.

I.R. (Nujol): 3200, 1770, 1640 $cm^{-1}$.

N.M.R. (DMSO-$d_6$, δ): 3.69 (2H, m), 4.41 (2H, q, J = 14.0Hz), 5.03—5.48 (5H, m), 5.84 (1H, dd, J = 5.0 and 8.0Hz), 6.81 (1H, s), 6.89—7.70 (4H, m), 9.72 (1H, d, J = 8.0Hz).

(4) 7 - [2 - Isopropoxyimino - 2 - (2 - aminothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 140 to 150°C.

I.R. (Nujol): 3260, 1770, 1660 $cm^{-1}$.

N.M.R. (DMSO-$d_6$): 1.27 (6H, d, J = 6Hz), 3.68 (2H, m), 3.98 (3H, s), 4.22—4.50 (3H, m), 5.16 (1H, d, J = 5Hz), 5.27 (2H, s), 5.77 (1H, dd, J = 5 and 8Hz), 6.73 (1H, s), 9.55 (1H, d, J = 8Hz).

(5) 7 - [2 - Allyloxyimino - 2 - (2 - aminothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 130 to 140°C (dec.).

I.R. (Nujol): 3250, 1770, 1650 $cm^{-1}$.

N.M.R. (DMSO-$d_6$, δ): 3.73 (2H, m), 4.02 (3H, s), 4.40 (2H, m), 4.65 (2H, m), 5.31 (2H, s), 5.60—5.07 (3H, m), 6.40—5.70 (2H, m), 7.82 (1H, s), 9.69 (1H, d, J = 8Hz).

(6) 7 - [2 - (2 - Cyclopenten - 1 - yl)oxyimino - 2 - (2 - aminothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 140 to 150°C (dec.).

I.R. (Nujol): 3260, 1770, 1650 $cm^{-1}$.

N.M.R. (DMSO-$d_6$, δ): 1.80—2.43 (4H, m), 3.63 (2H, m), 3.97 (3H, s), 4.35 (2H, m), 5.03—5.43 (4H, m), 5.60—6.20 (3H, m), 6.69 (1H, s), 9.50 (1H, d, J = 8Hz).

(7) 7 - [2 - Benzyloxyimino - 2 - (2 - aminothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 168 to 172°C (dec.).

I.R. (Nujol): 3260, 1770, 1655 $cm^{-1}$.

N.M.R. (DMSO-$d_6$, δ): 3.67 (2H, m), 4.00 (3H, s), 4.37 (2H, ABq, J = 14Hz), 5.17 (3H, m) 5.30 (2H, s), 5.84 (1H, dd, J = 5 and 8Hz), 6.75 (1H, s), 7.37 (5H, m), 9.67 (1H, d, J = 8Hz).

(8) 7 - [2 - (4 - Fluorobenzyl)oxyimino - 2 - (2 - aminothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position), mp 130 to 140°C (dec.).

I.R. (Nujol): 3270, 1770, 1625$cm^{-1}$.

N.M.R. (DMSO-$d_6$, δ): 3.63 (2H, m), 3.95 (3H, s), 4.33 (2H, ABq, J = 14Hz), 5.11 (3H, m), 5.24 (2H, s), 5.79 (1H, dd, J = 5 and 8Hz), 6.73 (1H, s), 6.97—7.60 (4H, m), 9.66 (1H, d, J = 8Hz).

(9) 7 - [2 - (2 - Cyclopenten - 1 - yl)oxyimino - 2 - (2 - aminothiazol - 4 - yl)acetamido] - 3 - [1 - (2 - carboxy - 2 - allyloxyiminoethyl) - 1H - tetrazol - 5 - yl]thiomethyl - 3 - cephem - 4 - carboxylic acid (syn isomer: in the 7-position), mp 140 to 150°C (dec.).

I.R. (Nujol): 3270, 1770, 1660, 1630 $cm^{-1}$.

N.M.R. (DMSO-$d_6$, δ): 1.70—2.43 (4H, m), 3.67 (2H, m), 4.35 (2H, m), 4.73 (2H, m), 5.00—6.30 (10H, m), 6.70 (1H, s), 9.48 (1H, d, J = 8Hz).

(10) 7 - [2 - Benzyloxyimino - 2 - (2 - aminothiazol - 4 - yl)acetamido] - 3 - [1 - (2 - carboxy - 2 - allyloxyiminoethyl) - 1H - tetrazol - 5 - yl]thiomethyl - 3 - cephem - 4 - carboxylic acid (syn isomer: in the 7-position), mp 180 to 190°C (dec.).

I.R. (Nujol): 3290, 1775, 1705, 1655 $cm^{-1}$.

N.M.R. (DMSO-$d_6$, δ): 3.68 (2H, m), 4.40 (2H, m), 4.77 (2H, m), 5.03—5.50 (7H, m), 5.66—6.29 (2H, m), 6.77 (1H, s), 7.39 (5H, m), 9.71 (1H, d, J = 8Hz).

(11) 7 - [2 - (4 - Fluorobenzyl)oxyimino - 2 - (2 - aminothiazol - 4 - yl)acetamido] - 3 - [1 - (2 - carboxy - 2 - hydroxyethyl) - 1H - tetrazol - 5 - yl]thiomethyl - 3 - cephem - 4 - carboxylic acid (syn isomer: in the 7 - position).

I.R. (Nujol): 3280, 1765, 1630 $cm^{-1}$.

N.M.R. (DMSO-$d_6$, δ): 3.70 (2H, m), 4.20—4.70 (5H, m), 5.17 (3H, m), 5.82 (1H, dd, J = 5 and 8Hz), 6.80 (1H, s), 7.03—7.61 (4H, m), 9.73 (1H, d, J = 8Hz).

(12) 7 - [2 - Methoxyimino - 2 - (2 - aminothiazol - 4 - yl)acetamido] - 3 - [1 - (2 - carboxy - 2 - hydroxyethyl) - 1H - tetrazol - 5 - yl]thiomethyl - 3 - cephem - 4 - carboxylic acid (syn isomer: in the 7 - position), mp 140 to 150°C (dec.).

I.R. (Nujol): 3320, 1770, 1635 $cm^{-1}$.

N.M.R. (DMSO-$d_6$, δ): 3.70 (2H, m), 3.86 (3H, s), 4.13—4.78 (5H, m), 5.11 (1H, d, J = 5Hz), 5.76 (1H, dd, J = 5 and 8Hz), 6.77 (1H, s), 9.62 (1H, d, J = 8Hz).

(13) 7 - [2 - Methoxyimino - 2 - (2 - aminothiazol - 4 - yl)acetamido] - 3 - [1 - (2 - carboxy - 2 - aminoethyl) - 1H - tetrazol - 5 - yl]thiomethyl - 3 - cephem - 4 - carboxylic acid (syn isomer: in the 7 - position).

I.R. (Nujol): 1765, 1640 $cm^{-1}$.

N.M.R. (DMSO-$d_6$, δ): 3.72 (2H, m), 3.84 (3H, s), 4.00—4.91 (5H, m), 5.10 (1H, d, J = 5Hz), 5.75 (1H, dd, J = 5 and 8Hz), 6.74 (1H, s), 9.56 (1H, d, J = 8Hz).

(14) 7 - [2 - Methoxyimino - 2 - (2 - aminothiazol - 4 - yl)acetamido] - 3 - [1 - (3 - carboxy - 1 - propenyl) - 1H - tetrazol - 5 - yl]thiomethyl - 3 - cephem - 4 - carboxylic acid (syn isomer: in the 7 - position).

I.R. (Nujol): 3300, 1760, 1660 $cm^{-1}$.

(15) 7 - [2 - (2 - Propynyloxyimino) - 2 - (2 - aminothiazol - 4 - yl)acetamido] - 3 - [1 - (3 - carboxy - 1 - propenyl) - 1H - tetrazol - 5 - yl]thiomethyl - 3 - cephem - 4 - carboxylic acid (syn isomer: in the 7 - position).

I.R. (Nujol): 3320, 2150, 1775, 1675 $cm^{-1}$.

N.M.R. (DMSO-$d_6$, δ): 3.37 (2H, d, J = 6Hz), 3.47 (1H, m), 3.70 (2H, m), 4.37 (2H, d, J = 5Hz), 4.70 (2H, d, J = 2Hz), 5.10 (1H, d, J = 6Hz), 5.77 (1H, d,d, J = 8 and 6Hz), 6.30 (1H, ddd, J = 2, 10 and 12Hz), 6.77 (1H, s), 7.00 (1H, m), 7.27 (2H, broad), 9.67 (1H, d, J=8Hz).

(16) 7 - [2 - Methoxyimino - 2 - (2 - aminothiazol - 4 - yl)acetamido] - 3 - [1 - (1 - carboxyallyl) - 1H - tetrazol - 5 - yl]thiomethyl - 3 - cephem - 4 - carboxylic acid (syn isomer: in the 7 - position).

I.R. (Nujol): 3350, 1765, 1660, 1605 $cm^{-1}$.

(17) 7 - [2 - (2 - Cyclopenten - 1 - yl)oxyimino - 2 - (2 - aminothiazol - 4 - yl)acetamido] - 3 - [1 - (3 - carboxy - 1 - propenyl) - 1H - tetrazol - 5 - yl]thiomethyl - 3 - cephem - 4 - carboxylic acid (syn isomer: in the 7 - position).

I.R. (Nujol): 3280, 1770, 1660 $cm^{-1}$.

N.M.R. (DMSO-$d_6$, δ): 1.81—2.93 (4H, m), 3.40 (2H, q, J = 16.0Hz), 3.71 (2H, m), 4.39 (2H, q, J = 14.0Hz), 5.06—5.54 (2H, m), 5.62—6.43 (4h, m) 6.74 (1H, s), 7.01 (1H, d), 9.53 (1H, d, J = 8.0Hz).

(18) 7 - [2 - Phenoxyimino - 2 - (2 - aminothiazol - 4 - yl)acetamido] - 3 - [1 - (3 - carboxy - 1 - propenyl) - 1H - tetrazol - 5 - yl]thiomethyl - 3 - cephem - 4 - carboxylic acid (syn isomer: in the 7 - position).

I.R. (Nujol): 3150, 1770, 1650, 1585 $cm^{-1}$.

N.M.R. (DMSO-$d_6$, δ): 3.37 (2H, m), 3.73 (2H, broad s), 4.37 (2H, q, J = 12Hz), 5.17 (1H, d, J = 5Hz), 5.73—6.37 (2H, m), 6.87—7.33 (6H, m), 7.00 (1H, s), 9.80 (1H, d, J = 8Hz).

(19) 7 - [2 - methoxyimino - 2 - (2 - aminothiazol - 4 - yl)acetamido] - 3 - [1 - [3 - (4 - methyl - 1 - piperazinyl)propyl] - 1H - tetrazol - 5 - yl]thiomethyl - 3 - cephem - 4 - carboxylic acid (syn isomer), powder.

I.R. (Nujol): 3300, 1760, 1660, 1600, 1530 $cm^{-1}$.

(20) 7 - [2 - methoxyimino - 2 - (2 - aminothiazol - 4 - yl)acetamido] - 3 - [1 - [3 - (4 - isopropyl - 1 - piperazinyl)propyl] - 1H - tetrazol - 5 - yl]thiomethyl - 3 - cephem - 4 - carboxylic acid (syn isomer), powder.

I.R. (Nujol): 3300, 1765, 1660, 1600, 1530 $cm^{-1}$.

(21) Sodium 7 - [2 - methoxyimino - 2 - (2 - aminothiazol - 4 - yl)acetamido] - 3 - [1 - [3 - (4 - benzyl - 1 - piperazinyl)propyl] - 1H - tetrazol - 5 - yl]thiomethyl - 3 - cephem - 4 - carboxylate (syn isomer), powder.

I.R. (Nujol): 3300, 1760, 1660, 1600 $cm^{-1}$.

(22) Sodium 7 - [2 - methoxyimino - 2 - (2 - aminothiazol - 4 - yl)acetamido] - 3 - [1 - [3 - (4-allyl - 1 - piperazinyl)propyl] - 1H - tetrazol - 5 - yl]thiomethyl - 3 - cephem - 4 - carboxylate (syn isomer), powder.

I.R. (Nujol): 3350, 1770, 1670, 1600, 1530 $cm^{-1}$.

(23) Sodium 7 - [2 - methoxyimino - 2 - (2 - aminothiazol - 4 - yl)acetamido] - 3 - [1 - [3 - (4 - phenyl - 1 - piperazinyl)propyl] - 1H - tetrazol - 5 - yl]thiomethyl - 3 - cephem - 4 - carboxylic acid (syn isomer), powder.

I.R. (Nujol): 3300, 1765, 1665, 1600, 1530 $cm^{-1}$.

(24) 7 - [2 - methoxyimino - 2 - (2 - aminothiazol - 4 - yl)acetamido] - 3 - [1 - [4 - (2 - hydroxyethyl) - 1 - piperazinyl]propyl] - 1H - tetrazol - 5 - yl]thiomethyl - 3 - cephem - 4 - carboxylic acid (syn isomer), powder.

I.R. (Nujol): 3350, 3250, 1775, 1670, 1610, 1540 $cm^{-1}$.

(25) 7 - [2 - methoxyimino - 2 - (2 - aminothiazol - 4 - yl)acetamido] - 3 - [1 - [3 - (4 - ethoxycarbonyl - 1 - piperazinyl)propyl] - 1H - tetrazol - 5 - yl]thiomethyl - 3 - cephem - 4 - carboxylic acid (syn isomer), powder.

I.R. (Nujol): 3300, 1770, 1670, 1600, 1530 $cm^{-1}$.

(26) 7 - [2 - methoxyimino - 2 - (2 - aminothiazol - 4 - yl)acetamido] - 3 - [1 - [2 - (4 - methyl - 1 - piperazinyl)ethyl] - 1H - tetrazol - 5 - yl]thiomethyl - 3 - cephem - 4 - carboxylic acid (syn isomer), powder.

I.R. (Nujol): 3300, 1765, 1660, 1600, 1530 $cm^{-1}$.

(27) 7 - [2 - methoxyimino - 2 - (2 - aminothiazol - 4 - yl)acetamido] - 3 - [1 - (4 - methyl - 1 - piperazinyl)carbonylmethyl - 1H - tetrazol - 5 - yl]thiomethyl - 3 - cephem - 4 - carboxylic acid (syn isomer), powder.

I.R. (Nujol): 3300, 1760, 1660, 1600, 1530 $cm^{-1}$.

## Example 20

A solution of 7 - [2 - methoxyimino - 2 - {2 - (2,2,2 - trifluoroacetamido)thiazol - 4 - yl}acetamido] - 3 - [1 - (2 - carboxy - 2 - tert - butoxycarboxamidoethyl) - 1H - tetrazol - 5 - yl]thiomethyl - 3 - cephem - 4 - carboxylic acid (syn isomer: in the 7 - position) (1.1 g) in formic acid (11 ml) was stirred for 3 hours at 35 to 40°C. The

reaction mixture was concentrated. The residue was pulverized in acetonitril (25 ml) and stirred vigorously. The precipitates were collected by filtration and successively washed with acetonitril and diethyl ether to give 7 - [2 - methoxyimino - 2 - {2 - (2,2,2 - trifluoroacetamido)thiazol - 4 - yl}acetamido] - 3 - [1 - (2 - carboxy-2 - aminoethyl) - 1H - tetrazol - 5 - yl]thiomethyl - 3 - cephem - 4 - carboxylic acid·formate (syn isomer: in the 7-position).

I.R. (Nujol): 3160, 1760, 1650 $cm^{-1}$.

N.M.R. (DMSO-$d_6$, δ): 3.73 (2H, m), 3.88 (3H, s), 4.00—4.83 (5H, m), 5.13 (1H, d, J = 5Hz), 5.87 (1H, dd, J = 5 and 8Hz), 7.33 (1H, s), 9.68 (1H, d, J = 8Hz).

Example 21

The following compound was prepared according to the similar manner to that of Example 20.

7 - [2 - Methoxyimino - 2 - (2 - aminothiazol - 4 - yl)acetamido] - 3 - [1 - (2 - carboxy - 2 - aminoethyl) - 1H - tetrazol - 5 - yl]thiomethyl - 3 - cephem - 4 - carboxylic acid (syn isomer: in the 7 - position).

I.R. (Nujol): 1765 1640 $cm^{-1}$.

**Claims for the Contracting States: BE CH DE FR GB IT LI NL SE**

1. A cephem compound of the formula:

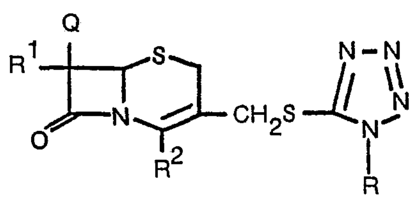

(I)

wherein

$R^1$ is amino or an acylamino group;

$R^2$ is carboxy or an esterified carboxy group;

R is a group of the formula: $—A_1—R^{3a}$ [in which $A_1$ is hydroxy($C_1$ to $C_6$)alkylene, amino($C_1$ to $C_6$)alkylene, protected amino($C_1$ to $C_6$)alkylene, alkenylene with up to 6C-atoms or hydroxyimino($C_1$ to $C_6$)alkylene wherein the hydrogen atom of the hydroxyimino group may be replaced with a ($C_1$ to $C_6$)aliphatic hydrocarbon group; and

$R^{3a}$ is carboxy or an esterified carboxy group] or a group of the formula

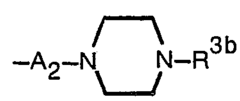

[in which $A_2$ is ($C_1$ to $C_6$)alkylene which may have an oxo group; and

$R^{3b}$ is carboxy, esterified carboxy, an aliphatic or aromatic group]; and

Q is hydrogen or ($C_1$ to $C_6$) alkoxy; and pharmaceutically acceptable salts thereof.

2. The compound of claim 1, wherein $R^1$, $R^2$, $R^{3a}$, $A_2$ and Q are as defined in claim 1, R is a group of the formula: $—A_1—R^{3a}$ [in which $A_1$ is hydroxy($C_1$ to $C_6$)alkylene, amino($C_1$ to $C_6$)alkylene, acylamino($C_1$ to $C_6$)alkylene, hydroxyimino($C_1$ to $C_6$)alkylene, ($C_1$ to $C_6$) alkoxyimino($C_1$ to $C_6$)alkylene, alkenyl-(with up to 6C-atoms)-oxyimino($C_1$ to $C_6$)alkylene or alkenylene with up to 6C-atoms; and

$R^{3B}$ is carboxy, esterified carboxy, ($C_1$ to $C_6$)alkyl, alkenyl with up to 6C-atoms, hydroxy($C_1$ to $C_6$)alkyl, ar($C_1$ to $C_6$)alkyl or aryl.

3. The compounds of claims 1 or 2, wherein

$R^1$ is amino;

$R^2$ is carboxy;

R is a group of the formula: $—A_1—R^{3a}$ [in which $A_1$ is hydroxy($C_1$ to $C_6$)alkylene, amino($C_1$ to $C_6$)alkylene, ($C_1$ to $C_6$)alkoxycarbonylamino($C_1$ to $C_6$)alkylene, hydroxyimino($C_1$ to $C_6$)alkylene, ($C_1$ to $C_6$) alkoxyimino($C_1$ to $C_6$)alkylene, alkenyl-(with up to 6C-atoms)-oxyimino($C_1$ to $C_6$)alkylene or alkenylene with up to 6C-atoms; and

$R^{3a}$ is carboxy] or a group of the formula:

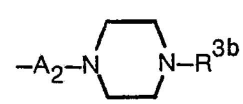

[in which $A_2$ is ($C_1$ to $C_6$)alkylene which may have an oxo group; and
$R^{3b}$ is carboxy, ($C_1$ to $C_6$)alkoxycarbonyl, ($C_1$ to $C_6$)alkyl, alkenyl with up to 6C-atoms, hydroxy($C_1$ to $C_6$)alkyl, phenyl($C_1$ to $C_6$)alkyl or phenyl]; and
Q is hydrogen.

4. The compound of claims 1 or 2, wherein
$R^1$ is a group of the formula:

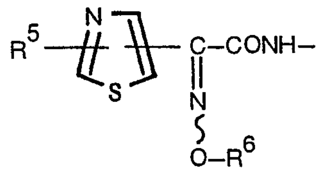

(in which $R^5$ is amino or a protected amino; and
$R^6$ is an aliphatic or aromatic group.

5. The compound of claim 4, which is the syn isomer.

6. The compound of claims 4 or 5, wherein the

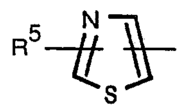

group is

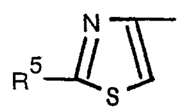

7. The compound of any of claims 4 to 6, wherein
$R^2$ is carboxy;
$R^5$ is amino; and
$R^6$ is $C_1$ to $C_6$ alkyl, alkenyl with up to 6C-atoms, alkynyl with up to 6C-atoms, cycloalkyl, cycloalkenyl with up to 6C-atoms, aryl, ar($C_1$ to $C_6$)alkyl which may have halogen.

8. The compound of any of claims 4 to 7,wherein
R is a group of the formula: —$A_1$—$R^{3a}$ with $A_1$ being as defined in claim 3 and $R^{3a}$ being as defined in claim 1, and
Q is hydrogen.

9. The compound of any of claims 4 to 7, wherein
R is group of the formula

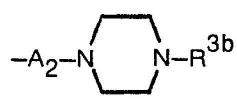

with $A_2$ being as defined in claim 1 and $R^{3b}$ as defined in claim 2, and
Q is hydrogen.

10. The compound of claims 1 or 2, wherein
$R^1$ is phenyl($C_1$ to $C_6$)alkanoylamino which may have hydroxy and/or carboxy.
R is a group of the formula:

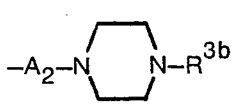

[in which $A_2$ is ($C_1$ to $C_6$)alkylene; $R^{3b}$ is ($C_1$ to $C_6$)alkyl];
$R^2$ is carboxy; and
Q is ($C_1$ to $C_6$)alkoxy.

11. A process for preparing a compound of formula (I) as defined in claim 1, or a pharmaceutically acceptable salt thereof, which comprises;
1) reacting a compound of the formula:

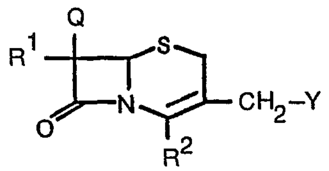

(II)

wherein
$R^1$, $R^2$ and Q are each as defined in claim 1 and
Y is a group which can be substituted by a group of the formula:

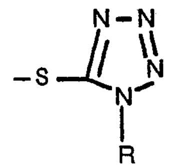

(wherein R is as defined in claim 1), or a salt thereof, with a compound of the formula:

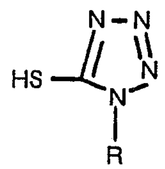

(III)

wherein
R is as defined in claim 1, or its reactive derivative at the mercapto group or a salt thereof; or
2) reacting a compound of the formula:

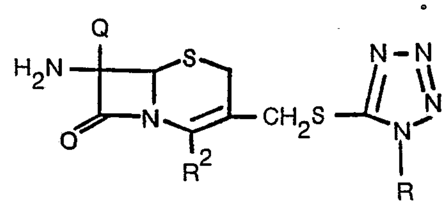

wherein
$R^2$, R and Q are each as defined in claim 1, or its reactive derivatives at the amino group or a salt thereof, with an acylating agent, to give a compound of the formula (I) wherein $R^1$ is acylamino, or a salt thereof; or
3) subjecting a compound of the formula:

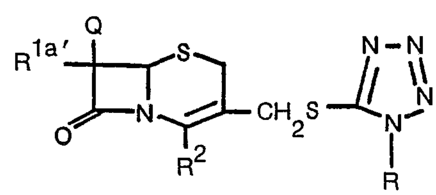

(Id)

wherein
$R^2$, R and Q are each as defined in claim 1 and
$R^{1a'}$ is acylamino having a protected amino group, or a salt thereof, to the elimination reaction of the amino protective group, to give a compound of the formula (I) wherein $R^1$ is acylamino having an amino group, or a salt thereof; or
4) subjecting a compound of the formula:

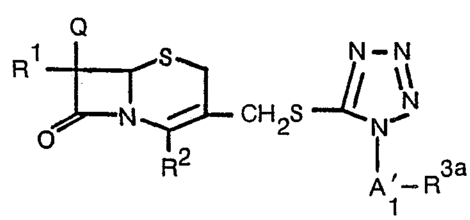

(If)

wherein

$R^1$, $R^2$, $R^{3a}$ and Q are each as defined in claim 1; and

$A^{1'}$ is a protected amino($C_1$ to $C_6$)alkylene, or a salt thereof to the elimination reaction of the amino protective group, to give a compound of the formula (I) wherein R is amino ($C_1$ to $C_6$)alkylene, or a salt thereof.

12. A pharmaceutical antibacterial composition comprising a compound of claim 1 in association with a pharmaceutically acceptable, substantially non-toxic carrier or excipient.

13. A compound of the formula I as defined in claim 1 for use as a medicament.

14. A compound of the formula I as defined in claim 1 for use in treating infectious diseases.

**Claims for the Contracting State: AT**

1. A process for preparing a compound of formula (I)

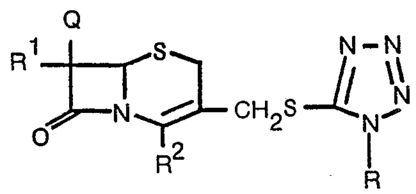

(I)

wherein

$R^1$ is amino or an acylamino group;

$R^2$ is carboxy or an esterified carboxy group;

R is a group of the formula: $—A_1—R^{3a}$ [in which $A_1$ is hydroxy($C_1$ to $C_6$)alkylene, amino($C_1$ to $C_6$)alkylene, protected amino($C_1$ to $C_6$)alkylene, alkenylene with up to 6C-atoms or hydroxyimino($C_1$ to $C_6$)alkylene wherein the hydrogen atom of the hydroxyimino group may be replaced with a ($C_1$ to $C_6$)aliphatic hydrocarbon group; and

$R^{3a}$ is carboxy or an esterified carboxy group] or a group of the formula

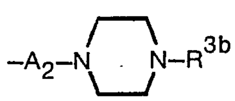

[in which $A_2$ is ($C_1$ to $C_6$)alkylene which may have an oxo group; and

$R^{3b}$ is carboxy, esterified carboxy, an aliphatic or aromatic group]; and

Q is hydrogen or ($C_1$ to $C_6$) alkoxy; and pharmaceutically acceptable salts thereof which comprises;

1) reacting a compound of the formula:

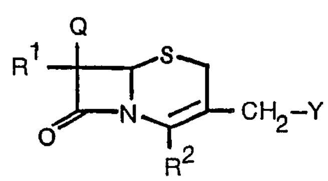

(II)

wherein

$R^1$, $R^2$ and Q are each as defined above and

Y is a group which can be substituted by a group of the formula:

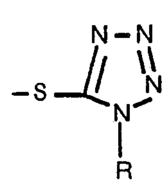

(wherein R is as defined in above), or a salt thereof, with a compound of the formula:

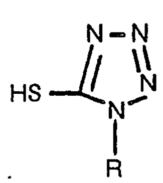

(III)

wherein

R is as defined above, or its reactive derivative at the mercapto group or a salt thereof; or

2) reacting a compound of the formula:

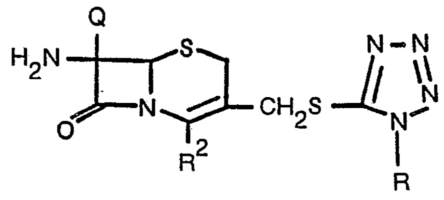

wherein

$R^2$, R and Q are each as defined above, or its reactive derivatives at the amino group or a salt thereof, with an acylating agent, to give a compound of the formula (I) wherein $R^1$ is acylamino, or a salt thereof; or

3) subjecting a compound of the formula:

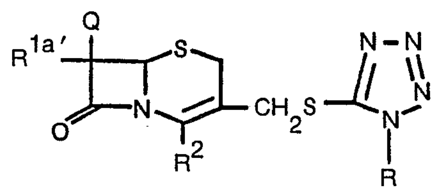

(Id)

wherein

$R^2$, R and Q are each as defined above and

$R^{1a'}$ is acylamino having a protected amino group, or a salt thereof, to the elimination reaction of the amino protective group, to give a compound of the formula (I) wherein $R^1$ is acylamino having an amino group, or a salt thereof; or

4) subjecting a compound of the formula:

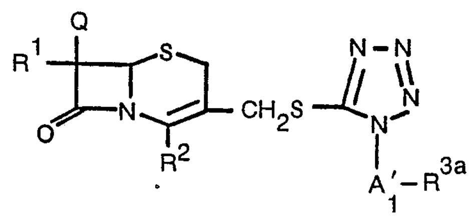

(If)

wherein

$R^1$, $R^2$, $R^{3a}$ and Q are each as defined above; and

$A^{1'}$ is a protected amino($C_1$ to $C_6$)alkylene, or a salt thereof to the elimination reaction of the amino protective group, to give a compound of the formula (I) wherein R is amino ($C_1$ to $C_6$)alkylene, or a salt thereof.

2. The process of claim 1, wherein $R^1$, $R^2$, $R^{3a}$, $A_2$ and Q are as defined in claim 1, R is a group of the formula: —$A_1$—$R^{3a}$ [in which $A_1$ is hydroxy($C_1$ to $C_6$)alkylene, amino($C_1$ to $C_6$)alkylene, acylamino($C_1$ to $C_6$)alkylene, hydroxyimino($C_1$ to $C_6$)alkylene, ($C_1$ to $C_6$) alkoxyimino($C_1$ to $C_6$)alkylene, alkenyl-(with up to 6C-atoms)-oxyimino($C_1$ to $C_6$)alkylene or alkenylene with up to 6C-atoms; and

$R^{3b}$ is carboxy, esterified carboxy, ($C_1$ to $C_6$)alkyl, alkenyl with up to 6C-atoms, hydroxy($C_1$ to $C_6$)alkyl, ar($C_1$ to $C_6$)alkyl or aryl.

3. The process of claims 1 or 2, wherein

$R^1$ is amino;

$R^2$ is carboxy;

R is a group of the formula: —$A_1$—$R^{3a}$ [in which $A_1$ is hydroxy($C_1$ to $C_6$)alkylene, amino($C_1$ to $C_6$)alkylene, ($C_1$ to $C_6$)alkoxycarbonylamino($C_1$ to $C_6$)alkylene, hydroxyimino($C_1$ to $C_6$)alkylene, ($C_1$ to $C_6$) alkoxyimino($C_1$ to $C_6$)alkylene, alkenyl-(with up to 6C-atoms)-oxyimino($C_1$ to $C_6$)alkylene or alkenylene with up to 6C-atoms; and

$R^{3a}$ is carboxy] or a group of the formula:

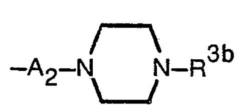

[in which $A_2$ is ($C_1$ to $C_6$)alkylene which may have an oxo group; and

$R^{3b}$ is carboxy, ($C_1$ to $C_6$)alkoxycarbonyl, ($C_1$ to $C_6$)alkyl, alkenyl with up to 6C-atoms, hydroxy($C_1$ to $C_6$)alkyl, phenyl($C_1$ to $C_6$)alkyl or phenyl]; and

Q is hydrogen.

4. The process of claims 1 or 2, wherein

$R^1$ is a group of the formula:

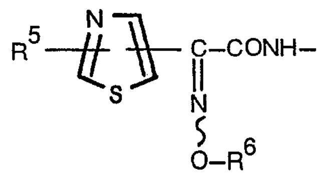

(in which $R^5$ is amino or a protected amino; and

$R^6$ is an aliphatic or aromatic group.

5. The process of claim 4, wherein the syn isomer is obtained.

6. The process of claims 4 or 5, wherein the

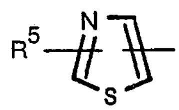

group is

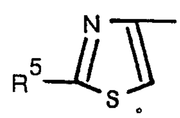

7. The process of any of claims 4 to 6, wherein

$R^2$ is carboxy;

$R^5$ is amino; and

$R^6$ is $C_1$ to $C_6$ alkyl, alkenyl with up to 6C-atoms, alkynyl with up to 6C-atoms, cycloalkyl, cycloalkenyl with up to 6C-atoms, aryl, ar($C_1$ to $C_6$)alkyl which may have halogen.

8. The process of any of claims 4 to 7, wherein

R is a group of the formula: —$A_1$—$R^{3a}$ with $A_1$ being as defined in claim 3 and $R^{3a}$ being as defined in claim 1, and

Q is hydrogen.

9. The process of any of claims 4 to 7, wherein

R is group of the formula

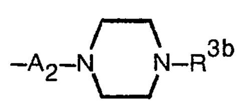

with $A_2$ being as defined in claim 1 and $R^{3b}$ as defined in claim 2, and

Q is hydrogen.

10. The process of claims 1 or 2, wherein

$R^1$ is phenyl($C_1$ to $C_6$)alkanoylamino which may have hydroxy and/or carboxy.

R is a group of the formula:

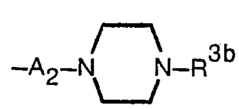

[in which $A_2$ is ($C_1$ to $C_6$)alkylene; $R^{3b}$ is ($C_1$ to $C_6$)alkyl];

$R^2$ is carboxy; and

Q is ($C_1$ to $C_6$)alkoxy.

**Patentansprüche für die Vertragsstaaten: BE CH DE FR GB IT LI NL SE**

1. Cephem-Verbindung der Formel:

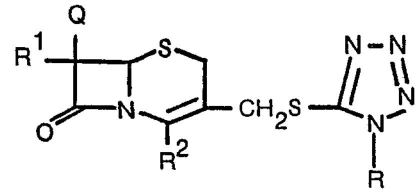

(I)

worin

$R^1$ Amino oder eine Acylaminogruppe ist;

$R^2$ Carboxy oder eine veresterte Carboxygruppe ist;

R eine Gruppe der Formel:

$$—A_1—R^{3a}$$

[worin $A^1$ Hydroxy-($C_1$ bis $C_6$)-alkylen, Amino-($C_1$ bis $C_6$)-alkylen, geschütztes Amino-($C_1$ bis $C_6$)-alkylen, Alkenylen mit bis zu 6C-Atomen oder Hydroxyimino-($C_1$ bis $C_6$)-alkylen ist, worin das Wasserstoffatom der Hydroxyiminogruppe ersetzt sein kann durch eine aliphatische ($C_1$ bis $C_6$)-Kohlenwasserstoffgruppe; und $R^{3a}$ Carboxy oder eine veresterte Carboxygruppe ist] oder eine Gruppe der Formel:

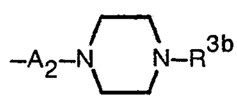

ist,

[worin $A^2$ ($C_1$ bis $C_6$)-Alkylen ist, das eine Oxogruppe haben kann; und $R^{3b}$ Carboxy, verestertes Carboxy, eine aliphatische oder aromatische Gruppe ist]; und

Q Wasserstoff oder ($C_1$ bis $C_6$)-Alkoxy ist;

und pharmazeutisch brauchbare Salze davon.

2. Verbindung nach Anspruch 1, worin $R^1$, $R^2$, $R^{3a}$, $A_2$ und Q wie in Anspruch 1 definiert sind R eine Gruppe der Formel:

$$—A_1—R^{3a}$$

ist,

[worin $A^1$ Hydroxy-($C_1$ bis $C_6$)-alkylen, Amino-($C_1$ bis $C_6$)-Alkylen, Acylamino-($C_1$ bis $C_6$)-alkylen, Hydroxyimino-($C_1$ bis $C_6$)-alkylen, ($C_1$ bis $C_6$)-Alkoxyimino-($C_1$ bis $C_6$)-alkylen, Alkenyl-(mit bis zu 6C-Atomen)-oxyimino-($C_1$ bis $C_6$)-alkylen oder Alkenylen mit bis zu 6C-Atomen ist; und $R^{3b}$ Carboxy, verestertes Carboxy, ($C_1$ bis $C_6$)-Alkyl, Alkenyl mit bis zu 6C-Atomen, Hydroxy-($C_1$ bis $C_6$)-alkyl, Ar-($C_1$ bis $C_6$)-alkyl oder Aryl ist].

3. Verbindung nach Anspruch 1 oder 2, worin $R^1$ Amino ist; $R^2$ Carboxy ist; R eine Gruppe der Formel:

$$—A_1—R^{3a}$$

[worin $A^1$ Hydroxy-($C_1$ bis $C_6$)-alkylen, Amino-($C_1$ bis $C_6$)-alkylen, ($C_1$ bis $C_6$)-Alkoxycarbonylamino-($C_1$ bis $C_6$)-alkylen, Hydroxyimino-($C_1$ bis $C_6$)-alkylen, ($C_1$ bis $C_6$)-Alkoxyimino-($C_1$ bis $C_6$)-alkylen, Alkenyl-(mit bis zu 6C-Atomen)-oxyimino-($C_1$ bis $C_6$)-alkylen oder Alkenylen mit bis zu 6C-Atomen ist; und $R^{3a}$ Carboxy ist] oder eine Gruppe der Formel:

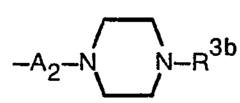

ist,

[worin $A_2$ ($C_1$ bis $C_6$)-Alkylen ist, das eine Oxogruppe haben kann; und $R^{3b}$ Carboxy, ($C_1$ bis $C_6$)-Alkoxycarbonyl, ($C_1$ bis $C_6$)-Alkyl, Alkenyl mit bis zu 6C-Atomen, Hydroxy-($C_1$ bis $C_6$)-alkyl, Phenyl-($C_1$ bis $C_6$)-alkyl oder Phenyl ist]; und

Q Wasserstoff ist.

4. Verbindung nach Anspruch 1 oder 2, worin $R^1$ eine Gruppe der Formel:

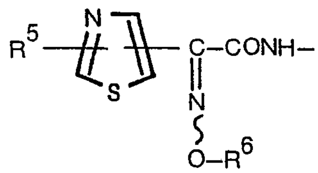

ist
(worin $R^5$ Amino oder ein geschütztes Amino ist; und $R^6$ eine aliphatische oder aromatische Gruppe ist).

5. Verbindung nach Anspruch 4, die das syn-Isomere ist.

6. Verbindung nach Anspruch 4 oder 5, in der die

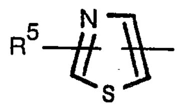

Gruppe folgende Gruppe ist

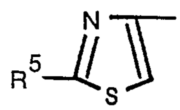

7. Verbindung nach einem der Ansprüche 4 bis 6, in der $R^2$ Carboxy ist; $R^5$ Amino ist; und $R^6$ $C_1$ bis $C_6$-Alkyl, Alkenyl mit bis zu 6C-Atomen, Alkinyl mit bis zu 6C-Atomen, Cycloalkyl, Cycloalkenyl mit bis zu 6C-Atomen, Aryl, Ar-($C_1$ bis $C_6$)-alkyl, das Halogen haben kann, ist.

8. Verbindung nach einem der Ansprüche 4 bis 7, worin R eine Gruppe der Formel:

$$—A_1—R^{3a}$$

ist,
wobei $A_1$ wie in Anspruch 3 definiert ist und $R^{3a}$ wie in Anspruch 1 definiert ist, und Q Wasserstoff ist.

9. Verbindung nach einem der Ansprüche 4 bis 7, worin R die Gruppe der Formel:

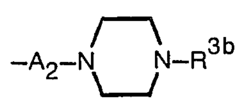

ist,
worin $A_2$ wie in Anspruch 1 definiert ist und $R^{3b}$ wie in Anspruch 2 definiert ist, und Q Wasserstoff ist.

10. Verbindung nach den Ansprüchen 1 oder 2, worin $R^1$ Phenyl-($C_1$ bis $C_6$)-alkanoylamino, das Hydroxy und/oder Carboxy haben kann, ist; R eine Gruppe der Formel:

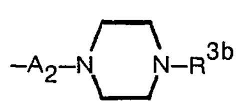

ist,
[worin $A_2$ ($C_1$ bis $C_6$)-Alkylen ist, $R^{3b}$ ($C_1$ bis $C_6$)-Alkyl ist]; $R^2$ Carboxy ist; und Q ($C_1$ bis $C_6$)-Alkoxy ist.

11. Verfahren zur Herstellung einer Verbindung der Formel I, wie in Anspruch 1 definiert, oder eines pharmazeutisch brauchbaren Salzes davon, durch

1) Reaktion einer Verbindung der Formel:

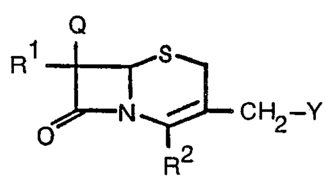

(II)

worin
$R^1$, $R^2$ und Q jeweils wie in Anspruch 1 definiert sind und
Y eine Gruppe ist, die substituiert sein kann durch eine Gruppe der Formel:

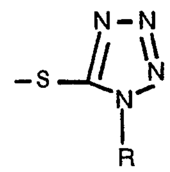

(worin R wie in Anspruch 1 definiert ist) oder eines Salzes davon mit einer Verbindung der Formel:

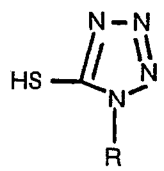
(III)

worin R wie in Anspruch 1 definiert ist, oder ihrem reaktiven Derivat an der Mercaptogruppe oder einem Salz davon, oder

2) Reaktion einer Verbindung der Formel:

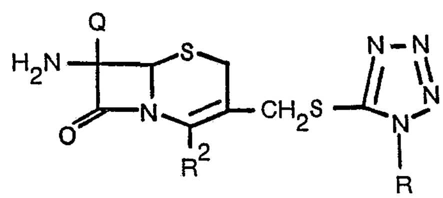

worin

$R^2$, R und Q jeweils wie in Anspruch 1 definiert sind, oder ihrer reaktiven Derivate an der Aminogruppe oder eines Salzes davon, mit einem Acylierungsmittel, unter Bildung einer Verbindung der Formel I, worin $R^1$ Acylamino ist oder eines Salzes davon, oder

3) Unterwerfen einer Verbindung der Formel:

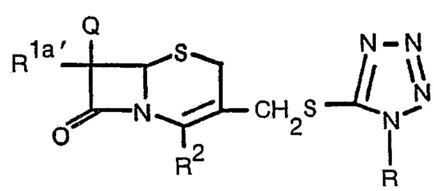
(Id)

worin

$R^2$, R und Q jeweils wie in Anspruch 1 definiert sind, und

$R^{1a'}$ Acylamino mit einer geschützten Aminogruppe ist, oder eines Salzes davon, der Eliminierungsreaktion der Aminoschutzgruppe, unter Bildung einer Verbindung der Formel I, worin $R^1$ Acylamino mit einer Aminogruppe ist, oder eines Salzes davon; oder

4) Unterwerfen einer Verbindung der Formel:

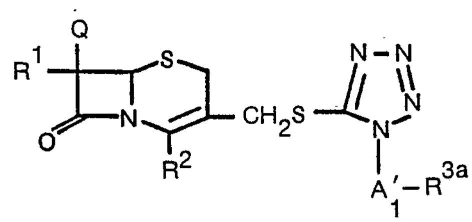
(If)

worin

$R^1$, $R^2$, $R^{3a}$ und Q jeweils wie in Anspruch 1 definiert sind; und

$A^{1'}$ ein geschütztes Amino-($C_1$ bis $C_6$)-alkylen ist, oder eines Salzes davon, der Eliminierungsreaktion für die Aminoschutzgruppe, unter Bildung einer Verbindung der Formel I, worin R Amino-($C_1$ bis $C_6$)-alkylen ist, oder eines Salzes davon.

12. Pharmazeutische antibakterielle Zusammensetzung, enthaltend eine Verbindung nach Anspruch 1, zusammen mit einem pharmazeutisch brauchbaren im wesentlichen nicht-toxischen Träger oder Exzipienten.

13. Verbindung der Formel I, wie in Anspruch 1 definiert, zur Verwendung als ein Arzneimittel.

14. Verbindung der Formel I, wie in Anspruch 1 definiert, zur Verwendung bei der Behandlung von Infektionserkrankungen.

**Patentansprüche für den Vertragsstaat: AT**

1. Verfahren zur Herstellung einer Verbindung der Formel (I)

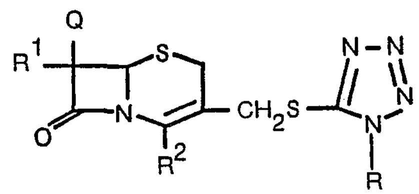

(I)

worin

$R^1$ Amino oder eine Acylaminogruppe ist;

$R^2$ Carboxy oder eine veresterte Carboxygruppe ist;

R eine Gruppe der Formel:

$$—A_1—R^{3a}$$

[worin $A^1$ Hydroxy-($C_1$ bis $C_6$)-alkylen, Amino-($C_1$ bis $C_6$)-alkylen, geschütztes Amino-($C_1$ bis $C_6$)-alkylen, Alkenylen mit bis zu 6C-Atomen oder Hydroxyimino-($C_1$ bis $C_6$)-alkylen, worin das Wasserstoffatom der Hydroxyiminogruppe ersetzt sein kann durch eine aliphatische ($C_1$ bis $C_6$)-Kohlenwasserstoffgruppe, ist; und $R^{3a}$ Carboxy oder eine veresterte Carboxygruppe ist] oder eine Gruppe der Formel:

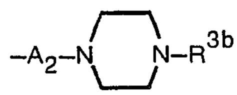

ist,

[worin $A^2$ ($C_1$ bis $C_6$)-Alkylen, das eine Oxogruppe haben kann, ist; und $R^{3b}$ Carboxy, verestertes Carboxy, eine aliphatische oder aromatische Gruppe ist]; und

Q Wasserstoff oder ($C_1$ bis $C_6$)-Alkoxy ist;

oder eines pharmazeutisch brauchbare Salzes davon, durch:

1) Reaktion einer Verbindung der Formel:

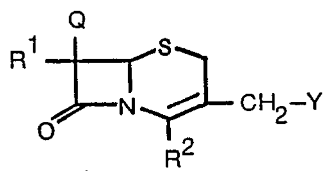

(II)

worin

$R^1$, $R^2$ und Q jeweils wie vorstehend definiert sind, und

Y eine Gruppe ist, die ersetzt werden kann durch eine Gruppe der Formel:

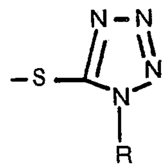

(worin R wie vorstehend definiert ist) oder eines Salzes davon mit einer Verbindung der Formel:

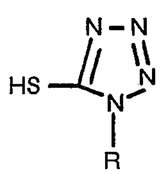

(III)

worin R wie vorstehend definiert ist, oder ihrem reaktiven Derivat an der Mercaptogruppe oder einem Salz davon, oder

2) Reaktion einer Verbindung der Formel:

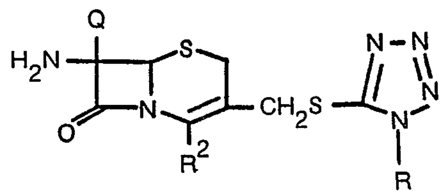

worin

$R^2$, R und Q jeweils wie vorstehend definiert sind oder ihrer reaktiven Derivate an der Aminogruppe oder eines Salzes davon, mit einem Acylierungsmittel, unter Bildung einer Verbindung der Formel I, worin $R^1$ Acylamino ist, oder eines Salzes davon, oder

3) Unterwerfen einer Verbindung der Formel:

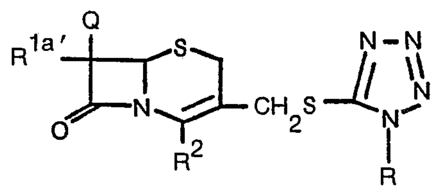

(Id)

worin

$R^2$, R und Q jeweils wie vorstehend definiert sind, und

$R^{1a'}$ Acylamino mit einer geschützten Aminogruppe ist, oder eines Salzes davon, der Eliminierungsreaktion der Aminoschutzgruppe, unter Bildung einer Verbindung der Formel I, worin $R^1$ Acylamino mit einer Aminogruppe ist, oder eines Salzes davon; oder

4) Unterwerfen einer Verbindung der Formel:

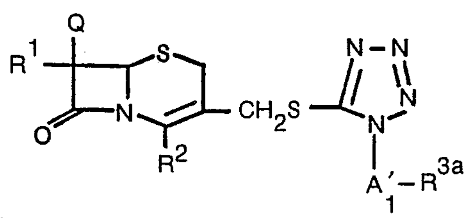

(If)

worin

$R^1$, $R^2$, $R^{3a}$ und Q jeweils wie vorstehend definiert sind; und

$A^{1'}$ ein geschütztes Amino-($C_1$ bis $C_6$)-alkylen ist, oder eines Salzes davon, der Eliminierungsreaktion für die Aminoschutzgruppe, unter Bildung einer Verbindung der Formel I, worin R Amino-($C_1$ bis $C_6$)-alkylen ist, oder eines Salzes davon.

2. Verfahren nach Anspruch 1, worin $R^1$, $R^2$, $R^{3a}$, $A_2$ und Q wie in Anspruch 1 definiert sind, R eine Gruppe der Formel:

$$—A_1—R^{3a}$$

ist,

[worin $A_1$ Hydroxy-($C_1$ bis $C_6$)-alkylen, Amino-($C_1$ bis $C_6$)-Alkylen, Acylamino-($C_1$ bis $C_6$)-alkylen, Hydroxyimino-($C_1$ bis $C_6$)-alkylen, ($C_1$ bis $C_6$)-Alkoxyimino-($C_1$ bis $C_6$)-alkylen, Alkenyl-(mit bis zu 6C-Atomen)-oxyimino-($C_1$ bis $C_6$)-alkylen oder Alkenylen mit bis zu 6C-Atomen ist; und $R^{3b}$ Carboxy, verestertes Carboxy, ($C_1$ bis $C_6$)-Alkyl, Alkenyl mit bis zu 6C-Atomen, Hydroxy-($C_1$ bis $C_6$)-alkyl, Ar-($C_1$ bis $C_6$)-alkyl oder Aryl ist].

3. Verfahren nach Anspruch 1 oder 2, worin $R^1$ Amino ist; $R^2$ Carboxy ist; R eine Gruppe der Formel:

$$—A_1—R^{3a}$$

[worin $A_1$ Hydroxy-($C_1$ bis $C_6$)-alkylen, Amino-($C_1$ bis $C_6$)-alkylen, ($C_1$ bis $C_6$)-Alkoxycarbonylamino-($C_1$ bis $C_6$)-alkylen, Hydroxyimino-($C_1$ bis $C_6$)-alkylen, ($C_1$ bis $C_6$)-Alkoxyimino-($C_1$ bis

$C_6$)-alkylen, Alkenyl-(mit bis zu 6C-Atomen)-oxyimino-($C_1$ bis $C_6$)-alkylen oder Alkenylen mit bis zu 6C-Atomen ist; und $R^{3a}$ Carboxy ist] oder eine Gruppe der Formel:

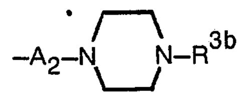

ist,

[worin $A_2$ ($C_1$ bis $C_6$)-Alkylen, das eine Oxogruppe haben kann; und $R^{3b}$ Carboxy, ($C_1$ bis $C_6$)-Alkoxycarbonyl, ($C_1$ bis $C_6$)-Alkyl, Alkenyl mit bis zu 6C-Atomen, Hydroxy-($C_1$ bis $C_6$)-Alkyl, Phenyl-($C_1$ bis $C_6$)-Alkyl oder Phenyl ist]; und

Q Wasserstoff ist.

4. Verfahren nach Anspruch 1 oder 2, worin $R^1$ eine Gruppe der Formel:

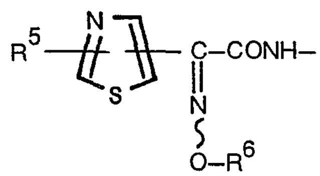

ist

(worin $R^5$ Amino oder ein geschütztes Amino ist; und $R^6$ eine aliphatische oder aromatische Gruppe ist).

5. Verfahren nach Anspruch 4, bei der das syn-Isomere erhalten wird.

6. Verfahren nach Anspruch 4 oder 5, in der die

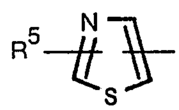

Gruppe die Gruppe

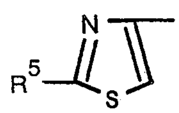

ist.

7. Verfahren nach einem der Ansprüche 4 bis 6, worin $R^2$ Carboxy ist; $R^5$ Amino ist; und $R^6$ $C_1$ bis $C_6$-Alkyl, Alkenyl mit bis zu 6C-Atomen, Alkinyl mit bis zu 6C-Atomen, Cycloalkyl, Cycloalkenyl mit bis zu 6C-Atomen, Aryl, Ar-($C_1$ bis $C_6$)-alkyl, das Halogen haben kann, ist.

8. Verfahren nach einem der Ansprüche 4 bis 7, worin R eine Gruppe der Formel:

$$—A_1—R^{3a}$$

ist, worin $A_1$ wie in Anspruch 3 definiert ist und $R^{3a}$ wie in Anspruch 1 definiert ist, und Q Wasserstoff ist.

9. Verfahren nach einem der Ansprüche 4 bis 7, worin R die Gruppe der Formel:

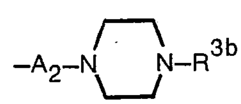

ist,

worin $A_2$ wie in Anspruch 1 definiert ist und $R^{3b}$ wie in Anspruch 2 definiert ist, und Q Wasserstoff ist.

10. Verfahren nach den Ansprüchen 1 oder 2, worin $R^1$ Phenyl-($C_1$ bis $C_6$)-alkanoylamino, das Hydroxy und/oder Carboxy haben kann, ist; R eine Gruppe der Formel:

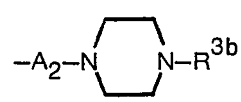

ist,

[worin $A_2$ ($C_1$ bis $C_6$)-Alkylen ist, $R^{3b}$ ($C_1$ bis $C_6$)-Alkyl ist]; $R^2$ Carboxy ist; und Q ($C_1$ bis $C_6$)-Alkoxy ist.

**Revendications pour les Etats contractants: BE CH DE FR GB IT LI NL SE**

1. Composé de céphen ayant la formule:

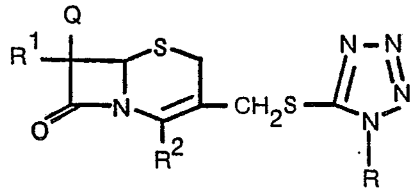

(I)

où

$R^1$ est le groupe amino ou un groupe acylamino;
$R^2$ est le groupe carboxy ou un groupe carboxy estérifié;
R est un groupe de formule:

$$—A_1—R^{3a}$$

[dans laquelle $A_1$ est un groupe hydroxyalkylène en $C_1$ à $C_6$, aminoalkylène en $C_1$ à $C_6$, amino protégé alkylène en $C_1$ à $C_6$, alkénylène ayant jusqu'à 6 atomes de carbone ou hydroxyiminoalkylène en $C_1$ à $C_6$ où l'atome d'hydrogène du groupe hydroxyimino peut être remplacé par un groupe hydrocarboné aliphatique en $C_1$ à $C_6$; et $R^{3a}$ est le groupe carboxy ou un groupe carboxy estérifié] ou un groupe de formule:

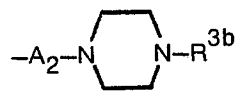

[où $A_2$ est un groupe alkylène en $C_1$ à $C_6$ qui peut avoir un groupe oxo; et $R^{3b}$ est un groupe carboxy, un groupe carboxy estérifié, un groupe aliphatique ou aromatique]; et
Q est l'hydrogène ou un groupe alcoxy en $C_1$ à $C_6$;
et ses sels pharmaceutiquement acceptables.

2. Composé selon la revendication 1, dans lequel $R^1$, $R^2$, $R^{3a}$, $A_2$ et Q sont comme définis dans la revendication 1, R est un groupe de formule:

$$—A_1—R^{3a}$$

[où $A_1$ est groupe hydroxyalkylène en $C_1$ à $C_6$ aminoalkylène, en $C_1$ à $C_6$, acylamiloalkylène en $C_1$ à $C_6$, hydroxyiminoalkylène en $C_1$ à $C_6$, alcoxy en $C_1$ à $C_6$ iminoalkylène en $C_1$ à $C_6$, alkényl (ayant jusqu'à 6 atomes de carbone) oxyiminoalkylène en $C_1$ à $C_6$ ou alkénylène ayant jusqu'à 6 atomes de carbone; et $R^{3b}$ est le groupe carboxy, un groupe carboxy estérifié, un groupe alkyle en $C_1$ à $C_6$, alkényle ayant jusqu'à 6 atomes de carbone, hydroxy alkyle en $C_1$ à $C_6$, ar-alkyle en $C_1$ ou $C_6$ aryle.

3. Composé selon les revendications 1 ou 2, dans lequel $R^1$ est le groupe amino; $R^2$ est le groupe carboxy; R est un groupe de formule:

$$—A_1—R^{3a}$$

[où $A_1$ est un groupe hydroxyalkylène en $C_1$ à $C_6$, aminoalkylène en $C_1$ à $C_6$, alcoxy en $C_1$ à $C_6$ carbonylaminoalkylène en $C_1$ à $C_6$, hydroxyiminoalkylène en $C_1$ à $C_6$, alcoxy en $C_1$ à $C_6$ iminoalkylène en $C_1$ à $C_6$ alkényl (ayant jusqu'à 6 atomes de carbone) oxyiminoalkylène en $C_1$ à $C_6$ ou alkénylène ayant jusqu'à 6 atomes de carbone; et $R^{3a}$ est le groupe carboxy] ou un groupe de formule:

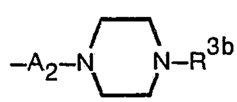

[où $A_2$ est un groupe alkylène en $C_1$ à $C_6$ qui peut avoir un groupe oxo; et
$R^{3b}$ est un groupe carboxy, un groupe alcoxy en $C_1$ à $C_6$ carbonyle, alkyle en $C_1$ à $C_6$, alkényle ayant jusqu'à 6 atomes de carbone, hydroxyalkyle en $C_1$ à $C_6$, phényl-alkylé en $C_1$ à $C_6$ ou phényle; et
Q est l'hydrogène

4. Composé selon la revendication 1 ou 2, dans lequel $R^1$ est un groupe de formule:

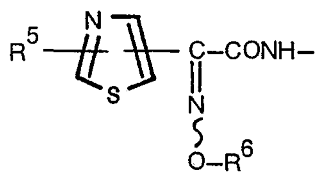

(où $R^5$ est le groupe amino ou un groupe amino protégé; et $R^6$ est un groupe aliphatique ou aromatique).

5. Composé selon la revendication 4, qui est l'isomère syn.

6. Composé selon les revendications 4 ou 5, dans lequel le groupe

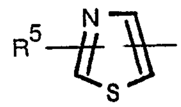

est

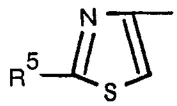

7. Composé selon l'une quelconque des revendications 4 à 6, dans lequel $R^2$ est le groupe carboxy; $R^5$ est le groupe amino; et $R^6$ est un groupe alkyle en $C_1$ à $C_6$, alkényle ayant jusqu'à 6 atomes de carbone, alkynyle ayant jusqu'à 6 atomes de carbone, cycloalkyle, cycloalkényle ayant jusqu'à 6 atomes de carbone, aryle, ar- alkyle en $C_1$ à $C_6$ qui peut avoir un halogène.

8. Composé selon l'une quelconque des revendications 4 à 7, dans lequel R est un groupe de formule:

$$—A_1—R^{3a}$$

$A_1$ étant comme défini dans la revendication 3, et $R^3$ étant comme défini dans la revendication 1, et Q est l'hydrogène.

9. Composé selon l'une quelconque des revendications 4 à 7, dans lequel R est un groupe de formule:

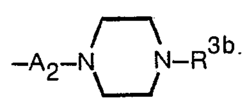

$A_2$ étant comme défini dans la revendication 1 et $R^{3b}$ comme défini dans la revendication 2 et Q est l'hydrogène.

10. Composé selon la revendication 1 ou 2, dans lequel $R^1$ est un groupe phénylalcanoyl (en $C_1$ à $C_6$) amino qui peut avoir un groupe hydroxy et/ou carboxy, R est un groupe de formule

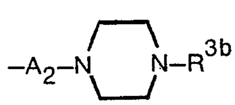

[où $A_2$ est un groupe alkylène en $C_1$ à $C_6$ et $R^{3b}$ est un groupe alkyle en $C_1$ à $C_6$]

$R^2$ est un groupe carboxy; et

Q est un groupe alkoxy en $C_1$ à $C_6$.

11. Procédé pour préparer un composé de formule (I) comme défini dans la revendication 1, ou son sel pharmaceutiquement acceptable, qui consiste:

1) à faire réagir un composé ayant la formule:

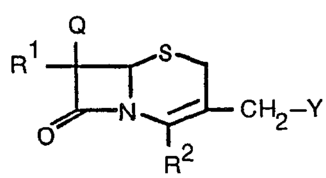

(II)

où $R^1$, $R^2$ et Q sont chacun comme définis dans la revendication 1, et Y est un groupe qui peut être substitué par un groupe ayant la formule

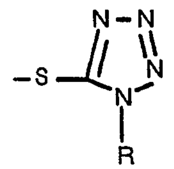

(où R est comme défini dans la revendication 1) ou son sel, avec un composé de formule:

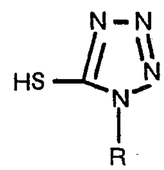

(III)

où R est comme défini dans la revendication 1, ou son dérivé réactif sur le groupe mercapto ou son sel; ou

2) à faire réagir un composé de formule:

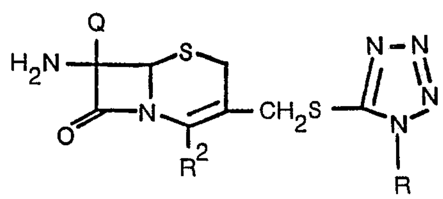

où $R^2$, R et Q sont comme définis dans la revendication 1, ou ses dérivés réactifs sur le groupe amino ou son sel, avec un agent d'acylation, pour donner un composé de formule (I) où $R^1$ est un groupe acylamino, ou son sel; ou

3) à soumettre un composé de formule:

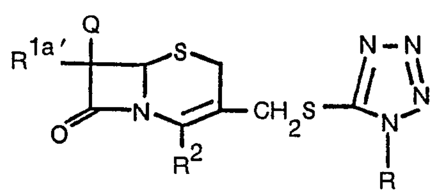

(Id)

où

$R^2$ et Q sont chacun comme définis dans la revendication 1 et

$R^{1a'}$ est un groupe acylamino ayant un groupe amino protégé, ou son sel, à la réaction d'élimination du groupe de protection du groupe amino, pour donner un composé de formule (I) où $R^1$ est un groupe acylamino ayant un groupe amino, ou son sel; ou

4) à soumettre un composé de formule:

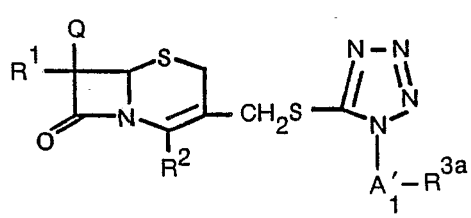

(If)

où

$R^1$, $R^2$, $R^{3a}$ et Q sont chacun comme définis dans la revendication 1 et

$A^{1'}$ est un groupe amino protégé alkylène en $C_1$ à $C_6$, ou son sel, à la réaction d'élimination du groupe de protection du groupe amino, pour donner un composé de formule (I) où R est un groupe amino alkylène en $C_1$ à $C_6$, ou son sel.

12. Composition pharmaceutique antibactérienne, comprenant un composé de la revendication 1, en association avec un support ou un excipient sensiblement non toxique, pharmaceutiquement acceptable.

13. Composé ayant la formule I, comme défini dans la revendication 1, pour l'utilisation comme médicament.

14. Composé ayant la formule I, comme défini dans la revendication 1, pour l'utilisation dans le traitement des maladies infectieuses.

**Revendication pour l'Etat contractant: AT**

1. Procédé pour préparer un composé de formule (I)

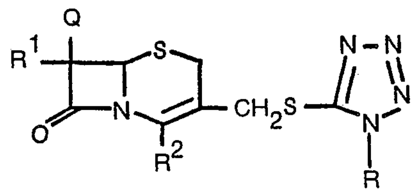

(I)

où

$R^1$ est le groupe amino ou un groupe acylamino;

$R^2$ est le groupe carboxy ou un groupe carboxy estérifié;

R est un groupe de formule:

$$-A_1-R^{3a}$$

[où $A_1$ est un groupe hydroxyalkylène en $C_1$ à $C_6$, aminoalkylène en $C_1$ à $C_6$, amino protégé alkylène en $C_1$ à $C_6$, alkénylène ayant jusqu'à 6 atomes de carbone ou hydroxyiminoalkylène en $C_1$ à $C_6$ où l'atome d'hydrogène du groupe hydroxyimino peut être remplacé par un groupe hydrocarbone aliphatique en $C_1$ à $C_6$; et $R^{3a}$ est un groupe carboxy ou un groupe carboxy estérifié] ou un groupe de formule:

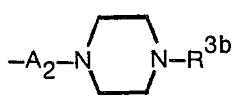

[où $A_2$ est un groupe alkylène en $C_1$ à $C_6$ qui peut avoir un groupe oxo; et $R^{3b}$ est le groupe carboxy, un groupe carboxy estérifié, un groupe aliphatique ou aromatique]; et

Q est l'hydrogène ou un groupe alcoxy en $C_1$ à $C_6$;

ou de son sel pharmaceutiquement acceptable; qui consiste:

1) à faire réagir un composé de formule:

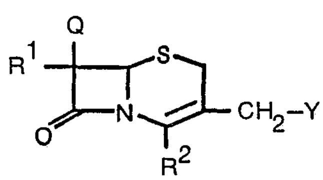

(II)

où $R^1$, $R^2$ et Q sont chacun comme définis ci-dessus et Y est un groupe qui peut être substitué par un groupe de formule

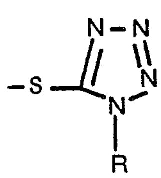

(où R est comme défini ci-dessus) ou son sel, avec un composé de formule:

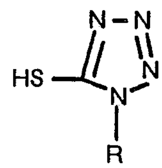

(III)

où R est comme défini ci-dessus, ou son dérivé réactif sur le groupe mercapto ou son sel; ou

2) à faire réagir un composé de formule:

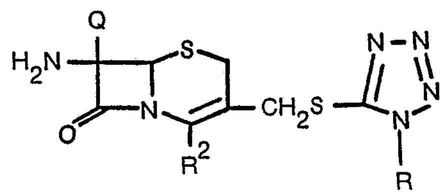

où $R^2$, R et Q sont chacun comme définis ci-dessus, ou ses dérivés réactifs sur le groupe amino ou son sel, avec un agent d'acylation, pour donner un composé de formule (I) où $R^1$ est un groupe acylamino, ou son sel; ou

3) à soumettre un composé de formule:

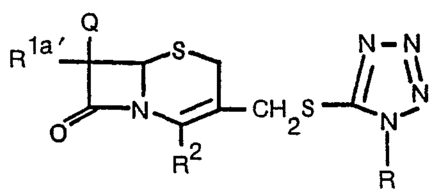

(Id)

où

$R^2$, R et Q sont chacun comme définis ci-dessus et

$R^{1a'}$ est un groupe acylamino ayant un groupe amino protégé, ou son sel, à la réaction d'élimination du groupe de protection du groupe amino, pour donner un composé de formule (I) où $R^1$ est un groupe acylamino ayant un groupe amino, ou son sel; ou

4) à soumettre un composé de formule:

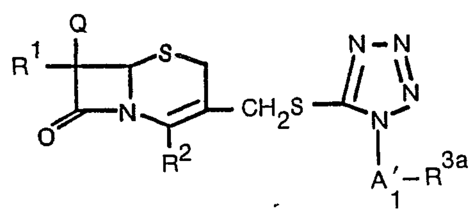

(If)

où

$R^1$, $R^2$, $R^{3a}$ et Q sont chacun comme définis ci-dessus; et

$A^{1'}$ est un groupe amino protégé alkylène en $C_1$ à $C_6$, ou son sel, à la réaction d'élimination du groupe de protection du groupe amino, pour donner un composé de formule (I) où R est un groupe amino alkylène en $C_1$ à $C_6$, ou son sel.

2. Procédé selon la revendication 1, dans lequel $R^1$, $R^2$, $R^{3a}$, $A_2$ et Q sont comme définis dans la revendication 1, R est un groupe de formule:

$$-A_1-R^{3a}$$

[où $A_1$ est un groupe hydroxyalkylène en $C_1$ à $C_6$ aminoalkylène, en $C_1$ à $C_6$, acylaminoalkylène en $C_1$ à $C_6$, hydroxyiminoalkylène en $C_1$ à $C_6$ alcoxy en $C_1$ à $C_6$ iminoalkylène en $C_1$ à $C_6$, alkényl (ayant jusqu'à 6 atomes de carbone) oxyiminoalkylène en $C_1$ à $C_6$ ou alkénylène ayant jusqu'à 6 atomes de carbone; et $R^{3b}$ est le groupe carboxy, un groupe carboxy estérifié, alkyle en $C_1$ à $C_6$, alkènyle ayant jusqu'à 6 atomes de carbone, hydroxy alkyle en $C_1$ à $C_6$ar-alkyle en $C_1$ à $C_6$ ou aryle.

3. Procédé selon la revendication 1 ou 2, dans lequel $R^1$ est le groupe amino; $R^2$ est le groupe carboxy; R est un groupe de formule:

$$—A_1—R^{3a}$$

[où $A_1$ est un groupe hydroxyalkylène en $C_1$ à $C_6$, aminoalkylène en $C_1$ à $C_6$, alcoxy en $C_1$ à $C_6$ carbonylaminoalkylène en $C_1$ à $C_6$, hydroxyiminoalkylène en $C_1$ à $C_6$, alcoxy en $C_1$ à $C_6$ iminoalkylène en $C_1$ à $C_6$ alkényl (ayant jusqu'à 6 atomes de carbone) oxyiminoalkylène en $C_1$ à $C_6$ ou alkénylène ayant jusqu'à 6 atomes de carbone; et $R^{3a}$ est le groupe carboxy] ou un groupe de formule:

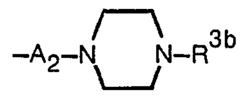

[où $A^2$ est un groupe alkylène en $C_1$ à $C_6$ qui peut avoir un groupe alcoxy en $C_1$ à $C_6$ carbonyle, alkyle en $C_1$ à $C_6$, alkényle ayant jusqu'à 6 atomes de carbone, hydroxyalkyle en $C_1$ à $C_6$, phényl-alkylé en $C_1$ à $C_6$ ou phényle]; et

Q est l'hydrogène

4. Procédé selon les revendications 1 ou 2, dans lequel $R^1$ est un groupe de formule:

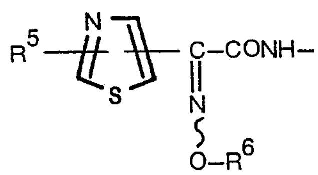

(où $R^5$ est le groupe amino ou un groupe amino protégé; et $R^6$ est un groupe aliphatique ou aromatique).

5. Procédé selon la revendication 4, dans lequel l'isomère syn est obtenu.

6. Procédé selon les revendications 4 ou 5, dans lequel le groupe

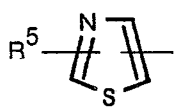

est

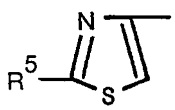

7. Procédé selon l'une quelconque des revendications 4 à 6, dans lequel $R^2$ est le groupe carboxy; $R^5$ est le groupe amino; et $R^6$ est un groupe alkyle en $C_1$ à $C_6$, alkényle ayant jusqu'à 6 atomes de carbone, alkynyle ayant jusqu'à 6 atomes de carbone, cycloalkyle, cycloalkényle ayant jusqu'à 6 atomes de carbone, aryle, ar-alkyle en $C_1$ à $C_6$ qui peut avoir un halogène.

8. Procédé selon l'une quelconque des revendications 4 à 7, dans lequel R est un groupe de formule:

$$—A_1—R^{3a}$$

$A_1$ étant comme défini dans la revendication 3, et $R^{3a}$ étant comme défini dans la revendication 1, et Q est l'hydrogène.

9. Procédé selon l'une quelconque des revendications 4 à 7, dans lequel R est un groupe de formule:

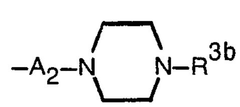

$A_2$ étant comme défini dans la revendication 1 et $R^{3b}$ étant comme défini dans la revendication 2, et Q est l'hydrogène.

10. Procédé selon les revendications 1 ou 2, dans lequel $R^1$ est un groupe phénylalcanoyl en $C_1$ à $C_6$ amino qui peut avoir un groupe hydroxy et/ou carboxy, R est un groupe de formule

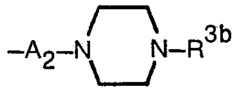

[où $A_2$ est un groupe alkylène en $C_1$ à $C_6$ et $R^{3b}$ est un groupe alkyle en $C_1$ à $C_6$]; $R^2$ est un groupe carboxy; et Q est un groupe alkoxy en $C_1$ à $C_6$.